# EUROPEAN PATENT APPLICATION

(11) **EP 4 428 528 A2**
(43) Date of publication of application: **11.09.2024**
(21) Application number: 24174606.4
(22) Date of filing: 20.05.2017
(51) Int. Cl.: G01N 21/64

(54) **LABELED NUCLEOTIDE COMPOSITIONS AND METHODS FOR NUCLEIC ACID SEQUENCING**

(30) Priority: 20.05.2016 US 201662339790 P; 23.11.2016 US 201662426144 P
(62) Divisional of application: 17728708.3
(71) Applicant: Quantum-Si Incorporated, Branford, CT 06405 (US)
(72) Inventor: ROTHBERG, Jonathan, M., Guilford, 06437 (US); LACKEY, Jeremy, Guilford, 06437 (US); REED, Brian, Madison, 06443 (US); SHI, Xinghua, Madison, 06443 (US); HUANG, Haidong, Madison, 06643 (US); DODD, David, Guilford, 06437 (US)
(74) Representative: Carpmaels & Ransford LLP

(57) **Abstract**

Methods of sequencing molecules based on luminescence lifetimes and/or intensities are provided. In some aspects, methods of sequencing nucleic acids involve determining the luminescence lifetimes, and optionally luminescence intensities, of a series of luminescently labeled nucleotides incorporated during a nucleic acid sequencing reaction. In some aspects, the disclosure provides compositions comprising luminescently labeled nucleotides.

## Description

### RELATED APPLICATIONS

This application claims priority under 35 U.S.C. § 119(e) to U.S. Provisional Patent Application No. 62/426,144, filed November 23, 2016, and U.S. Provisional Patent Application No. 62/339,790, filed May 20, 2016, each of which is hereby incorporated by reference in its entirety.

### FIELD OF THE APPLICATION

The present application is directed generally to methods, compositions, and devices for performing rapid, massively parallel, quantitative analysis of biological and/or chemical samples, and methods of fabricating said devices.

### BACKGROUND

Detection and analysis of biological samples may be performed using biological assays (''bioassays''). Bioassays conventionally involve large, expensive laboratory equipment requiring research scientists trained to operate the equipment and perform the bioassays. Moreover, bioassays are conventionally performed in bulk such that a large amount of a particular type of sample is necessary for detection and quantitation.

Some bioassays are performed by tagging samples with luminescent markers that emit light of a particular wavelength. The markers are illuminated with a light source to cause luminescence, and the luminescent light is detected with a photodetector to quantify the amount of luminescent light emitted by the markers. Bioassays using luminescent markers conventionally involve expensive laser light sources to illuminate samples and complicated luminescent detection optics and electronics to collect the luminescence from the illuminated samples.

### SUMMARY

Among other aspects, the disclosure provides a luminescently labeled nucleotide comprising one or more luminescent labels connected to one or more nucleoside polyphosphates via a nucleic acid, wherein the nucleic acid comprises a protecting element. In some embodiments, the protecting element relates to one or more features of and/or modifications to the nucleic acid that protect a polymerase from damaging effects from the one or more luminescent labels when a nucleoside polyphosphate of the one or more nucleoside polyphosphates is in or near the active site of the polymerase.

In some embodiments, the nucleic acid is single-stranded. In some embodiments, the nucleic acid is double-stranded. In some embodiments, a double-stranded nucleic acid according to the disclosure comprises a first oligonucleotide strand that comprises the one or more luminescent labels attached at an internal position having one or more nucleotides on either side along the first oligonucleotide strand, and a second oligonucleotide strand that comprises the one or more nucleoside polyphosphates, wherein the second oligonucleotide strand is annealed to the first oligonucleotide strand.

In some embodiments, a luminescently labeled nucleotide of the disclosure comprises two or more nucleoside polyphosphates attached to a nucleic acid via a linker that comprises a plurality of thymidine nucleotides. In some embodiments, the linker comprises a branched linker, e.g., a branched thymidine linker. In some embodiments, the branched linker comprises a branched thymidine linker. For example, in some embodiments, each nucleoside polyphosphate comprises a thymidine linker of the formula Nu-T(T)ₙT-R, where Nu represents a nucleoside polyphosphate. T represents a thymidine nucleotide, n is an integer with a value between 1 and 30, and R represents a point of convergence connecting one or more additional nucleoside polyphosphates. In some embodiments, the point of convergence is further attached directly to an oligonucleotide strand of the nucleic acid. In some embodiments, the point of convergence is further attached indirectly to the oligonucleotide strand, e.g., through further thymidine linkers and/or further points of convergence.

In some embodiments, the protecting element of the nucleic acid comprises at least one energy-absorbing modification. In some embodiments, the at least one energy-absorbing modification comprises a triplet state quencher. In some embodiments, the at least one energy-absorbing modification comprises a dendron modification. In some embodiments, the at least one energy-absorbing modification comprises a monosaccharide-TEG, a disaccharide, an N-acetyl monosaccharide, a TEMPO-TEG, a trolox-TEG, or a glycerol dendrimer. In some embodiments, the protecting element comprises one or more stem-loops. In some embodiments, the one or more stem-loops are unlabeled, e.g., the one or more stem-loops do not comprise a luminescent label attached at a loop and/or stem of the stem-loop. In some embodiments, the one or more stem-loops are in a position on the nucleic acid that is between the one or more luminescent labels and the one or more nucleoside polyphosphates. In such embodiments, the one or more unlabeled stem-loops can provide a steric barrier and/or an absorbing barrier that provides polymerase-protecting effects.

In some embodiments, one or more luminescent labels can be attached at a loop of at least one or more stem-loops. However, in some embodiments, a protecting element, for example one or more unlabeled stem-loops, separates the one or more luminescent labels from the one or more nucleoside polyphosphates.

In some embodiments, the nucleic acid of the luminescently labeled nucleotide further comprises a third oligonucleotide strand annealed to at least one of the first and second oligonucleotide strands. In some embodiments, the nucleic acid further comprises a fourth oligonucleotide strand annealed to at least one of the first, second, and third oligonucleotide strands. In some embodiments, the oligonucleotide strands form a Holliday junction.

In some aspects, the disclosure provides methods of determining the sequence of a template nucleic acid. In some embodiments, the methods include a step comprising exposing a complex in a target volume, the complex comprising the template nucleic acid, a primer, and a polymerizing enzyme, to a plurality of types of luminescently labeled nucleotides. In some embodiments, each type of luminescently labeled nucleotide comprises one or more luminescent labels connected to one or more nucleoside polyphosphates via a nucleic acid. In some embodiments, the nucleic acid comprises a protecting element. Accordingly, in some aspects, the disclosure provides methods of nucleic acid sequencing that utilize any of the luminescently labeled nucleoside polyphosphate compositions described herein.

In some embodiments, the methods further comprise a step of directing a series of pulses of one or more excitation energies towards a vicinity of the target volume. In some embodiments, the methods further comprise a step of detecting a plurality of emitted photons from luminescently labeled nucleotides during sequential incorporation into a nucleic acid comprising the primer. In some embodiments, the methods further comprise a step of identifying the sequence of incorporated nucleotides by determining timing and optionally frequency of the emitted photons.

In some embodiments, four different types of nucleotides (e.g., adenine, guanine, cytosine, thymine/uracil) in a reaction mixture can each be labeled with one or more luminescent molecules (e.g., one or more luminescent labels). In some embodiments, each type of nucleotide can be connected to more than one of the same luminescent molecule (e.g., two or more of the same fluorescent dye connected to a nucleotide). In some embodiments, each luminescent molecule can be connected to more than one nucleotide (e.g., two or more of the same nucleotide). In some embodiments, more than one nucleotide can be connected (e.g., via a nucleic acid linker comprising one or more protecting elements) to more than one luminescent molecule. In some embodiments, all four nucleotides are labeled with luminescent molecules that absorb and emit within the same spectral range (e.g., 520-570 nm).

In some embodiments, the luminescent labels among a set of four nucleotides can be selected from dyes comprising an aromatic or heteroaromatic compound and can be a pyrene, anthracene, naphthalene, acridine, stilbene, indole, benzindole, oxazole, carbazole, thiazole, benzothiazole, phenanthridine, phenoxazine, porphyrin, quinoline, ethidium, benzamide, cyanine, carbocyanine, salicylate, anthranilate, coumarin, fluoroscein, rhodamine, or other like compound. Exemplary dyes include xanthene dyes, such as fluorescein or rhodamine dyes, naphthalene dyes, coumarin dyes, acridine dyes, cyanine dyes, benzoxazole dyes, stilbene dyes, pyrene dyes, phthalocyanine dyes, phycobiliprotein dyes, squaraine dyes, BODIPY dyes, and the like.

In some embodiments, the luminescent labels among a set of four nucleotides comprise Alexa Fluor^{®} 546, Cy^{®}3B, Alexa Fluor^{®} 555 and Alexa Fluor^{®} 555, and the FRET pair Alexa Fluor^{®} 555 and Cy^{®}3.5. In some embodiments, the luminescent labels among a set of four nucleotides comprise Alexa Fluor^{®} 555, Cy^{®}3.5. Alexa Fluor^{®} 546, and DyLight^{®} 554-R1. In some embodiments, the luminescent labels among a set of four nucleotides comprise Alexa Fluor^{®} 555, Cy^{®}3.5, ATTO Rho6G, and DyLight^{®} 554-R1. In some embodiments, the luminescent labels among a set of four nucleotides comprise Alexa Fluor^{®} 555, Cy^{®}3B, ATTO Rho6G, and DyLight^{®} 554-R1. In some embodiments, the luminescent labels among a set of four nucleotides comprise Alexa Fluor^{®} 555, Cy^{®}3B, ATTO 542, and DyLight^{®} 554-R1. In some embodiments, the luminescent labels among a set of four nucleotides comprise Alexa Fluor^{®} 555, Cy^{®}3B, ATTO 542, and Alexa Fluor^{®} 546. In some embodiments, the luminescent labels among a set of four nucleotides comprise Cy^{®}3.5, Cy^{®}3B, ATTO Rho6G, and DyLight^{®} 554-R1.

In certain embodiments, at least one type, at least two types, at least three types, or at least four of the types of luminescently labeled nucleotides comprise a luminescent dye selected from the group consisting of 6-TAMRA, 5/6-Carboxyrhodamine 6G, Alexa Fluor^{®} 546, Alexa Fluor^{®} 555, Alexa Fluor^{®} 568, Alexa Fluor^{®} 610. Alexa Fluor^{®} 647, Aberrior Star 635, ATTO 425, ATTO 465, ATTO 488, ATTO 495, ATTO 514, ATTO 520, ATTO Rho6G, ATTO 542, ATTO 647N, ATTO Rho14, Chromis 630, Chromis 654A, Chromeo^{™} 642, CF^{™}514, CF^{™}532, CF^{™}543, CF^{™}546, CF^{™}546, CF^{™}555, CF^{™}568, CF^{™}633, CF^{™}640R, CF^{™}660C, CF^{™}660R, CF^{™}680R, Cy^{®}3, Cy^{®}3B, Cy^{®}3.5, Cy^{®}5, Cy^{®}5.5, Dyomics-530, Dyomics-547P1, Dyomics-549P1, Dyomics-550, Dyomics-554, Dyomics-555, Dyomics-556, Dyomics-560, Dyomics-650, Dyomics-680, DyLight^{®} 554-R1, DyLight^{®} 530-R2, DyLight^{®} 594, DyLight^{®} 635-B2, DyLight^{®} 650, DyLight^{®} 655-134, DyLight^{®} 675-B2, DyLight^{®} 675-B4, DyLight^{®} 680, HiLyte^{™} Fluor 532, HiLyte^{™} Fluor 555, HiLyte^{™} Fluor 594, LightCycler^{®} 640R, Seta^{™} 555. Seta^{™} 670. Seta^{™}700, Seta^{™}u 647, and Seta^{™}u 665, or are of formulae (Dye 101), (Dye 102), (Dye 103), (Dye 104), (Dye 105), or (Dye 106), as described herein.

In some embodiments, at least one type, at least two types, at least three types, or at least four of the types of luminescently labeled nucleotides each comprise a luminescent dye selected from the group consisting of Alexa Fluor^{®} 546, Alexa Fluor^{®} 555, Cy^{®}3B, Cy^{®}3.5, DyLight^{®} 554-R1, Alexa Fluor^{®} 546, Atto Rho6G, ATTO 425, ATTO 465, ATTO 488, ATTO 495, ATTO 514, ATTO 520, ATTO Rho6G, and ATTO 542.

In some embodiments, a first type of luminescently labeled nucleotide comprises Alexa Fluor^{®} 546, a second type of luminescently labeled nucleotide comprises Cy^{®}3B, a third type of luminescently labeled nucleotide comprises two Alexa Fluor^{®} 555, and a fourth type of luminescently labeled nucleotide comprises Alexa Fluor^{®} 555 and Cy^{®}3.5.

In some embodiments, a first type of luminescently labeled nucleotide comprises Alexa Fluor^{®} 555. a second type of luminescently labeled nucleotide comprises Cy^{®}3.5, a third type of luminescently labeled nucleotide comprises Alexa Fluor^{®} 546, and a fourth type of luminescently labeled nucleotide comprises DyLight^{®} 554-R1.

In some embodiments, a first type of luminescently labeled nucleotide comprises Alexa Fluor^{®} 555, a second type of luminescently labeled nucleotide comprises Cy^{®}3.5, a third type of luminescently labeled nucleotide comprises ATTO Rho6G, and a fourth type of luminescently labeled nucleotide comprises DyLight^{®} 554-R1.

In some embodiments, a first type of luminescently labeled nucleotide comprises Alexa Fluor^{®} 555, a second type of luminescently labeled nucleotide comprises Cy^{®}3B, a third type of luminescently labeled nucleotide comprises ATTO Rho6G, and a fourth type of luminescently labeled nucleotide comprises DyLight^{®} 554-R1.

In some embodiments, a first type of luminescently labeled nucleotide comprises Alexa Fluor^{®} 555, a second type of luminescently labeled nucleotide comprises Cy^{®}3B, a third type of luminescently labeled nucleotide comprises ATTO 542, and a fourth type of luminescently labeled nucleotide comprises DyLight^{®} 554-R1.

In some embodiments, a first type of luminescently labeled nucleotide comprises Alexa Fluor^{®} 555, a second type of luminescently labeled nucleotide comprises Cy^{®}3B, a third type of luminescently labeled nucleotide comprises ATTO 542, and a fourth type of luminescently labeled nucleotide comprises Alexa Fluor^{®} 546.

In some embodiments, a first type of luminescently labeled nucleotide comprises Cy^{®}3.5. a second type of luminescently labeled nucleotide comprises Cy^{®}3B, a third type of luminescently labeled nucleotide comprises ATTO Rho6G, and a fourth type of luminescently labeled nucleotide comprises DyLight^{®} 554-R1.

In some embodiments, at least one type, at least two types, at least three types, or at least four of the types of luminescently labeled nucleotides comprise a luminescent dye selected from the group consisting of Alexa Fluor^{®} 532, Alexa Fluor^{®} 546, Alexa Fluor^{®} 555, Alexa Fluor^{®} 594, Alexa Fluor^{®} 610, CF^{™}532, CF^{™}543, CF^{™}555, CF^{™}594, Cy^{®}3, DyLight^{®} 530-R2, DyLight^{®} 554-R1, DyLight^{®} 590-R2, DyLight^{®} 594, DyLight^{®} 610-B1, or are of formulae (Dye 101), (Dye 102), (Dye 103), (Dye 104), (Dye 105), or (Dye 106).

In some embodiments, a first and second type of luminescently labeled nucleotide comprise a luminescent dye selected from the group consisting of Alexa Fluor^{®} 532, Alexa Fluor^{®} 546. Alexa Fluor^{®} 555, CF^{™}532, CF^{™}543, CF^{™}555, Cy^{®}3, DyLight^{®} 530-R2, DyLight^{®} 554-R1, and a third and fourth type of luminescently labeled nucleotide comprise a luminescent dye selected from the group consisting of Alexa Fluor^{®} 594. Alexa Fluor^{®} 610, CF^{™}594, DyLight^{®} 590-R2, DyLight^{®} 594, DyLight^{®} 610-13 1, or are of formulae (Dye 101), (Dye 102), (Dye 103), (Dye 104). (Dye 105), or (Dye 106).

In certain embodiments, at least one type, at least two types, at least three types, or at least four of the types of luminescently-labeled nucleotide molecules comprise a luminescent protein selected from the group consisting of TagBFP, mTagBFP2, Azurite, EBFP2, mKalama1, Sirius, Sapphire, T-Sapphire, ECFP, Cerulean, SCFP3A, mTurquoise, mTurquoise2, monomeric Midoriishi-Cyan, TagCFP, mTFP1, EGFP, Emerald, Superfolder GFP, monomeric Azami Green, TagGFP2, mUKG, mWasabi, Clover, mNeonGreen, EYFP, Citrine, Venus, SYFP2, TagYFP, monomeric Kusabira-Orange, mKOK, mKO2, mOrange, mOrange2, mRaspberry, mCherry, mStrawberry, mTangerine, tdTomato, TagRFP, TagRFP-T. mApple, mRuby, mRuby2, mPlum, HcRed-Tandem, mKate2, mNeptune, NirFP, TagRFP657, IFP1.4, iRFP, mKeima Red, LSS-mKate1, LSS-mKate2, mBeRFP, PA-GFP, PAmCherry1. PATagRFP. Kaede (green), Kaede (red), KikGR1 (green), KikGR1 (red), PS-CFP2, mEos2 (green), mEos2 (red), PSmOrange, or Dronpa.

Aspects of the present application provide methods for delivering an excitation energy to a molecule (e.g., a luminescently labeled nucleoside polyphosphate) to be identified and detecting emitted photons after the excitation. In certain embodiments, detecting comprises recording for each detected luminescence the time duration between the luminescence and the prior pulse of excitation energy. In certain embodiments, detecting comprises recording for each of a plurality of detected luminescences the time duration between the luminescence and the prior pulse of excitation energy. In certain embodiments, a plurality of pulses of excitation energy are delivered. The luminescent marker (e.g., the one or more luminescent labels) of the molecule to be identified may be excited by each pulse or a portion of the pulses. In certain embodiments, a plurality of luminescences are detected by one or more sensors. The luminescent marker of the molecule to be identified may emit luminescence after each excitation or a portion of the excitations. The fraction of excitation events that result in a luminescence is based on the luminescence quantum yield of the marker. In some embodiments, increasing the number of luminescent labels can increase the quantum yield (e.g., increase the number of luminescence emissions). Additionally, not all luminescences emitted by a marker will be detected, for example, some luminescences will be directed away from the sensors. In certain embodiments, the excitation energy or energies are selected based on the luminescent properties of the luminescent markers, including the absorption spectra and wavelengths at which a marker emits photons after excitation in a given spectral range.

In certain embodiments, the frequency of pulsed excitation energies is selected based on the luminescent properties (e.g., luminescent lifetime) of the luminescently labeled molecule (e.g., luminescently labeled nucleoside polyphosphate) to be detected. In some embodiments, the gap between pulses is longer than the luminescent lifetime of one or more luminescently labeled molecules being excited. In some embodiments, the gap is between about two times and about ten times, between about ten times and about 100 times, or between about 100 times and about 1000 times longer than the luminescent lifetime of one or more luminescently labeled molecules being excited. In some embodiments, the gap is about 10 times longer than the luminescent lifetime of one or more luminescently labeled molecules being excited.

In certain embodiments, the frequency of pulsed excitation energies is selected based on the chemical process being monitored. For a sequencing reaction the number of pulses delivered to the target volume while a luminescently labeled nucleotide is being incorporated will in part determine the number of emitted photons detected. In some embodiments, the frequency is selected to allow for a sufficient number of photons to be detected during the incorporation of a luminescently labeled nucleotide, wherein a sufficient number is the number of photons necessary to distinguish the luminescently labeled nucleotide from amongst a plurality of types of luminescently labeled nucleotides. In some embodiments, the luminescently labeled nucleotide is distinguished based on the wavelength of the emitted photons. In some embodiments, the luminescently labeled nucleotide is distinguished based on the luminescent emission lifetime, e.g., the time between pulse excitation and emission detection. In some embodiments, the luminescently labeled nucleotide is distinguished based on the wavelength and the luminescent emission lifetime of the emitted photons. In some embodiments, the luminescently labeled nucleotide is distinguished based on the luminescent intensity of the emission signal (e.g.. based on the frequency of emission or the total number of emission events within a time period). In some embodiments, the luminescently labeled nucleotide is distinguished based on the luminescent intensity of the emission signal and the luminescent lifetime. In some embodiments, the luminescently labeled nucleotide is distinguished based on the luminescent intensity and the wavelength. In some embodiments, the luminescently labeled nucleotide is distinguished based on the luminescent intensity, the wavelength, and the luminescent lifetime.

According to another aspect of the present application, a method of sequencing a target nucleic acid is provided. In some embodiments, the method of sequencing a target nucleic acid comprises steps of: (i) providing a mixture comprising (a) said target nucleic acid, (b) a primer complementary to said target nucleic acid, (c) a nucleic acid polymerase, and (d) nucleotides for incorporation into a growing nucleic acid strand complementary to said target nucleic acid, wherein said nucleotides include different types of luminescently labeled nucleotides, wherein said luminescently labeled nucleotides yield detectable signals during sequential incorporation into said growing nucleic acid strand, which detectable signals for said different types of luminescently labeled nucleotides are differentiable from one another in a time domain (e.g., by determining timing and/or frequency of the detectable signals); (ii) subjecting said mixture of (i) to a polymerization reaction under conditions that are sufficient to yield said growing nucleic acid strand by extension of said primer; (iii) measuring said detectable signals from said luminescently labeled nucleotides during sequential incorporation into said growing nucleic acid strand; and (iv) determining the timing and/or frequency of said measured detectable signals from said luminescently labeled nucleotides upon sequential incorporation into said growing nucleic acid strand to identify a time sequence of incorporation of said luminescently labeled nucleotides into said growing nucleic acid strand, thereby determining a sequence of said target nucleic acid.

In some embodiments, the target nucleic acid or the nucleic acid polymerase is attached to a support. In some embodiments, the time sequence of incorporation is identified subsequent to subjecting the mixture of (i) to the polymerization reaction. In some embodiments, the detectable signals are optical signals. In some embodiments, the optical signals are luminescent signals. In some embodiments, determining the timing and/or frequency of the measured detectable signals comprises (i) receiving said detectable signals at one or more sensors; and (ii) selectively directing charge carriers of a plurality of charge carriers produced in response to said detectable signals received at said one or more sensors into at least one charge carrier storage region based upon times at which said charge carriers are produced.

In some embodiments, the timing and/or frequency of said measured detectable signals comprise measurements of decay lifetimes. In some embodiments, the timing and/or frequency of the measured detectable signals comprise measurements of arrival times of the detectable signals at one or more sensors that detect the detectable signals. In some embodiments, the method further comprises segregating charge carriers produced by the detectable signals into bins associated with the one or more sensors based on the arrival times of the detectable signals. In some embodiments, the timing and/or frequency of the measured detectable signals are non-spectral measurements.

### BRIEF DESCRIPTION OF THE DRAWINGS

The skilled artisan will understand that the figures, described herein, are for illustration purposes only. It is to be understood that in some instances various aspects of the invention may be shown exaggerated or enlarged to facilitate an understanding of the invention. In the drawings, like reference characters generally refer to like features, functionally similar and/or structurally similar elements throughout the various figures. The drawings are not necessarily to scale, emphasis instead being placed upon illustrating the principles of the teachings. The drawings are not intended to limit the scope of the present teachings in any way.

The features and advantages of the present invention will become more apparent from the detailed description set forth below when taken in conjunction with the drawings.

When describing embodiments in reference to the drawings, direction references ("above," "below," "top," "bottom," "left," "right," "horizontal," "vertical," etc.) may be used. Such references are intended merely as an aid to the reader viewing the drawings in a normal orientation. These directional references are not intended to describe a preferred or only orientation of an embodied device. A device may be embodied in other orientations.

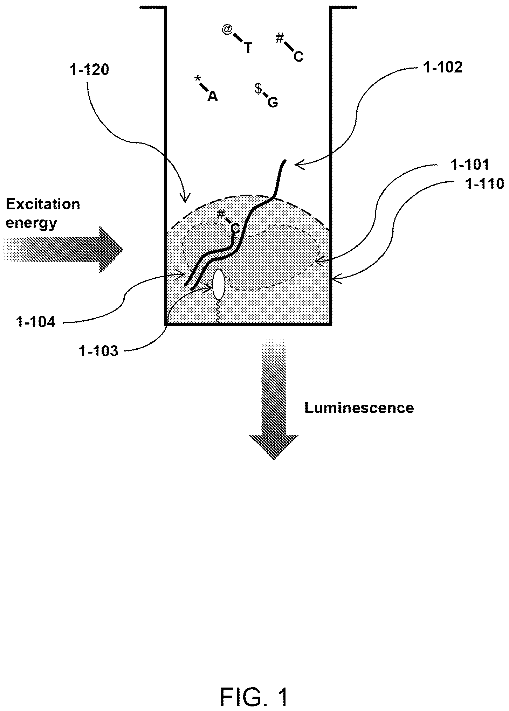

As is apparent from the detailed description, the examples depicted in the figures (e.g., FIGs. 1-9) and further described for the purpose of illustration throughout the application describe non-limiting embodiments, and in some cases may simplify certain processes or omit features or steps for the purpose of clearer illustration.
FIG. 1 shows a non-limiting schematic of a sample well containing various components for nucleic acid sequencing.
FIG. 2 shows a non-limiting, exemplary experiment of nucleic acid sequencing for four stages; (A) before incorporation of a luminescently labeled nucleotide; (13) a first incorporation event; (C) a period between the first and second incorporation events; and (D) a second incorporation event: along with corresponding examples of raw and processed data during stages (A)-(D).
FIG. 3 depicts a non-limiting signal vs. emission time for four luminescent molecules with different luminescent lifetimes and normalized cumulative distribution function for the probability of decay.
FIG. 4 shows a non-limiting chart of luminescent lifetimes and a chart for luminescent intensities for exemplary luminescently labeled nucleotides.
FIG. 5 depicts a non-limiting sequencing experiment with four luminescently labeled nucleotides: (A) trace of detected luminescences from green and red pulses; (B) reduction of data from green pulses based on luminescent lifetime and intensity of each nucleotide incorporation; and (C) alignment of the experimentally determined sequence with the template sequence.
FIG. 6 depicts a non-limiting sequencing experiment with four luminescently labeled nucleotides: (A) trace of detected luminescences from green pulses; (B) reduction of data from green pulses based on luminescent lifetime and intensity of each nucleotide incorporation; and (C) alignment of the experimentally determined sequence with the template sequence.
FIG. 7 depicts a non-limiting sequencing experiment, wherein the luminescent properties of a luminescently-labeled nucleotide are used to identify the base being incorporated into the sequencing reaction.
FIG. 8A and FIG. 8B depict non-limiting examples of a luminescent molecule and a nucleotide separated by a nucleic acid protecting molecule.
FIG. 8C depicts a non-limiting luminescently labeled nucleotide comprising a nucleic acid protecting molecule docked in a polymerase active site. As shown, the dye molecule is attached to the nucleic acid via a 5'-amine-dye linker, and the nucleotide comprises a nucleoside hexaphosphate attached to the nucleic acid via an azido-propanol linker.
FIG. 8D depicts non-limiting examples of nucleic acid protecting molecules.
FIG. 8E depicts a non-limiting example of a sequencing reaction performed with a luminescently labeled deoxythymidine hexaphosphate comprising a 15-base-pair nucleic acid protecting molecule.
FIG. 8F depicts a non-limiting example of a sequencing reaction performed with a Cy3-labeled adenosine and an AlexaFluor-546-labeled cytidine, each comprising a 25-base-pair nucleic acid protecting molecule.
FIG. 8G depicts a non-limiting comparison of intensity traces obtained using an internal luminescent label or an external luminescent label on a 20-base-pair nucleic acid protecting molecule.
FIG. 8H depicts a non-limiting example of a nucleic acid protecting molecule having a luminescent label and a nucleotide attached at opposite ends of the same oligonucleotide strand.
FIG. 8I depicts a non-limiting example of a nucleic acid protecting molecule having a luminescent label and a nucleotide attached to different oligonucleotide strands.
FIG. 8J depicts a non-limiting method of attaching multiple dyes to a strand of a nucleic acid protecting molecule.
FIG. 8K depicts a non-limiting single-stranded nucleic acid protecting molecule comprising three stem loops and two Cy3.5 dyes attached within the backbone of the oligonucleotide strand.
FIG. 8L depicts non-limiting double-stranded oligonucleotide protecting molecules comprising stem loop structures that can provide distance and/or bulk structure between the dye and the nucleoside polyphosphate to protect the polymerase from the dye.
FIG. 8M depicts a non-limiting example of a nucleic acid protecting molecule that forms a Holliday junction.
FIG. 8N depicts non-limiting configurations of luminescently labeled nucleoside polyphosphates comprising a double-stranded nucleic acid protecting molecule.
FIG. 8O depicts non-limiting unlabeled strand configurations having more than one nucleotide (Nu) in a nucleotide domain via branched thymidine oligonucleotide linkers.
FIGs. 8P-8Q depict non-limiting oligonucleotide structures having a plurality of energy-absorbing modifications (non-limiting examples of modifications are shown attached to a 5' end of an oligonucleotide strand).
FIG. 9 depicts a non-limiting reaction scheme for nucleotide linker synthesis and exemplary structures.

### DETAILED DESCRIPTION

Aspects of the disclosure relate to nucleic acid linkers for the attachment of a luminescent label to a nucleoside polyphosphate. Such compositions can be advantageously used in technologies that involve the use of a nucleoside polyphosphate as a reaction substrate. For example, certain DNA sequencing technologies can involve the use of polymerase enzymes in sequencing-by-synthesis reactions, whereby sequence information can be obtained based on unique signals attributable to the incorporation of specific types of labeled nucleotides in a growing strand. In some embodiments, the polymerase activity can be determinative of the robustness of the sequencing results. It is therefore often desirable to protect the polymerase from photo-induced damage in order to preserve proper functionality and overall activity, e.g., for the sake of optimizing readout parameters such as read length and accuracy. Accordingly, the nucleic acid linkers described herein provide polymerase-protecting functionality that eliminates or minimizes photo-induced damage that could otherwise result from a luminescently labeled nucleotide being in close proximity to the polymerase active site.

In some aspects, the disclosure provides luminescently labeled nucleotides that comprise a luminescent label attached to a nucleoside polyphosphate via a nucleic acid linker. In some embodiments, the nucleoside polyphosphate serves as a polymerase substrate in a synthesis reaction, and the nucleic acid linker confers polymerase-protecting properties on the luminescently labeled nucleotide. In some embodiments, a nucleic acid linker provides a separation distance between a luminescent label and a nucleoside polyphosphate to minimize the extent of interaction between the label and a polymerase or other enzyme during an enzyme catalyzed reaction involving the nucleoside polyphosphate. However, the inventors have further recognized and appreciated additional features of nucleic acid linkers that can provide protecting effects. In some embodiments, a nucleic acid linker comprises one or more protecting elements located between the luminescent label and the nucleoside polyphosphate to further protect a polymerase or other enzyme during an enzyme catalyzed reaction involving the nucleoside polyphosphate. For example, in some embodiments a nucleic acid linker comprises one or more structural motifs (e.g., stem-loops, Holliday junctions), a plurality of hybridized oligonucleotide strands, one or more cross-linked base pairs within a hybridized region (e.g., between two or more oligonucleotide strands connecting a luminescent label to a nucleoside polyphosphate), and/or one or more energy-absorbing modifications (e.g., triplet-state quenchers, branched polymeric structures, branched dendritic structures) to provide further polymerase-protecting effects. In some embodiments, the nucleic acid linkers described herein (and the associated labeled nucleotides) are not attached to a particle of material (e.g., are not attached to a particle of metallic, magnetic, polymeric, or other material). In some embodiments, a nucleic acid linker is a linear molecule. In some embodiments, a nucleic acid linker is a circular molecule. In some embodiments, a nucleic acid linker is single stranded (e.g., with or without stem-loop structures). In some embodiments, a nucleic acid linker is double stranded (e.g., with or without stem loop structures). In some embodiments, the two strands of a double stranded nucleic acid linker are hybridized (due to complementary sequences) and not covalently attached. However, in some embodiments, one or more covalent bonds may be introduced (e.g., using one or more chemical linkers) to covalently attach two strands of a double stranded linker. In some embodiments, a nucleic acid linker may include one or more additional moieties as described herein. In some embodiments, a nucleic acid linker includes i) one or more additional moieties within or at the end(s) of the sugar phosphate backbone, ii) one or more modifications (e.g., one or more modified bases or sugars), or a combination of i) and ii). However, in some embodiments a nucleic acid linker does not include i), ii), or either of i) or ii).

In some embodiments, a nucleic acid linker comprises one or more structural motifs, such as stem loops, that provide a steric barrier between a luminescent label and a nucleoside polyphosphate. A stem-loop, or hairpin loop, is an unpaired loop of nucleotides on an oligonucleotide strand that is formed when the oligonucleotide strand folds and forms base pairs with another section of the same strand. In some embodiments, the unpaired loop of a stem-loop comprises three to ten nucleotides. Accordingly, a stem-loop can be formed by two regions of an oligonucleotide strand having inverted complementary sequences that hybridize to form a stem, where the two regions are separated by the three to ten nucleotides that form the unpaired loop. In some embodiments, the stem can be designed to have one or more (G/C nucleotides, which can provide added stability with the addition hydrogen bonding interaction that forms compared to A/T nucleotides. In some embodiments, the stem comprises G/C nucleotides immediately adjacent to an unpaired loop sequence. In some embodiments, the stem comprises G/C nucleotides within the first 2, 3, 4, or 5 nucleotides adjacent to an unpaired loop sequence. In some embodiments, one or more luminescent labels are attached to an unpaired loop of a stem-loop, with the stem region providing rigidity and distance between the one or more luminescent labels and a nucleoside polyphosphate. In some embodiments, one or more luminescent labels are attached to a stem of a stem-loop. In some embodiments, a stem-loop of a nucleic acid linker does not comprise a luminescent label. In some embodiments, a stem-loop of a nucleic acid linker comprises an unpaired region within the stem (e.g., a "bulge"). In some embodiments, one or more unlabeled structural motifs, such as stem-loops, are included in the nucleic acid linker (e.g.. in a position on the nucleic acid linker that is between the one or more luminescent labels and the one or more nucleoside polyphosphates). In such embodiments, the one or more unlabeled structural motifs can provide a steric barrier and/or an absorbing barrier that provides polymerase-protecting effects.

In some embodiments, a nucleic acid linker comprises one or more energy-absorbing modifications, such as quenching moieties, to absorb or otherwise mitigate photo-induced damage to a polymerase. In some embodiments, a nucleic acid linker can comprise additional features that provide polymerase protecting effects. For example, in some embodiments, the position of attachment of a luminescent label on the nucleic acid linker can be selected to provide polymerase protecting effects. In some embodiments, a luminescent label is attached at an internal site on a nucleic acid linker such that the label is held in proximity to a greater extent of the nucleic acid linker relative to a terminally-linked label. In such embodiments, the luminescent label is relatively restricted in its availability to interact with a polymerase in space.

In some embodiments, a nucleic acid linker is double-stranded. In some embodiments, a double-stranded nucleic acid linker comprises a first oligonucleotide strand attached to a luminescent label and a second oligonucleotide strand attached to a nucleoside polyphosphate. In some embodiments, the second oligonucleotide strand further comprises one or more energy-absorbing modifications adjacent to the luminescent label of the annealed first oligonucleotide strand. For example, in some embodiments, the one or more energy-absorbing modifications of the nucleic acid linker are in closer proximity to the luminescent label than the nucleoside polyphosphate. Accordingly, in such embodiments, the one or more energy-absorbing modifications serve as a steric and/or absorbing barrier that intercepts radiative and/or non-radiative decay emitted by the luminescent molecule.

As used herein, a "nucleic acid linker" refers to a nucleic acid that connects a luminescent label to a nucleoside polyphosphate. In some embodiments, the nucleic acid linker provides polymerase-protecting effects. Accordingly, in some embodiments, a nucleic acid linker is alternatively referred to as a "nucleic acid protecting molecule," or more generally, as a "protecting molecule." In some embodiments, the terms nucleic acid and oligonucleotide may be used interchangeably depending on context. As such, a nucleic acid linker, an oligonucleotide linker, a nucleic acid protecting molecule, and an oligonucleotide protecting molecule can refer to the same or similar concepts.

It should be understood that, in the context of a nucleic acid linker (e.g., an oligonucleotide dimer), a "nucleotide" or "nucleoside polyphosphate" attached thereto refers to the one or more nucleotides (e.g., nucleoside phosphates) that are configured to be incorporated into a growing nucleic acid strand (e.g., during a sequencing reaction). In some embodiments, the one or more nucleotides comprise one or more nucleoside monophosphates or nucleoside polyphosphates (e.g., nucleoside di- or triphosphates, or nucleosides with more than three 5' phosphates, etc.). In some embodiments, the one or more nucleoside phosphates (e.g., nucleoside polyphosphates) may be attached through a terminal phosphate to an oligonucleotide (e.g., an unlabeled oligonucleotide strand) that forms part of a protecting molecule as described in this application. In some embodiments of any of the compositions or methods described in this application, a phosphate portion (e.g., a polyphosphate portion) of a nucleoside phosphate (e.g., of a nucleoside polyphosphate) includes one or more phosphates or variants thereof. For example, in some embodiments, a phosphate portion (e.g., a polyphosphate portion) of a nucleoside phosphate (e.g., of a nucleoside polyphosphate) can include a phosphate ester, a thioester, a phosphoramidate, an alkyl phosphonate linkage, other suitable linkage, or more than one such modifications, or a combination of two or more thereof. In some embodiments, the labeled and unlabeled strands of a nucleic acid linker are substantially complementary to one another (e.g., over the length of a dimerization domain wherein the strands within the dimerization domain can have, for example, at least 60%, at least 70%, at least 75%, at least 80%, at least 85%, at least 90%, at least 95%, at least 98%, at least 99%, or 100% complementary to one another).

As described herein, aspects of the disclosure relate to compositions having polymerase-protecting effects. It should be appreciated that there are a variety of parameters by which a practitioner could evaluate polymerase-protecting effects. Generally, the effects of a protecting element can be evaluated by conducting a comparative assessment between a composition having the protecting element and a composition lacking the protective element. For example, in some embodiments a polymerase protecting element can increase sequence read length (e.g., by 10%-25%, 25-50%, 50-75%, 75-100%, or more than 100%, for example by about 2 fold, 3 fold, 4 fold, 5 fold or more relative to a sequencing reaction performed under the same conditions and the same reagents with the exception of the nucleic acid linker that lacks the one or more protecting element but is otherwise similar or identical. In some embodiments, a protecting element can increase sequence accuracy (e.g.. by around 5%, around 10%, around 15%, or more relative to a sequencing reaction performed under comparative conditions as described above). In some embodiments, protection of the polymerase can be measured by a decrease (e.g., by around 10%, 20%, 30%, 40%, 50%, or more) in the extent to which the polymerase is inactivated by photo-induced damage during strand synthesis as evaluated under comparative conditions described above.

In some embodiments, the disclosure provides new methods and compositions for identifying single molecules based on one or more luminescent properties of those molecules. In some embodiments, a molecule (e.g., a luminescently labeled nucleoside polyphosphate) is identified based on its luminescent lifetime, absorption spectra, emission spectra, luminescent quantum yield, luminescent intensity, or a combination of two or more thereof. Identifying may mean assigning the exact molecular identity of a molecule, or may mean distinguishing or differentiating the particular molecule from a set of possible molecules. In some embodiments, a plurality of single molecules can be distinguished from each other based on different luminescent lifetimes, absorption spectra, emission spectra, luminescent quantum yields, luminescent intensities, or combinations of two or more thereof. In some embodiments, a single molecule is identified (e.g., distinguished from other molecules) by exposing the molecule to a series of separate light pulses and evaluating the timing or other properties of each photon that is emitted from the molecule. In some embodiments, information for a plurality of photons emitted sequentially from a single molecule is aggregated and evaluated to identify the molecule. In some embodiments, a luminescent lifetime of a molecule is determined from a plurality of photons that are emitted sequentially from the molecule, and the luminescent lifetime can be used to identify the molecule. In some embodiments, a luminescent intensity of a molecule is determined from a plurality of photons that are emitted sequentially from the molecule, and the luminescent intensity can be used to identify the molecule. In some embodiments, a luminescent lifetime and luminescent intensity of a molecule is determined from a plurality of photons that are emitted sequentially from the molecule, and the luminescent lifetime and luminescent intensity can be used to identify the molecule.

Aspects of the present application are useful for detecting and/or identifying one or more biological or chemical molecules. In some embodiments, chemical or biological reactions can be evaluated by determining the presence or absence of one or more reagents or products at one or more time points.

Aspects of the present application interrogate a molecule by exposing the molecule to light and determining one or more properties of one or more photons emitted from the molecule. In certain embodiments, the molecule is interrogated by exposing the molecule to a pulse of light and determining one or more properties of a photon emitted from the molecule. In some embodiments, the molecule is exposed to a plurality of separate light pulse events and one or more properties of separate photons emitted after separate light pulse events are determined. In some embodiments, the molecule does not emit a photon in response to each light pulse. However, a plurality of emitted photons can be evaluated by exposing the molecule to a series of separate light pulses and evaluating separate photons that are emitted after a subset of the light pulse events (e.g., photons emitted after about 10% of pulse events, or photons emitted after about 1% of pulse events).

Aspects of the present application are useful to monitor a chemical or biological reaction by determining the presence or absence of one or more reagents, intermediates, and/or products of the reaction at one or more time points. In some embodiments, the progression of a reaction over time can be analyzed by exposing a reaction sample to a series of separate light pulses and analyzing any emitted photon that is detected after each light pulse.

Accordingly, in some aspects of the application, a reaction sample is exposed to a plurality of separate light pulses and a series of emitted photons are detected and analyzed. In some embodiments, the series of emitted photons provides information about a single molecule that is present and that does not change in the reaction sample over the time of the experiment. However, in some embodiments, the series of emitted photons provides information about a series of different molecules that are present at different times in the reaction sample (e.g., as a reaction or process progresses).

In some embodiments, aspects of the present application can be used to assay biological samples, for example to determine the sequence of one or more nucleic acids or polypeptides in the sample and/or to determine the presence or absence of one or more nucleic acid or polypeptide variants (e.g., one or more mutations in a gene of interest) in the sample. In some embodiments, tests can be performed on patient samples (e.g.. human patient samples) to provide nucleic acid sequence information or to determine the presence or absence of one or more nucleic acids of interest for diagnostic, prognostic, and/or therapeutic purposes. In some examples, diagnostic tests can include sequencing a nucleic acid molecule in a biological sample of a subject, for example by sequencing cell free deoxyribonucleic acid (DNA) molecules and/or expression products (e.g., ribonucleic acid (RNA)) in a biological sample of the subject.

In some embodiments, one or more molecules that are being analyzed (e.g., interrogated and/or identified) using luminescent lifetime and/or intensity can be labeled molecules (e.g., molecules that have been labeled with one or more luminescent markers). In some embodiments, individual subunits of biomolecules may be identified using markers. In some examples, luminescent markers are used to identify individual subunits of biomolecules. Some embodiments use luminescent markers (also referred to herein as "markers"), which may be exogenous or endogenous markers. Exogenous markers may be external luminescent markers used as a reporter and/or tag for luminescent labeling. Examples of exogenous markers may include, but are not limited to, fluorescent molecules, fluorophores, fluorescent dyes, fluorescent stains, organic dyes, fluorescent proteins, species that participate in fluorescence resonance energy transfer (FRET), enzymes, and/or quantum dots. Other exogenous markers are known in the art. Such exogenous markers may be conjugated to a probe or functional group (e.g., molecule, ion, and/or ligand) that specifically binds to a particular target or component. Attaching an exogenous tag or reporter to a probe allows identification of the target through detection of the presence of the exogenous tag or reporter. Examples of probes may include proteins, nucleic acid (e.g., DNA, RNA) molecules, lipids and antibody probes. The combination of an exogenous marker and a functional group may form any suitable probes, tags, and/or labels used for detection, including molecular probes, labeled probes, hybridization probes, antibody probes, protein probes (e.g., biotin-binding probes), enzyme labels, fluorescent probes, fluorescent tags, and/or enzyme reporters.

Although the present disclosure makes reference to luminescent markers, other types of markers may be used with devices, systems and methods provided herein. Such markers may be mass tags, electrostatic tags, electrochemical labels, or any combination thereof.

While exogenous markers may be added to a sample. endogenous markers may be already part of the sample. Endogenous markers may include any luminescent marker present that may luminesce or "autofluoresce" in the presence of excitation energy. Autofluorescence of endogenous fluorophores may provide for label-free and noninvasive labeling without requiring the introduction of exogenous fluorophores. Examples of such endogenous fluorophores may include hemoglobin, oxyhemoglobin, lipids, collagen and elastin crosslinks, reduced nicotinamide adenine dinucleotide (NADH), oxidized flavins (FAD) and FMN), lipofuscin, keratin, and/or porphyrins, by way of example and not limitation.

Having recognized the need for simple, less complex apparatuses for performing single molecule detection and/or nucleic acid sequencing, the inventors have conceived of techniques for detecting single molecules using sets of luminescent tags (e.g., luminescent markers, luminescent labels) to label different molecules. Such single molecules may be nucleotides or amino acids having tags. Tags may be detected while bound to single molecules, upon release from the single molecules, or while bound to and upon release from the single molecules. In some examples, tags are luminescent tags. Each luminescent tag in a selected set is associated with a respective molecule. For example, a set of four tags may be used to "label" the nucleobases present in DNA - each tag of the set being associated with a different nucleobase, e.g., a first tag being associated with adenine (A), a second tag being associated with cytosine (C), a third tag being associated with guanine (G), and a fourth tag being associated with thymine (T). Moreover, each of the luminescent tags in the set of tags has different properties that may be used to distinguish a first tag of the set from the other tags in the set. In this way, each tag is uniquely identifiable using one or more of these distinguishing characteristics. By way of example and not limitation, the characteristics of the tags that may be used to distinguish one tag from another may include the emission energy and/or wavelength of the light that is emitted by the tag in response to excitation energy, the wavelength of the excitation light that is absorbed by a particular tag to place the tag in an excited state, and/or the emission lifetime of the tag.

### Sequencing

Some aspects of the application are useful for sequencing biological polymers, such as nucleic acids and proteins. In some embodiments, methods, compositions, and devices described in the application can be used to identify a series of nucleotide or amino acid monomers that are incorporated into a nucleic acid or protein (e.g., by detecting a time-course of incorporation of a series of labeled nucleotide or amino acid monomers). In some embodiments, methods, compositions, and devices described in the application can be used to identify a series of nucleotides that are incorporated into a template-dependent nucleic acid sequencing reaction product synthesized by a polymerase enzyme.

In certain embodiments, the template-dependent nucleic acid sequencing product is carried out by naturally occurring nucleic acid polymerases. In some embodiments, the polymerase is a mutant or modified variant of a naturally occurring polymerase. In some embodiments, the template-dependent nucleic acid sequence product will comprise one or more nucleotide segments complementary to the template nucleic acid strand. In one aspect, the application provides a method of determining the sequence of a template (or target) nucleic acid strand by determining the sequence of its complementary nucleic acid strand.

The term "polymerase," as used herein, generally refers to any enzyme (or polymerizing enzyme) capable of catalyzing a polymerization reaction. Examples of polymerases include, without limitation, a nucleic acid polymerase, a transcriptase or a ligase. A polymerase can be a polymerization enzyme.

Embodiments directed towards single molecule nucleic acid extension (e.g., for nucleic acid sequencing) may use any polymerase that is capable of synthesizing a nucleic acid complementary to a target nucleic acid molecule. In some embodiments, a polymerase may be a DNA polymerase, an RNA polymerase, a reverse transcriptase, and/or a mutant or altered form of one or more thereof.

Examples of polymerases include, but are not limited to, a DNA polymerase, an RNA polymerase, a thermostable polymerase, a wild-type polymerase, a modified polymerase, E. coli DNA polymerase I, T7 DNA polymerase, bacteriophage T4 DNA polymerase ϕ29 (psi29) DNA polymerase, Taq polymerase, Tth polymerase, Tli polymerase, Pfu polymerase, Pwo polymerase, VENT polymerase, DEEPVENT polymerase, EX-Taq polymerase, LA-Taq polymerase, Sso polymerase, Poc polymerase, Pab polymerase, Mth polymerase. ES4 polymerase, Tru polymerase, Tac polymerase, Tne polymerase, Tma polymerase, Tca polymerase, Tih polymerase, Tfi polymerase, Platinum Taq polymerases, Tbr polymerase. Tfl polymerase, Tth polymerase, Pfutubo polymerase, Pyrobest polymerase, Pwo polymerase, KOD polymerase. Bst polymerase. Sac polymerase, Klenow fragment, polymerase with 3' to 5' exonuclease activity, and variants, modified products and derivatives thereof. In some embodiments, the polymerase is a single subunit polymerase. Non-limiting examples of DNA polymerases and their properties are described in detail in, among other places, DNA Replication 2nd edition, Kornberg and Baker. W. H. Freeman, New York, N.Y. (1991).

Upon base pairing between a nucleobase of a target nucleic acid and the complementary dNTP, the polymerase incorporates the dNTP into the newly synthesized nucleic acid strand by forming a phosphodiester bond between the 3' hydroxyl end of the newly synthesized strand and the alpha phosphate of the dNTP. In examples in which the luminescent tag conjugated to the dNTP is a fluorophore, its presence is signaled by excitation and a pulse of emission is detected during or after the step of incorporation. For detection labels that are conjugated to the terminal (gamma) phosphate of the dNTP, incorporation of the dNTP into the newly synthesized strand results in release the beta and gamma phosphates and the detection label, which is free to diffuse in the sample well, resulting in a decrease in emission detected from the fluorophore.

In some embodiments, the polymerase is a polymerase with high processivity. However, in some embodiments, the polymerase is a polymerase with reduced processivity. Polymerase processivity generally refers to the capability of a polymerase to consecutively incorporate dNTPs into a nucleic acid template without releasing the nucleic acid template. In some embodiments, the polymerase is a polymerase with low 5'-3' exonuclease activity and/or 3'-5' exonuclease. In some embodiments, the polymerase is modified (e.g., by amino acid substitution) to have reduced 5'-3' exonuclease activity and/or 3'-5' activity relative to a corresponding wild-type polymerase. Further non-limiting examples of DNA polymerases include 9°Nm^{™} DNA polymerase (New England Biolabs), and a P680G mutant of the Klenow exo- polymerase (Tuske et al. (2000) JBC 275(31):23759-23768). In some embodiments, a polymerase having reduced processivity provides increased accuracy for sequencing templates containing one or more stretches of nucleotide repeats (e.g., two or more sequential bases of the same type). In some embodiments, the polymerase is a polymerase that has a higher affinity for a labeled nucleotide than for a non-labeled nucleic acid.

In another aspect, the application provides methods of sequencing target nucleic acids by sequencing a plurality of nucleic acid fragments, wherein the target nucleic acid comprises the fragments. In certain embodiments, the method comprises combining a plurality of fragment sequences to provide a sequence or partial sequence for the parent target nucleic acid. In some embodiments, the step of combining is performed by computer hardware and software. The methods described herein may allow for a set of related target nucleic acids, such as an entire chromosome or genome to be sequenced.

During sequencing, a polymerizing enzyme may couple (e.g., attach) to a priming location of a target nucleic acid molecule. The priming location can be a primer that is complementary to a portion of the target nucleic acid molecule. As an alternative the priming location is a gap or nick that is provided within a double stranded segment of the target nucleic acid molecule. A gap or nick can be from 0 to at least 1, 2, 3, 4, 5, 6, 7, 8, 9, 10, 20, 30, or 40 nucleotides in length. A nick can provide a break in one strand of a double stranded sequence, which can provide a priming location for a polymerizing enzyme, such as, for example, a strand displacing polymerase enzyme.

In some cases, a sequencing primer can be annealed to a target nucleic acid molecule that may or may not be immobilized to a solid support. A solid support can comprise, for example, a sample well (e.g., a nanoaperture, a reaction chamber) on a chip used for nucleic acid sequencing. In some embodiments, a sequencing primer may be immobilized to a solid support and hybridization of the target nucleic acid molecule also immobilizes the target nucleic acid molecule to the solid support. In some embodiments, a polymerase is immobilized to a solid support and soluble primer and target nucleic acid are contacted to the polymerase. However, in some embodiments a complex comprising a polymerase, a target nucleic acid and a primer is formed in solution and the complex is immobilized to a solid support (e.g., via immobilization of the polymerase, primer, and/or target nucleic acid). In some embodiments, none of the components in a sample well (e.g., a nanoaperture, a reaction chamber) are immobilized to a solid support. For example, in some embodiments, a complex comprising a polymerase, a target nucleic acid, and a primer is formed in solution and the complex is not immobilized to a solid support.

Under appropriate conditions, a polymerase enzyme that is contacted to an annealed primer/target nucleic acid can add or incorporate one or more nucleotides onto the primer, and nucleotides can be added to the primer in a 5' to 3', template-dependent fashion. Such incorporation of nucleotides onto a primer (e.g., via the action of a polymerase) can generally be referred to as a primer extension reaction. Each nucleotide can be associated with a detectable tag that can be detected and identified (e.g.. based on its luminescent lifetime and/or other characteristics) during the nucleic acid extension reaction and used to determine each nucleotide incorporated into the extended primer and, thus, a sequence of the newly synthesized nucleic acid molecule. Via sequence complementarity of the newly synthesized nucleic acid molecule, the sequence of the target nucleic acid molecule can also be determined. In some cases, annealing of a sequencing primer to a target nucleic acid molecule and incorporation of nucleotides to the sequencing primer can occur at similar reaction conditions (e.g., the same or similar reaction temperature) or at differing reaction conditions (e.g., different reaction temperatures). In some embodiments, sequencing by synthesis methods can include the presence of a population of target nucleic acid molecules (e.g., copies of a target nucleic acid) and/or a step of amplification of the target nucleic acid to achieve a population of target nucleic acids. However, in some embodiments sequencing by synthesis is used to determine the sequence of a single molecule in each reaction that is being evaluated (and nucleic acid amplification is not required to prepare the target template for sequencing). In some embodiments, a plurality of single molecule sequencing reactions are performed in parallel (e.g., on a single chip) according to aspects of the present application. For example, in some embodiments, a plurality of single molecule sequencing reactions are each performed in separate reaction chambers (e.g., nanoapertures, sample wells) on a single chip.

Embodiments are capable of sequencing single nucleic acid molecules with high accuracy and long read lengths, such as an accuracy of at least about 50%, 60%. 70%, 75%, 80%, 85%, 90%, 95%, 96%, 97%, 98%, 99%, 99.9%, 99.99%, 99.999%, or 99.9999%, and/or read lengths greater than or equal to about 10 base pairs (bp), 50 bp, 100 bp, 200 bp, 300 bp, 400 bp, 500 bp, 1000 bp, 10,000 bp, 20,000 bp, 30,000 bp, 40,000 bp, 50,000 bp, or 100,000 bp. In some embodiments, the target nucleic acid molecule used in single molecule sequencing is a single stranded target nucleic acid (e.g., deoxyribonucleic acid (DNA), DNA derivatives, ribonucleic acid (RNA), RNA derivatives) template that is added or immobilized to a sample well (e.g., nanoaperture) containing at least one additional component of a sequencing reaction (e.g., a polymerase such as, a DNA polymerase, a sequencing primer) immobilized or attached to a solid support such as the bottom or side walls of the sample well. The target nucleic acid molecule or the polymerase can be attached to a sample wall, such as at the bottom or side walls of the sample well directly or through a linker. The sample well (e.g., nanoaperture) also can contain any other reagents needed for nucleic acid synthesis via a primer extension reaction, such as, for example suitable buffers, co-factors, enzymes (e.g., a polymerase) and deoxyribonucleoside polyphosphates, such as, e.g., deoxyribonucleoside triphosphates, including deoxyadenosine triphosphate (dATP), deoxycytidine triphosphate (dCTP), deoxyguanosine triphosphate (dGTP), deoxyuridine triphosphate (dUTP) and deoxythymidine triphosphate (dTTP) dNTPs, that include luminescent tags, such as fluorophores. In some embodiments, each class of dNTPs (e.g., adenine-containing dNTPs (e.g., dATP), cytosine-containing dNTPs (e.g., dCTP), guanine-containing dNTPs (e.g., dGTP), uracil-containing dNTPs (e.g., dUTPs) and thymine-containing dNTPs (e.g., dTTP)) is conjugated to a distinct luminescent tag such that detection of light emitted from the tag indicates the identity of the dNTP that was incorporated into the newly synthesized nucleic acid. Emitted light from the luminescent tag can be detected and attributed to its appropriate luminescent tag (and, thus, associated dNTP) via any suitable device and/or method, including such devices and methods for detection described elsewhere herein. The luminescent tag may be conjugated to the dNTP at any position such that the presence of the luminescent tag does not inhibit the incorporation of the dNTP into the newly synthesized nucleic acid strand or the activity of the polymerase. In some embodiments, the luminescent tag is conjugated to the terminal phosphate (e.g., the gamma phosphate) of the dNTP.

In some embodiments, the single-stranded target nucleic acid template can be contacted with a sequencing primer, dNTPs, polymerase and other reagents necessary for nucleic acid synthesis. In some embodiments, all appropriate dNTPs can be contacted with the single-stranded target nucleic acid template simultaneously (e.g., all dNTPs are simultaneously present) such that incorporation of dNTPs can occur continuously. In other embodiments, the dNTPs can be contacted with the single-stranded target nucleic acid template sequentially, where the single-stranded target nucleic acid template is contacted with each appropriate dNTP separately, with washing steps in between contact of the single-stranded target nucleic acid template with differing dNTPs. Such a cycle of contacting the single-stranded target nucleic acid template with each dNTP separately followed by washing can be repeated for each successive base position of the single-stranded target nucleic acid template to be identified.

In some embodiments, the sequencing primer anneals to the single-stranded target nucleic acid template and the polymerase consecutively incorporates the dNTPs (or other deoxyribonucleoside polyphosphate) to the primer based on the single-stranded target nucleic acid template. The unique luminescent tag associated with each incorporated dNTP can be excited with the appropriate excitation light during or after incorporation of the dNTP to the primer and its emission can be subsequently detected, using, any suitable device(s) and/or method(s), including devices and methods for detection described elsewhere herein. Detection of a particular emission of light (e.g., having a particular emission lifetime, intensity, spectrum and/or combination thereof) can be attributed to a particular dNTP incorporated. The sequence obtained from the collection of detected luminescent tags can then be used to determine the sequence of the single-stranded target nucleic acid template via sequence complementarity.

While the present disclosure makes reference to dNTPs, devices, systems and methods provided herein may be used with various types of nucleotides, such as ribonucleotides and deoxyribonucleotides (e.g., deoxyribonucleoside polyphosphates with at least 4, 5, 6, 7, 8, 9, or 10 phosphate groups). Such ribonucleotides and deoxyribonucleotides can include various types of tags (or markers) and linkers. In some embodiments, the present disclosure provides methods and compositions that may be advantageously utilized in the technologies described in co-pending U.S. Patent App. Nos.: 14/543.865. 14/543,867, 14/543,888, 14/821,656, 14/821,686, 14/821,688, 15/161,067, 15/161,088, 15/161,125, 15/255,245, 15/255,303, 15/255.624, 15/261,697, 15/261,724, 62/289,019, 62/296,546, 62/310,398, 62/339,790, 62/343,997, 62/344,123, and 62/426,144, the contents of each of which are incorporated herein by reference.

### Example of Nucleic Acid Sequencing

The following example is meant to illustrate some of the methods, compositions and devices described herein. All aspects of the example are non-limiting. FIG. 1 schematically illustrates the setup of a single molecule nucleic acid sequencing method. 1-110 is a sample well (e.g., nanoaperture, reaction chamber) configured to contain a single complex comprising a nucleic acid polymerase 1-101, a target nucleic acid 1-102 to be sequenced, and a primer 1-104. In this example, a bottom region of sample well 1-110 is depicted as a target volume (e.g., the excitation region) 1-120.

As described elsewhere herein, the target volume is a volume towards which the excitation energy is directed. In some embodiments, the volume is a property of both the sample well volume and the coupling of excitation energy to the sample well. The target volume may be configured to limit the number of molecules or complexes confined in the target volume. In some embodiments, the target volume is configured to confine a single molecule or complex. In some embodiments, the target volume is configured to confine a single polymerase complex. In FIG. 1 the complex comprising polymerase 1-101 is confined in target volume 1-120. The complex may optionally be immobilized by attachment to a surface of the sample well. Exemplary processes for sample well surface preparation and functionalization are discussed in further detail elsewhere in the application. In this example the complex is immobilized by a linker 1-103 comprising one or more biomolecules (e.g., biotin) suitable for attaching the linker to the polymerase 1-101.

The volume of the aperture also contains a reaction mixture with suitable solvent, buffers, and other additives necessary for the polymerase complex to synthesize a nucleic acid strand. The reaction mixture also contains a plurality of types of luminescently labeled nucleotides. Each type of nucleotide is represented by the symbols * A, @ T, $-G, #-C, wherein A, T, G, and C represent the nucleotide base, and the symbols *, @, $, and # represent a unique luminescent label attached to each nucleotide, through linker -. In FIG. 1, a #-C' nucleotide is currently being incorporated into the complementary strand 1-102. The incorporated nucleotide is within the target volume 1-120.

FIG. 1 also indicates with arrows the concept of an excitation energy being delivered to a vicinity of the target volume, and a luminescence being emitted towards a detector. The arrows are schematic, and are not meant to indicate the particular orientation of excitation energy delivery or luminescence. In some embodiments, the excitation energy is a pulse of light from a light source. The excitation energy may travel through one or more device components, such as waveguides or filters, between the light source and the vicinity of the target volume. The emission energy may also travel through one or more device components, such as waveguides or filters, between the luminescent molecule and the detector. Some luminescences may emit on a vector which is not directed to the detector (e.g., towards the sidewall of the sample well) and may not be detected.

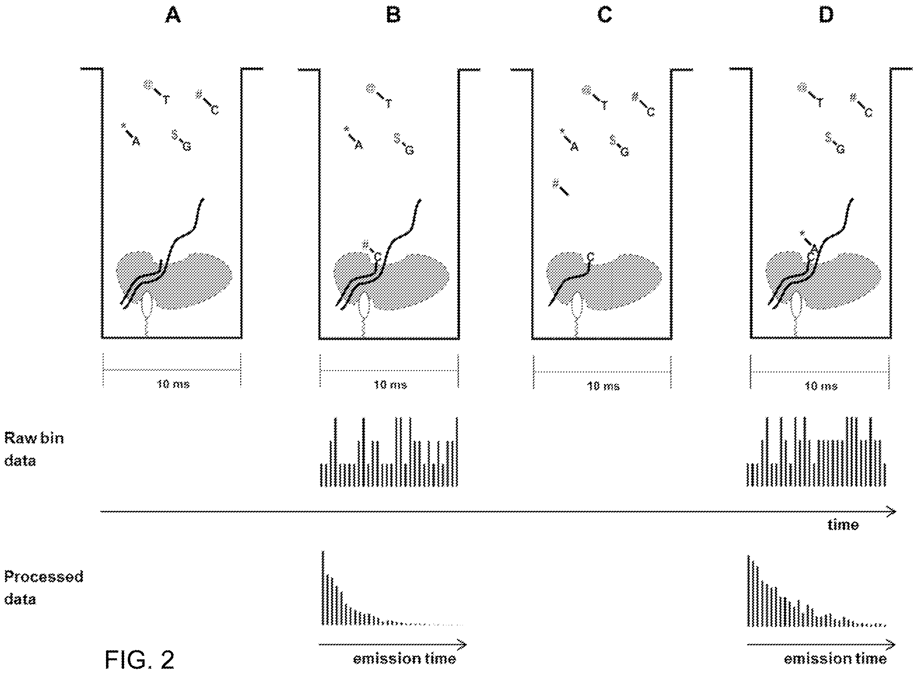

FIG. 2 schematically illustrates a sequencing process in a single sample well (e.g., a nanoaperture) over time. Stages A through D depict a sample well with a polymerase complex as in FIG. 1. Stage A depicts the initial state before any nucleotides have been added to the primer. Stage B depicts the incorporation event of a luminescently labeled nucleotide (#-C). Stage C depicts the period between incorporation events. In this example, nucleotide C has been added to the primer, and the label and linker previously attached to the luminescently labeled nucleotide (#-C) has been cleaved. Stage D depicts a second incorporation event of a luminescently labeled nucleotide (*-A). The complementary strand after Stage D consists of the primer, a C nucleotide, and an A nucleotide.

Stage A and C, both depict the periods before or between incorporation events, which are indicated in this example to last for about 10 milliseconds. In stages A and C, because there is no nucleotide being incorporated, there is no luminescently labeled nucleotide in the target volume (not drawn in FIG. 2), though background luminescence or spurious luminescence from luminescently labeled nucleotide which is not being incorporated may be detected. Stage B and D show incorporation events of different nucleotides (#-C, and *-A, respectively). In this example these events are also indicated to last for about 10 milliseconds.

The row labeled "Raw bin data'' depicts the data generated during each Stage. Throughout the example experiment, a plurality of pulses of light are delivered to the vicinity of the target volume. For each pulse a detector is configured to record any emitted photon received by the detector. When an emitted photon is received by the detector it is separated into one of a plurality of time bins, of which there are 3 in this example. In some embodiments, the detector is configured with between 2 and 16 time bins. The "Raw bin data" records a value of 1 (shortest bars), 2 (medium bars), or 3 (longest bars), corresponding to the shortest, middle, and longest bins, respectively. Each bar indicates detection of an emitted photon.

Since there is no luminescently labeled nucleotide present in the target volume for Stage A or C, there are no photons detected. For each of Stage B and D a plurality of photon emission events (luminescent events or "luminescences" as used herein) is detected during the incorporation event. Luminescent label # has a shorter luminescence lifetime than luminescent label *. The Stage B data is thus depicted as having recorded lower average bin values, than Stage D where the bin values are higher.

The row labeled "Processed data" depicts raw data which has been processed to indicate the number (counts) of emitted photons at times relative to each pulse. Since each bar corresponds to the photon count of a particular time bin, the exemplary curves depicting processed data correspond to raw bin data comprising more time bins than the three time bins described in the figure. In this example, the data is only processed to determine luminescent lifetime, but the data may also be evaluated for other luminescent properties, such as luminescent intensity or the wavelength of the absorbed or emitted photons. The exemplary processed data approximates an exponential decay curve characteristic for the luminescence lifetime of the luminescent label in the target volume. Because luminescent label # has a shorter luminescence lifetime than luminescent label *, the processed data for Stage B has fewer counts at longer time durations, while the processed data for Stage D has relatively more counts at longer time durations.

The example experiment of FIG. 2 would identify the first two nucleotides added to the complementary strand as CA. For DNA, the sequence of the target strand immediately after the region annealed to the primer would thus be identified as GT. In this example the nucleotides C and A could be distinguished from amongst the plurality of C, G, T, and A, based on luminescent lifetime alone. In some embodiments, other properties, such as the luminescent intensity or the wavelength of the absorbed or emitted photons may be necessary to distinguish one or more particular nucleotide.

Signals emitted upon the incorporation of nucleotides can be stored in memory and processed at a later point in time to determine the sequence of the target nucleic acid template. This may include comparing the signals to a reference signals to determine the identities of the incorporated nucleotides as a function of time. Alternatively or in addition to, signal emitted upon the incorporation of nucleotide can be collected and processed in real time (e.g., upon nucleotide incorporation) to determine the sequence of the target nucleic acid template in real time.

The term "nucleic acid," as used herein, generally refers to a molecule comprising one or more nucleic acid subunits. A nucleic acid may include one or more subunits selected from adenine (A), cytosine (C), guanine (G), thymine (T), and uracil (U), or variants thereof. In some examples, a nucleic acid is deoxyribonucleic acid (DNA) or ribonucleic acid (RNA), or derivatives thereof. A nucleic acid may be single-stranded or double stranded. A nucleic acid may be circular.

The term "nucleotide." as used herein, generally refers to a nucleic acid subunit, which can include A, C, G, T or U, or variants or analogs thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (e.g., A or G, or variant or analogs thereof) or a pyrimidine (e.g., C, T or U, or variant or analogs thereof). A subunit can enable individual nucleic acid bases or groups of bases (e.g., AA, TA, AT, GC, CG, CT, TC, GT, TG, AC, CA, or uracil-counterparts thereof) to be resolved.

A nucleotide generally includes a nucleoside and at least 1, 2, 3, 4, 5, 6, 7, 8, 9, 10, or more phosphate (PO₃) groups. A nucleotide can include a nucleobase, a five-carbon sugar (either ribose or deoxyribose), and one or more phosphate groups. Ribonucleotides are nucleotides in which the sugar is ribose. Deoxyribonucleotides are nucleotides in which the sugar is deoxyribose. A nucleotide can be a nucleoside monophosphate or a nucleoside polyphosphate. A nucleotide can be a deoxyribonucleoside polyphosphate, such as, e.g., a deoxyribonucleoside triphosphate, which can be selected from deoxyadenosine triphosphate (dATP), deoxycytidine triphosphate (dCTP), deoxyguanosine triphosphate (dGTP), deoxyuridine triphosphate (dUTP) and deoxythymidine triphosphate (dTTP) dNTPs, that include detectable tags, such as luminescent tags or markers (e.g., fluorophores).

A nucleoside polyphosphate can have 'n' phosphate groups, where 'n' is a number that is greater than or equal to 2, 3, 4, 5, 6, 7, 8, 9, or 10. Examples of nucleoside polyphosphates include nucleoside diphosphate and nucleoside triphosphate. A nucleotide can be a terminal phosphate labeled nucleoside, such as a terminal phosphate labeled nucleoside polyphosphate. Such label can be a luminescent (e.g., fluorescent or chemiluminescent) label, a fluorogenic label, a colored label, a chromogenic label, a mass tag, an electrostatic label, or an electrochemical label. A label (or marker) can be coupled to a terminal phosphate through a linker. The linker can include, for example, at least one or a plurality of hydroxyl groups, sulfhydryl groups, amino groups or haloalkyl groups, which may be suitable for forming, for example, a phosphate ester, a thioester, a phosphoramidate or an alkyl phosphonate linkage at the terminal phosphate of a natural or modified nucleotide. A linker can be cleavable so as to separate a label from the terminal phosphate, such as with the aid of a polymerization enzyme. Examples of nucleotides and linkers are provided in U.S. Patent No. 7.041,812, which is entirely incorporated herein by reference.

A nucleotide (e.g., a nucleotide polyphosphate) can comprise a methylated nucleobase. For example, a methylated nucleotide can be a nucleotide that comprises one or more methyl groups attached to the nucleobase (e.g.. attached directly to a ring of the nucleobase, attached to a substituent of a ring of the nucleobase). Exemplary methylated nucleobases include 1-methylthymine, 1-methyluracil, 3-methyluracil, 3-methylcytosine, 5-methylcytosine, 1-methyladenine, 2-methyladenine, 7-methyladenine, N6-methyladenine, N6,N6-dimethyladenine, 1-methylguanine, 7-methylguanine, N2-methylguanine, and N2,N2-dimethylguanine.

The term "primer," as used herein, generally refers to a nucleic acid molecule (e.g., an oligonucleotide), which can include a sequence comprising A, C, G, T and/or U, or variants or analogs thereof. A primer can be a synthetic oligonucleotide comprising DNA, RNA, PNA, or variants or analogs thereof. A primer can be designed such that its nucleotide sequence is complementary to a target strand, or the primer can comprise a random nucleotide sequence. In some embodiments, a primer can comprise a tail (e.g., a poly-A tail, an index adaptor, a molecular barcode, etc.). In some embodiments, a primer can comprise 5 to 15 bases, 10 to 20 bases, 15 to 25 bases, 20 to 30 bases, 25 to 35 bases, 30 to 40 bases, 35 to 45 bases, 40 to 50 bases, 45 to 55 bases, 50 to 60 bases, 55 to 65 bases, 60 to 70 bases, 65 to 75 bases, 70 to 80 bases, 75 to 85 bases, 80 to 90 bases, 85 to 95 bases, 90 to 100 bases, 95 to 105 bases. 100 to 150 bases, 125 to 175 bases. 150 to 200 bases, or more than 200 bases.

### Luminescent Properties

As described herein, a luminescent molecule is a molecule that absorbs one or more photons and may subsequently emit one or more photons after one or more time durations. The luminescence of the molecule is described by several parameters, including but not limited to luminescent lifetime, absorption spectra, emission spectra, luminescent quantum yield, and luminescent intensity. The terms absorption and excitation are used interchangeably throughout the application. A typical luminescent molecule may absorb, or undergo excitation by, light at multiple wavelengths. Excitation at certain wavelengths or within certain spectral ranges may relax by a luminescent emission event, while excitation at certain other wavelengths or spectral ranges may not relax by a luminescent emission event. In some embodiments, a luminescent molecule is only suitably excited for luminescence at a single wavelength or within a single spectral range. In some embodiments, a luminescent molecule is suitably excited for luminescence at two or more wavelengths or within two or more spectral ranges. In some embodiments, a molecule is identified by measuring the wavelength of the excitation photon or the absorption spectrum.

The emitted photon from a luminescent emission event will emit at a wavelength within a spectral range of possible wavelengths. Typically the emitted photon has a longer wavelength (e.g., has less energy or is red-shifted) compared to the wavelength of the excitation photon. In certain embodiments, a molecule is identified by measuring the wavelength of an emitted photon. In certain embodiments, a molecule is identified by measuring the wavelength of a plurality of emitted photon. In certain embodiments, a molecule is identified by measuring the emission spectrum.

Luminescent lifetime refers to the time duration between an excitation event and an emission event. In some embodiments, luminescent lifetime is expressed as the constant in an equation of exponential decay. In some embodiments, wherein there are one or more pulse events delivering excitation energy, the time duration is the time between the pulse and the subsequent emission event.

"Determining a luminescent lifetime" of a molecule can be performed using any suitable method (e.g., by measuring the lifetime using a suitable technique or by determining time-dependent characteristics of emission). In some embodiments, determining the luminescent lifetime of a molecule comprises determining the lifetime relative to one or more molecules (e.g., different luminescently labeled nucleotides in a sequencing reaction). In some embodiments, determining the luminescent lifetime of a molecule comprises determining the lifetime relative to a reference. In some embodiments, determining the luminescent lifetime of a molecule comprises measuring the lifetime (e.g., fluorescence lifetime). In some embodiments, determining the luminescent lifetime of a molecule comprises determining one or more temporal characteristics that are indicative of lifetime. In some embodiments, the luminescent lifetime of a molecule can be determined based on a distribution of a plurality of emission events (e.g., 1, 2, 3, 4, 5, 6, 7, 8, 9, 10, 11, 12, 13, 14, 15, 16, 17, 18, 19, 20, 30, 40, 50, 60, 70, 80, 90, 100, or more emission events) occurring across one or more time-gated windows relative to an excitation pulse. For example, a luminescent lifetime of a single molecule can be distinguished from a plurality of molecules having different luminescent lifetimes based on the distribution of photon arrival times measured with respect to an excitation pulse.

It should be appreciated that a luminescent lifetime of a single molecule is indicative of the timing of photons emitted after the single molecule reaches an excited state and the single molecule can be distinguished by information indicative of the timing of the photons. Some embodiments may include distinguishing a molecule from a plurality of molecules based on the molecule's luminescent lifetime by measuring times associated with photons emitted by the molecule. The distribution of times may provide an indication of the luminescent lifetime which may be determined from the distribution. In some embodiments, the single molecule is distinguishable from the plurality of molecules based on the distribution of times, such as by comparing the distribution of times to a reference distribution corresponding to a known molecule. In some embodiments, a value for the luminescent lifetime is determined from the distribution of times.

Luminescent quantum yield refers to the fraction of excitation events at a given wavelength or within a given spectral range that lead to an emission event, and is typically less than 1. In some embodiments, the luminescent quantum yield of a molecule described herein is between 0 and about 0.001, between about 0.001 and about 0.01, between about 0.01 and about 0.1, between about 0.1 and about 0.5, between about 0.5 and 0.9, or between about 0.9 and 1. In some embodiments, a molecule is identified by determining or estimating the luminescent quantum yield.

As used herein for single molecules, luminescent intensity refers to the number of emitted photons per unit time that are emitted by a molecule which is being excited by delivery of a pulsed excitation energy. In some embodiments, the luminescent intensity refers to the detected number of emitted photons per unit time that are emitted by a molecule which is being excited by delivery of a pulsed excitation energy, and are detected by a particular sensor or set of sensors.

The luminescent lifetime, luminescent quantum yield, and luminescent intensity may each vary for a given molecule under different conditions. In some embodiments, a single molecule will have a different observed luminescent lifetime, luminescent quantum yield, or luminescent intensity than for an ensemble of the molecules. In some embodiments, a molecule confined in a sample well (e.g., a nanoaperture) will have a different observed luminescent lifetime, luminescent quantum yield, or luminescent intensity than for molecules not confined in a sample well. In some embodiments, a luminescent label or luminescent molecule attached to another molecule will have a different luminescent lifetime, luminescent quantum yield, or luminescent intensity than the luminescent label or luminescent molecule not attached to another molecule. In some embodiments, a molecule interacting with a macromolecular complex (e.g., protein complex (e.g., nucleic acid polymerase)) will have different luminescent lifetime, luminescent quantum yield, or luminescent intensity than a molecule not interacting with a macromolecular complex.

In certain embodiments, a luminescent molecule described in the application absorbs one photon and emits one photon after a time duration. In some embodiments, the luminescent lifetime of a molecule can be determined or estimated by measuring the time duration. In some embodiments, the luminescent lifetime of a molecule can be determined or estimated by measuring a plurality of time durations for multiple pulse events and emission events. In some embodiments, the luminescent lifetime of a molecule can be differentiated amongst the luminescent lifetimes of a plurality of types of molecules by measuring the time duration. In some embodiments, the luminescent lifetime of a molecule can be differentiated amongst the luminescent lifetimes of a plurality of types of molecules by measuring a plurality of time durations for multiple pulse events and emission events. In certain embodiments, a molecule is identified or differentiated amongst a plurality of types of molecules by determining or estimating the luminescent lifetime of the molecule. In certain embodiments, a molecule is identified or differentiated amongst a plurality of types of molecules by differentiating the luminescent lifetime of the molecule amongst a plurality of the luminescent lifetimes of a plurality of types of molecules.

In certain embodiments, the luminescent emission event is a fluorescence. In certain embodiments, the luminescent emission event is a phosphorescence. As used herein, the term luminescence encompasses all luminescent events including both fluorescence and phosphorescence.

In one aspect, the application provides a method of determining the luminescent lifetime of a single luminescent molecule comprising: providing the luminescent molecule in a target volume, delivering a plurality of pulses of an excitation energy to a vicinity of the target volume; and detecting a plurality of luminescences from the luminescent molecule. In some embodiments, the method further comprises evaluating the distribution of the plurality of time durations between each pair of pulses and luminescences. In some embodiments, the method further comprises immobilizing the single luminescent molecule in the target volume.

In another aspect, the application provides a method of determining the luminescent lifetime of a plurality of molecules comprising: providing a plurality of luminescent molecules in a target volume; delivering a plurality of pulses of an excitation energy to a vicinity of the target volume; and detecting a plurality of luminescences from the luminescent molecules. In some embodiments, the method further comprises evaluating the distribution of the plurality of time durations between each pair of pulses and luminescences. In some embodiments, the method further comprises immobilizing the luminescent molecules in the target volume. In some embodiments, the plurality consists of between 2 and about 10 molecules, between about 10 and about 100 molecules, or between about 100 and about 1000 molecules. In some embodiments, the plurality consists of between about 1000 and about 10⁶ molecules , between about 10⁶ and about 10⁹ molecules, between about 10⁹ and about 10¹² molecules, between about 10¹² and about 10¹⁵ molecules, or between about 10¹⁵ and about 10¹⁸ molecules. In some embodiments, all molecules of the plurality are the same type of molecule.

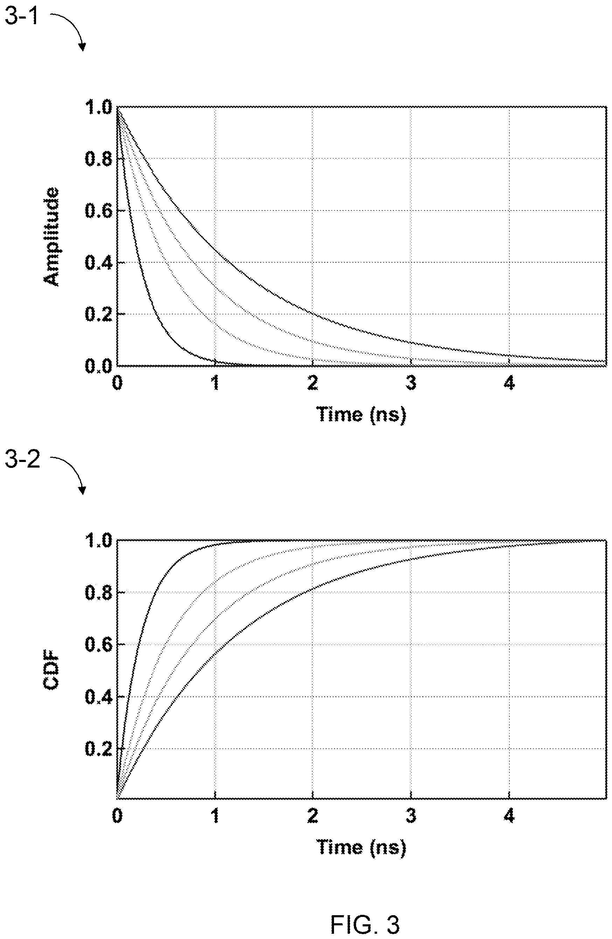

FIG. 3 shows the exemplary decay profile 3-1 for four luminescent molecules with different luminescent lifetimes (longest to shortest, top to bottom). The amplitude can refer to the intensity of luminescence from a sample comprising many molecules, which decreases exponentially over time after the initial excitation based on the luminescent lifetime. The amplitude can alternatively refer to a number or count of emissions detected after a time duration after a plurality of pulses of excitation energy, for example, for a single molecule. The normalized cumulative distribution function 3-2 corresponds to 3-1, for four luminescent molecules with different luminescent lifetimes (shortest to longest, top to bottom). The CDF can represent the normalized probability of the luminescence amplitude of reaching zero (e.g., the cumulative probability of all excited molecules having luminesced) over time after the initial excitation based on the luminescent lifetime. The CDF can alternatively represent the normalized probability of a single molecule emitting luminescence at a certain time duration after a single pulse or after each of a plurality of pulses of excitation energy.

In one aspect, the application provides a method of determining the luminescent intensity of a single luminescent molecule comprising: providing the luminescent molecule in a target volume; delivering a plurality of pulses of an excitation energy to a vicinity of the target volume; and detecting a plurality of luminescences from the luminescent molecule. In some embodiments, the method further comprises determining the number of the plurality of detected luminescence per unit time. In some embodiments, the method further comprises immobilizing the single luminescent molecule in the target volume.

In another aspect, the application provides a method of determining the luminescent intensity of a plurality of molecules comprising: providing a plurality of luminescent molecules in a target volume; delivering a plurality of pulses of an excitation energy to a vicinity of the target volume; and detecting a plurality of luminescences from the luminescent molecules. In some embodiments, the method further comprises determining the number of the plurality of detected luminescence per unit time. In some embodiments, the method further comprises immobilizing the luminescent molecules in the target volume. In some embodiments, the plurality consists of between 2 and about 10 molecules, between about 10 and about 100 molecules, or between about 100 and about 1000 molecules. In some embodiments, the plurality consists of between about 1000 and about 10⁶ molecules , between about 10⁶ and about 10⁹ molecules, between about 10⁹ and about 10¹² molecules, between about 10¹² and about 10¹⁵ molecules, or between about 10¹⁵ and about 10¹⁸ molecules. In some embodiments, all molecules of the plurality are the same type of molecule.

### Excitation Energy

In one aspect of methods described herein, one or more excitation energy is used to excite the luminescent labels of the molecules to be identified or distinguished (e.g., during a sequencing reaction). In some embodiments, an excitation energy is in the visible spectrum. In some embodiments, an excitation energy is in the ultraviolet spectrum. In some embodiments, an excitation energy is in the infrared spectrum. In some embodiments, one excitation energy is used to excite the luminescently labeled molecules. In some embodiments, two excitation energies are used to excite the luminescently labeled molecules. In some embodiments, three or more excitation energies are used to excite the luminescently labeled molecules. In some embodiments, each luminescently labeled molecule is excited by only one of the delivered excitation energies. In some embodiments, a luminescently labeled molecule is excited by two or more of the delivered excitation energies. In certain embodiments, an excitation energy may be monochromatic or confined to a spectral range. In some embodiments, a spectral range has a range of between about 0.1 nm and about 1 nm, between about 1 nm and about 2 nm, or between about 2 nm and about 5 nm. In some embodiments a spectral range has a range of between about 5 nm and about 10 nm, between about 10 nm and about 50 nm, or between about 50 nm and about 100 nm.

In certain embodiments, excitation energy is delivered as a pulse of light. In certain embodiments, excitation energy is delivered as a plurality of pulses of light. In certain embodiments, two or more excitation energies are used to excite the luminescently labeled molecules. In some embodiments, each excitation energy is delivered at the same time (e.g., in each pulse). In some embodiments, each excitation energy is delivered at different times (e.g., in separate pulses of each energy). The different excitation energies may be delivered in any pattern sufficient to allow detection of luminescence from the target molecules. In some embodiments, two excitation energies are delivered in each pulse. In some embodiments, a first excitation energy and a second excitation energy are delivered in alternating pulses. In some embodiments, a first excitation energy is delivered in a series of sequential pulses, and a second excitation energy is delivered in a subsequent series of sequential pulses, or an alternating pattern of such series.

In certain embodiments, the frequency of pulses of light is selected based on the luminescent properties of the luminescently labeled molecule. In certain embodiments, the frequency of pulses of light is selected based on the luminescent properties of a plurality of luminescently labeled nucleotides. In certain embodiments, the frequency of pulses of light is selected based on the luminescent lifetime of a plurality of luminescently labeled nucleotides. In some embodiments, the frequency is selected so that the gap between pulses is longer than the luminescent lifetimes of one or more luminescently labeled nucleotides. In some embodiments, the frequency is selected based on the longest luminescent lifetime of the plurality of luminescently labeled nucleotides. For example, if the luminescent lifetimes of the four luminescently labeled nucleotides are 0.25, 0.5, 1.0, and 1.5 ns, the frequency of pulses of light may be selected so that the gap between pulses exceeds 1.5 ns. In some embodiments, the gap is between about two times and about ten times, between about ten times and about 100 times, or between about 100 times and about 1000 times longer than the luminescent lifetime of one or more luminescently labeled molecules being excited. In some embodiments, the gap is about 10 times longer than the luminescent lifetime of one or more luminescently labeled molecules being excited. In some embodiments, the gap is between about 0.01 ns and about 0.1 ns, between about 1 ns and about 5 ns, between about 5 ns and about 15 ns, between about 15 ns and about 25 ns, or between about 25 ns and about 50 ns. In some embodiments, the gap is selected such that there is a 50%, 75%, 90%, 95%, or 99% probability that the molecules excited by the pulse will luminescently decay or that the excited state will relax by another mechanism.

In certain embodiments, wherein there are multiple excitation energies, the frequency of the pulses for each excitation energy is the same. In certain embodiments, wherein there are multiple excitation energies, the frequencies of the pulses for each excitation energy is different. For example, if a red laser is used to excite luminescent molecules with lifetimes of 0.2 and 0.5 ns, and a green laser is used to excite luminescent molecules with lifetimes of 5 ns and 7 ns, the gap after each red laser pulse may be shorter (e.g., 5 ns) than the gap after each green laser pulse (e.g., 20 ns).

In certain embodiments, the frequency of pulsed excitation energies is selected based on the chemical process being monitored. For a sequencing reaction the frequency may be selected such that a number of pulses are delivered sufficient to allow for detection of a sufficient number of emitted photons to be detected. A sufficient number, in the context of detected photons, refers to a number of photons necessary to identify or distinguish the luminescently labeled nucleotide from the plurality of luminescently labeled nucleotides. For example, a DNA polymerase may incorporate an additional nucleotide once every 20 milliseconds on average. The time that a luminescently labeled nucleotide interacts with the complex may be about 10 milliseconds, and the time between when the luminescent marker is cleaved and the next luminescently labeled nucleotide begins to interact may be about 10 milliseconds. The frequency of the pulsed excitation energy could then be selected to deliver sufficient pulses over 10 milliseconds such that a sufficient number of emitted photons are detected during the 10 millisecond when the luminescently labeled nucleotide is being incorporated. For example, at a frequency of 100 MHz, there will be 1 million pulses in 10 milliseconds (the approximate length of the incorporation event). If 0.1% of these pulses leads to a detected photon there will be 1,000 luminescent data points that can be analyzed to determine the identity of the luminescently labeled nucleotide being incorporated. Any of the above values are non-limiting. In some embodiments incorporation events may take between 1 ms and 20 ms, between 20 ms and 100 ms, or between 100 ms and 500 ms. In some embodiments, in which multiple excitation energies are delivered in separately timed pulses the luminescently labeled nucleotide may only be excited by a portion of the pulses. In some embodiments, the frequency and pattern of the pulses of multiple excitation energies is selected such that the number of pulses is sufficient to excite any one of the plurality of luminescently labeled nucleotides to allow for a sufficient number of emitted photons to be detected.

In some embodiments, the frequency of pulses is between about 1 MHz and about 10 MHz. In some embodiments, the frequency of pulses is between about 10 MHz and about 100 MHz. In some embodiments, the frequency of pulses is between about 100 MHz and about 1 GHz. In some embodiments, the frequency of pulses is between about 50 MHz and about 200 MHz. In some embodiments, the frequency of pulses is about 100 MHz. In some embodiments, the frequency is stochastic.

In certain embodiments, the excitation energy is between about 500 nm and about 700 nm. In some embodiments, the excitation energy is between about 500 nm and about 600 nm, or about 600 nm and about 700 nm. In some embodiments, the excitation energy is between about 500 nm and about 550 nm, between about 550 nm and about 600 nm, between about 600 nm and about 650 nm, or between about 650 nm and about 700 nm.

In certain embodiments, a method described herein comprises delivery of two excitation energies. In some embodiments, the two excitation energies are separated by between about 5 nm and about 20 nm, between about 20 nm and about 40 nm, between about 40 nm and about 60 nm , between about 60 nm and about 80 nm, between about 80 nm and about 100 nm, between about 100 nm and about 150 nm, between about 150 nm and about 200 nm, between about 200 nm and about 400 nm, or between at least about 400 nm. In some embodiments, the two excitation energies are separated by between about 20 nm and about 80 nm, or between about 80 nm and about 160 nm.

When an excitation energy is referred to as being in a specific range, the excitation energy may comprise a single wavelength, such that the wavelength is between or at the endpoints of the range, or the excitation energy may comprise a spectrum of wavelengths with a maximum intensity, such that the maximum intensity is between or at the endpoints of the range.

In certain embodiments, the first excitation energy is in the range of 450 nm to 500 nm and the second excitation energy is in the range of 500 nm to 550 nm, 550 nm to 600 nm, 600 nm to 650 nm, or 650 nm to 700 nm. In certain embodiments, the first excitation energy is in the range of 500 nm to 550 nm and the second excitation energy is in the range of 450 nm to 500 nm, 550 nm to 600 nm. 600 nm to 650 nm, or 650 nm to 700 nm. In certain embodiments, the first excitation energy is in the range of 550 nm to 600 nm and the second excitation energy is in the range of 450 nm to 500 nm, 500 nm to 550 nm, 600 nm to 650 nm, or 650 nm to 700 nm. In certain embodiments, the first excitation energy is in the range of 600 nm to 650 nm and the second excitation energy is in the range of 450 nm to 500 nm , 500 nm to 550 nm. 550 nm to 600 nm, or 650 nm to 700 nm. In certain embodiments, the first excitation energy is in the range of 650 nm to 700 nm and the second excitation energy is in the range of 450 nm to 500 nm , 500 nm to 550 nm, 550 nm to 600 nm, or 600 nm to 650 nm.

In certain embodiments, the first excitation energy is in the range of 450 nm to 500 nm and the second excitation energy is in the range of 500 nm to 550 nm. In certain embodiments, the first excitation energy is in the range of 450 nm to 500 nm and the second excitation energy is in the range of 550 nm to 600 nm. In certain embodiments, the first excitation energy is in the range of 450 nm to 500 nm and the second excitation energy is in the range of 600 nm to 670 nm. In certain embodiments, the first excitation energy is in the range of 500 nm to 550 nm and the second excitation energy is in the range of 550 nm to 600 nm. In certain embodiments, the first excitation energy is in the range of 500 nm to 550 nm and the second excitation energy is in the range of 600 nm to 670 nm. In certain embodiments, the first excitation energy is in the range of 550 nm to 600 nm and the second excitation energy is in the range of 600 nm to 670 nm. In certain embodiments, the first excitation energy is in the range of 470 nm to 510 nm and the second excitation energy is in the range of 510 nm to 550 nm. In certain embodiments, the first excitation energy is in the range of 470 nm to 510 nm and the second excitation energy is in the range of 550 nm to 580 nm. In certain embodiments, the first excitation energy is in the range of 470 nm to 510 nm and the second excitation energy is in the range of 580 nm to 620 nm. In certain embodiments, the first excitation energy is in the range of 470 nm to 510 nm and the second excitation energy is in the range of 620 nm to 670 nm. In certain embodiments, the first excitation energy is in the range of 510 nm to 550 nm and the second excitation energy is in the range of 550 nm to 580 nm. In certain embodiments, the first excitation energy is in the range of 510 nm to 550 nm and the second excitation energy is in the range of 580 nm to 620 nm. In certain embodiments, the first excitation energy is in the range of 510 nm to 550 nm and the second excitation energy is in the range of 620 nm to 670 nm. In certain embodiments, the first excitation energy is in the range of 550 nm to 580 nm and the second excitation energy is in the range of 580 nm to 620 nm. In certain embodiments, the first excitation energy is in the range of 550 nm to 580 nm and the second excitation energy is in the range of 620 nm to 670 nm. In certain embodiments, the first excitation energy is in the range of 580 nm to 620 nm and the second excitation energy is in the range of 620 nm to 670 nm.

Certain embodiments of excitation energy sources and devices for delivery of excitation energy pulses to a target volume are described elsewhere herein.

### Luminescently Labeled Nucleotides

In one aspect, methods and compositions described herein comprise one or more luminescently labeled nucleotides (e.g., one or more nucleoside polyphosphates connected to one or more labels via a nucleic acid linker comprising one or more protecting elements). In certain embodiments, one or more nucleotides comprise deoxyribose nucleosides. In some embodiments, all nucleotides comprises deoxyribose nucleosides. In certain embodiments, one or more nucleotides comprise ribose nucleosides. In some embodiments, all nucleotides comprise ribose nucleosides. In some embodiments, one or more nucleotides comprise a modified ribose sugar or ribose analog (e.g., a locked nucleic acid). In some embodiments, one or more nucleotides comprise naturally occurring bases (e.g., cytosine, guanine, adenine, thymine, uracil). In some embodiments, one or more nucleotides comprise derivatives or analogs of cytosine, guanine, adenine, thymine, or uracil.

In certain embodiments, a method comprises the step of exposing a polymerase complex to a plurality of luminescently labeled nucleotides. In certain embodiments, a composition or device comprises a reaction mixture comprising a plurality of luminescently labeled nucleotides. In some embodiments, the plurality of nucleotides comprises four types of nucleotides. In some embodiments, the four types of nucleotides each comprise one of cytosine, guanine, adenine, and thymine. In some embodiments, the four types of nucleotides each comprise one of cytosine, guanine, adenine, and uracil.

In certain embodiments, the concentration of each type of luminescently labeled nucleotide in the reaction mixture is between about 50 nM and about 200 nM, about 200 nM and about 500 nM, about 500 nM and about 1 µM, about 1µM and about 50 µM, or about 50 µM and 250 µM. In some embodiments, the concentration of each type of luminescently labeled nucleotide in the reaction mixture is between about 250 nM and about 2 µM. In some embodiments, the concentration of each type of luminescently labeled nucleotide in the reaction mixture is about 1 µM.

In certain embodiments, the reaction mixture contains additional reagents of use for sequencing reactions. In some embodiments, the reaction mixture comprises a buffer. In some embodiments, a buffer comprises 3-(*N*-morpholino)propanesulfonic acid (MOPS). In some embodiments, a buffer is present in a concentration of between about 1 mM and between about100 mM. In some embodiments, the concentration of MOPS is about 50 mM. In some embodiments, the reaction mixture comprises one or more salt. In some embodiments, a salt comprises potassium acetate. In some embodiments, the concentration of potassium acetate is about 140 mM. In some embodiments, a salt is present in a concentration of between about 1 mM and about 200 mM. In some embodiments, the reaction mixture comprises a magnesium salt (e.g., magnesium acetate). In some embodiments, the concentration of magnesium acetate is about 20 mM. In some embodiments, a magnesium salt is present in a concentration of between about 1 mM and about 50 mM. In some embodiments, the reaction mixture comprises a reducing agent. In some embodiments, a reducing agent is dithiothreitol (DTT). In some embodiments, a reducing agent is present in a concentration of between about 1 mM and about 50 mM. In some embodiments, the concentration of DTT is about 5 mM. In some embodiments, the reaction mixture comprises one or photostabilizers. In some embodiments, the reaction mixture comprises an anti-oxidant, oxygen scavenger, or triplet state quencher. In some embodiments, a photostabilizer comprises protocatechuic acid (PCA). In some embodiments, a photostabilizer comprises 4-nitrobenzyl alcohol (NBA). In some embodiments, a photostabilizer is present in a concentration of between about 0.1 mM and about 20 mM. In some embodiments, the concentration of PCA is about 3 mM. In some embodiments, the concentration of NBA is about 3 mM. A mixture with a photostabilizer (e.g., PCA) may also comprise an enzyme to regenerate the photostabilizer (e.g., protocatechuic acid dioxygenase (PCD)). In some embodiments, the concentration of PCD is about 0.3 mM.

The application contemplates different methods for differentiating nucleotides amongst a plurality of nucleotides. In certain embodiments, each of the luminescently labeled nucleotides has a different luminescent lifetime. In certain embodiments, two or more of the luminescently labeled nucleotides have the same luminescent lifetimes or substantially the same luminescent lifetimes (e.g., lifetimes that cannot be distinguished by the method or device).

In certain embodiments, each of the luminescently labeled nucleotides absorbs excitation energy in a different spectral range. In certain embodiments, two of the luminescently labeled nucleotides absorb excitation energy in the same spectral range. In certain embodiments, three of the luminescently labeled nucleotides absorb excitation energy in the same spectral range. In certain embodiments, four or more of the luminescently labeled nucleotides absorb excitation energy in the same spectral range. In certain embodiments, two of the luminescently labeled nucleotides absorb excitation energy a different spectral range. In certain embodiments, three of the luminescently labeled nucleotides absorb excitation energy a different spectral range. In certain embodiments, four or more of the luminescently labeled nucleotides absorb excitation energy a different spectral range.

In certain embodiments, each of the luminescently labeled nucleotides emits photons in a different spectral range. In certain embodiments, two of the luminescently labeled nucleotides emits photons in the same spectral range. In certain embodiments, three of the luminescently labeled nucleotides emits photons in the same spectral range. In certain embodiments, four or more of the luminescently labeled nucleotides emits photons in the same spectral range. In certain embodiments, two of the luminescently labeled nucleotides emits photons in the different spectral range. In certain embodiments, three of the luminescently labeled nucleotides emits photons in the different spectral range. In certain embodiments, four or more of the luminescently labeled nucleotides emits photons in the different spectral range.

In certain embodiments, each of four luminescently labeled nucleotides has a different luminescent lifetime. In certain embodiments, two or more luminescently labeled nucleotides have different luminescent lifetimes and absorb and/or emit photons in a first spectral range, and one or more luminescently labeled nucleotides absorb and/or emit photons in a second spectral range. In some embodiments, each of three luminescently labeled nucleotides has a different luminescent lifetime and emit luminescence in a first spectral range, and a fourth luminescently labeled nucleotide absorbs and/or emits photons in a second spectral range. In some embodiments, each of two luminescently labeled nucleotides has a different luminescent lifetime and emit luminescence in a first spectral range, and a third and fourth luminescently labeled nucleotide each have different luminescent lifetimes and emit luminescence in a second spectral range.

In certain embodiments, each of four luminescently labeled nucleotides has a different luminescent intensity. In certain embodiments, two or more luminescently labeled nucleotides have different luminescent intensity and emit luminescence in a first spectral range, and one or more luminescently labeled nucleotides absorbs and/or emits photons in a second spectral range. In some embodiments, each of three luminescently labeled nucleotides has a different luminescent intensity and emit luminescence in a first spectral range, and a fourth luminescently labeled nucleotide absorbs and/or emits photons in a second spectral range. In some embodiments, each of two luminescently labeled nucleotides has a different luminescent intensity and emit luminescence in a first spectral range, and a third and fourth luminescently labeled nucleotide each have different luminescent intensity and emit luminescence in a second spectral range.

In certain embodiments, each of four luminescently labeled nucleotides has a different luminescent lifetime or luminescent intensity. In certain embodiments, two or more luminescently labeled nucleotides have different luminescent lifetime or luminescent intensity and emit luminescence in a first spectral range, and one or more luminescently labeled nucleotides absorbs and/or emits photons in a second spectral range. In some embodiments, each of three luminescently labeled nucleotides has a different luminescent lifetime or luminescent intensity and emit luminescence in a first spectral range, and a fourth luminescently labeled nucleotide absorbs and/or emits photons in a second spectral range. In some embodiments, each of two luminescently labeled nucleotides has a different luminescent lifetime or luminescent intensity and emit luminescence in a first spectral range, and a third and fourth luminescently labeled nucleotide each have different luminescent lifetime or luminescent intensity and emit luminescence in a second spectral range.

In certain embodiments, two or more luminescently labeled nucleotides have different luminescent lifetimes and absorb excitation energy in a first spectral range, and one or more luminescently labeled nucleotides absorbs excitation energy in a second spectral range. In some embodiments, each of three luminescently labeled nucleotides has a different luminescent lifetime and absorb excitation energy in a first spectral range, and a fourth luminescently labeled nucleotide absorbs excitation energy in a second spectral range. In some embodiments, each of two luminescently labeled nucleotides has a different luminescent lifetime and absorb excitation energy in a first spectral range, and a third and fourth luminescently labeled nucleotide each have different luminescent lifetimes and absorb excitation energy in a second spectral range.

In certain embodiments, two or more luminescently labeled nucleotides have different luminescent lifetime or luminescent intensity and absorb excitation energy in a first spectral range, and one or more luminescently labeled nucleotides absorbs excitation energy in a second spectral range. In some embodiments, each of three luminescently labeled nucleotides has a different luminescent lifetime or luminescent intensity and absorb excitation energy in a first spectral range, and a fourth luminescently labeled nucleotide absorbs excitation energy in a second spectral range. In some embodiments, each of two luminescently labeled nucleotides has a different luminescent lifetime or luminescent intensity and absorb excitation energy in a first spectral range, and a third and fourth luminescently labeled nucleotide each have different luminescent lifetime or luminescent intensity and absorb excitation energy in a second spectral range.

During sequencing the method of identifying a nucleotide may vary between various base pairs in the sequence. In certain embodiments, two types of nucleotides may be labeled to absorb at a first excitation energy, and those two types of nucleotides (e.g., A, Ci) are distinguished based on different luminescent intensity, whereas two additional types of nucleotides (e.g., C, T) may be labeled to absorb at a second excitation energy, and those two additional types of nucleotides are distinguished based on different luminescent lifetime. For such an embodiment, during sequencing certain segments of the sequence may be determined only based on luminescent intensity (e.g., segments incorporating only A and G), whereas other segments of the sequence may be determined only based on luminescent lifetime (e.g., segments incorporating only C and T). In some embodiments, between 2 and 4 luminescently labeled nucleotide are be differentiated based on luminescent lifetime. In some embodiments, between 2 and 4 luminescently labeled nucleotides are differentiated based on luminescent intensity. In some embodiments, between 2 and 4 luminescently labeled nucleotides are differentiated based on luminescent lifetime and luminescent intensity.

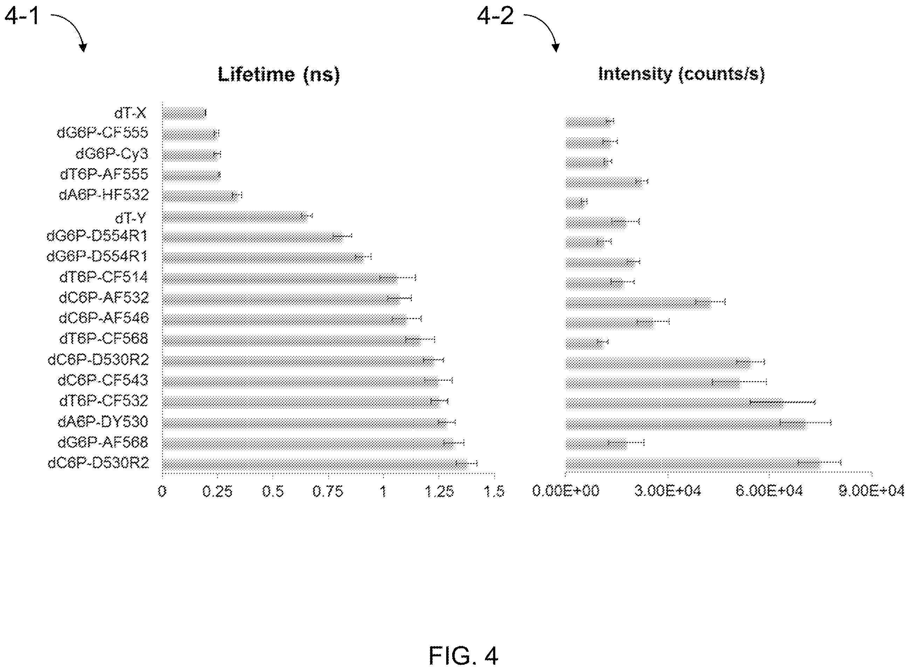

FIG. 4 shows the luminescent lifetime 4-1 of exemplary luminescently labeled nucleotides and the luminescent intensity 4-2 for the same exemplary nucleotides. For example, the fourth row shows data for a deoxythymidine hexaphosphate (dT6P) nucleotide linked to the fluorophore Alexa Fluor^{®} 555 (AF555). This luminescently labeled nucleotide has a lifetime of approximately 0.25 ns and displays a luminescent intensity of approximately 20000 counts/s. The observed luminescent lifetime and luminescent intensity of any luminescently labeled nucleotide may, in general, differ for the nucleotide under incorporation conditions (e.g., in a single molecule complex, in a nanoaperture) versus other more typical conditions such as those for 4-1 and 4-2.

### Luminescence Detection

In one aspect of methods described herein, an emitted photon (a luminescence) or a plurality of emitted photons is detected by one or more sensors. For a plurality of luminescently labeled molecules or nucleotides, each of the molecules may emit photons in a single spectral range, or a portion of the molecules may emit photons in a first spectral range and another portion of molecules may emit photons in a second spectral range. In certain embodiments, the emitted photons are detected by a single sensor. In certain embodiments, the emitted photons are detected by multiple sensors. In some embodiments, the photons emitted in a first spectral range are detected by a first sensor, and the photons emitted in a second spectral range are detected by a second sensor. In some embodiments, the photons emitted in each of a plurality of spectral ranges are detected by a different sensor.

In certain embodiments, each sensor is configured to assign a time bin to an emitted photon based on the time duration between the excitation energy and the emitted photon. In some embodiments, photons emitted after a shorter time duration will be assigned an earlier time bin, and photons emitted after a longer duration will be assigned a later time bin.

In some embodiments, a plurality of pulses of excitation energy is delivered to vicinity of a target volume and a plurality of photons, which may include photon emission events, are detected. In some embodiments, the plurality of luminescences (e.g., photon emission events) correspond to incorporation of a luminescently labeled nucleotide into a nucleic acid product. In some embodiments, the incorporation of a luminescently labeled nucleotide lasts for between about 1 ms and about 5 ms, between about 5 ms and about 20 ms, between about 20 ms and about 100 ms, or between about 100 ms and about 500 ms. In some embodiments, between about 10 and about 100, between about 100 and about 1000, about 1000 and about 10000, or about 10000 and about 100000 luminescences are detected during incorporation of a luminescently labeled nucleotide.

In certain embodiments, there are no luminescences detected if a luminescently labeled nucleotide is not being incorporated. In some embodiments, there is a luminescence background. In some embodiments, spurious luminescences are detected when no luminescently labeled nucleotide is being incorporated. Such spurious luminescences may occur if one or more luminescently labeled nucleotides is in the target volume (e.g., diffuses into the target volume, or interacts with polymerase but is not incorporated) during a pulse of excitation energy, but is not being incorporated by the sequencing reaction. In some embodiments, the plurality of luminescences detected from a luminescently labeled nucleotide in the target volume but not being incorporated is smaller (e.g.. ten times, 100 times, 1000 times, 10000 times) than the plurality of luminescences from a luminescently labeled nucleotide.

In some embodiments, for each plurality of detected luminescences corresponding to incorporation of a luminescently labeled nucleotide the luminescences are assigned a time bin based on the time duration between the pulse and the emitted photon. This plurality for an incorporation event is referred to herein as a "burst". In some embodiments, a burst refers to a series of signals (e.g., measurements) above a baseline (e.g., noise threshold value), wherein the signals correspond to a plurality of emission events that occur when the luminescently labeled nucleotide is within the excitation region. In some embodiments, a burst is separated from a preceding and/or subsequent burst by a time interval of signals representative of the baseline. In some embodiments, the burst is analyzed by determining the luminescent lifetime based on the plurality of time durations. In some embodiments, the burst is analyzed by determining the luminescent intensity based on the number of detected luminescences per a unit of time. In some embodiments, the burst is analyzed by determining the spectral range of the detected luminescences. In some embodiments, analyzing the burst data will allow assignment of the identity of the incorporated luminescently labeled nucleotide, or allow one or more luminescently labeled nucleotides to be differentiated from amongst a plurality of luminescently labeled nucleotides. The assignment or differentiation may rely on any one of luminescent lifetime, luminescent intensity, spectral range of the emitted photons, or any combination thereof.

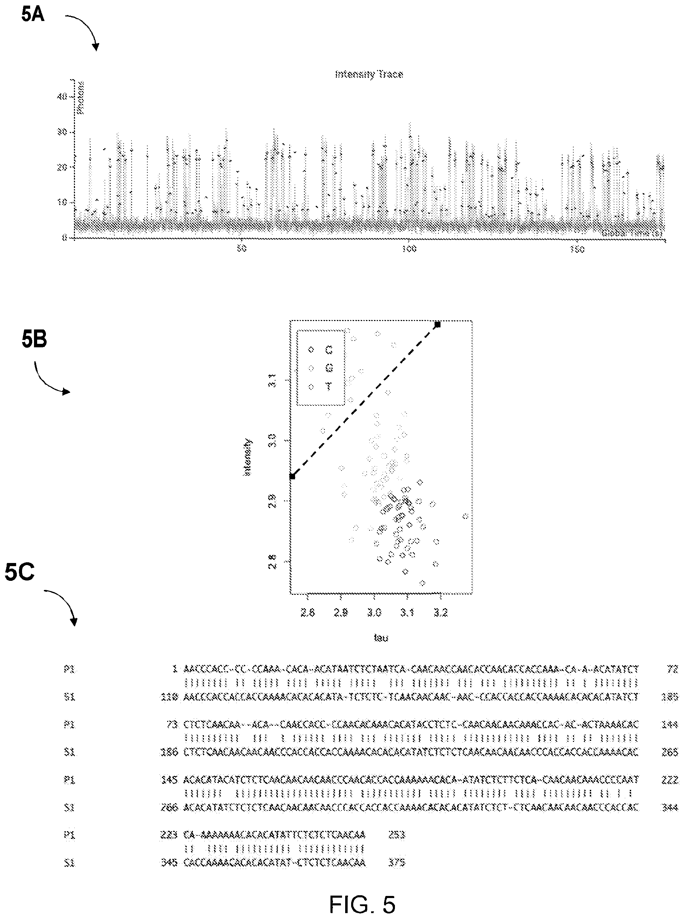

FIG. 5 depicts the sequencing of an exemplary template nucleic acid. The sequencing experiment was run with 4 luminescently labeled nucleotides: deoxyadenosine linked to Alexa Fluor^{®} 647 (A-AF647), dexoythymidine linked to Alex Fluor 555 (T-AF555), deoxy guanidine linked to DyLight^{®} 554-R1 (G-D554R1), and dexoycytidine linked to DyLight^{®} 530-R2 (C-D530R2). The nucleotide A-AF647 is excited by excitation energy in the red spectral range, and T, G, and C nucleotides are excited by excitation in the green spectral range. The number of photons detected over ~200 s of a sequencing reaction are shown in an intensity trace 5-1. Each spike corresponds to a burst of detected luminescences and is marked with a dot. Each burst may correspond to the incorporation of a luminescently labeled nucleotide, and comprises thousands of detected luminescences. Different colored traces can be utilized to denote different excitation pulses. For example, a purple trace can be used for green excitation pulses, and a blue trace can be used for red excitation pulses. Bursts from the blue trace can be assigned to the incorporation of the nucleotide A-AF647 (the only nucleotide with red luminescent molecule in this example).

FIG. 5 shows one way of reducing the raw data to differentiate bursts of the same color (e.g., bursts in the purple trace between T, G, and C) using an intensity versus lifetime plot 5-2. Each circle represents a burst from the purple trace. Each burst has been analyzed to determine the luminescent lifetime of the luminescently labeled nucleotide based on the time duration between pulse and emission of each detected photon. Additionally, each burst has been analyzed to determine the luminescent intensity of the luminescently labeled nucleotide based on the number of detected photons per second. The incorporation events are clustered in three groups corresponding to each of the three luminescently labeled nucleotides. The dark cluster in the lower portion of the plot (area of the plot below the dashed line) is assigned to C-D530R2 which has the longest luminescent lifetime and the lowest luminescent intensity. The light cluster in the lower portion of the plot (area of the plot below the dashed line) is assigned to G-D554R1 which has the intermediate lifetime and intensity. And the light cluster in the upper portion of the plot (area of the plot above the dashed line) is assigned to T-AF555 which has the shortest lifetime and highest intensity. FIG. 5 shows the alignment 5-3 between the sequence determined from the data and the known sequence of the template nucleic acid. Vertical bars indicate a match between the experimentally determined base and the target sequence. Dashes indicate a position in the template sequence for which no nucleotide was assigned in the determined sequence, or an extra position in the determined sequence which does not correspond to any position in the template sequence.

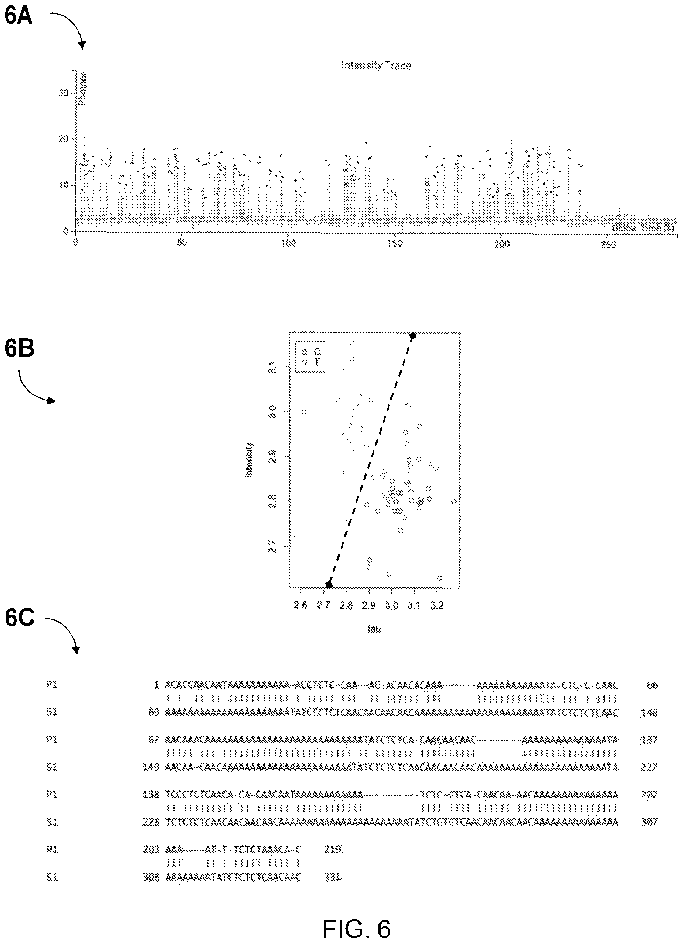

FIG. 6 depicts a second example for sequencing of a template nucleic acid. The sequencing experiment was run with 4 luminescently labeled nucleotides: deoxyadenosine linked to Alexa Fluor^{®} 647 (A-AF647), dexoythymidine linked to Alex Fluor 555 (T-AF555), deoxyguanidine linked to Alexa Fluor^{®} 647 (G-AF647), and a dexoycytidine linked to Alexa Fluor^{®} 546 (C-AF546). The nucleotides A-AF647and G-AF647 are excited by excitation energy in the red spectral range, and T and C nucleotides are excited by excitation in the green spectral range. In this experiment, A and G have the same luminescent marker, and are not discriminated. FIG. 6 shows the number of photons detected over ~300 s of a sequencing reaction in an intensity trace 6-1. Each spike corresponds to a burst of detected luminescences and is marked with a dot. Each burst may correspond to the incorporation of a luminescently labeled nucleotide, and comprises thousands of detected luminescences. The trace shows detected luminescences for green excitation pulses (corresponding to bases T and C).

FIG. 6 shows one way of reducing the raw data to differentiate T and C using an intensity versus lifetime plot 6-2. Each circle represents a burst from the intensity trace 6-1. Each burst has been analyzed to determine the luminescent lifetime of the luminescently labeled nucleotide based on the time duration between pulse and emission of each detected photon. Additionally each burst has been analyzed to determine the luminescent intensity of the luminescently labeled nucleotide based on the number of detected photons per second. The incorporation events are clustered in two groups corresponding to each of the two luminescently labeled nucleotides. The dark cluster in the right portion of the plot (area of the plot to the right of the dashed line) is assigned to C-AF546 which has the longest luminescent lifetime and the lowest luminescent intensity. The light cluster in the right portion of the plot (area of the plot to the right of the dashed line) is assigned to T-AF555 which has the shortest lifetime and highest intensity. FIG. 6 shows the alignment 6-3 between the sequence determined from the data and the known sequence of the template nucleic acid. Vertical bars indicate a match between the experimentally determined base and the target sequence. Dashes indicate a position in the template sequence for which no nucleotide was assigned in the determined sequence, or an extra position in the determined sequence which does not correspond to any position in the template sequence.

### Luminescent Labels

The terms luminescent tag, luminescent label and luminescent marker are used interchangeably throughout, and relate to molecules comprising one or more luminescent molecules. In certain embodiments, the incorporated molecule is a luminescent molecule, e.g., without attachment of a distinct luminescent label. Typical nucleotide and amino acids are not luminescent, or do not luminesce within suitable ranges of excitation and emission energies. In certain embodiments, the incorporated molecule comprises a luminescent label. In certain embodiments, the incorporated molecule is a luminescently labeled nucleotide. In certain embodiments, the incorporated molecule is a luminescently labeled amino acid or luminescently labeled tRNA. In some embodiments, a luminescently labeled nucleotide comprises a nucleotide and a luminescent label. In some embodiments, a luminescently labeled nucleotide comprises a nucleotide, a luminescent label, and a linker. In some embodiments, the luminescent label is a fluorophore.

In certain embodiments, the luminescent label, and optionally the linker, remain attached to the incorporated molecule. In certain embodiments, the luminescent label, and optionally the linker, are cleaved from the molecule during or after the process of incorporation.

In certain embodiments, the luminescent label is a cyanine dye, or an analog thereof. In some embodiments, the cyanine dye is of formula: 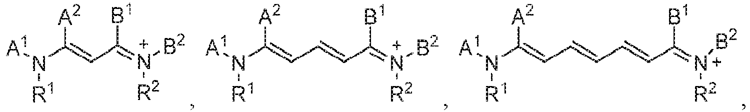 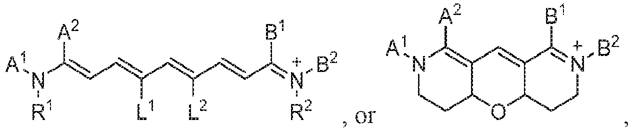 or a salt, stereoisomer, or tautomer thereof, wherein:
A¹ and A² are joined to form an optionally substituted, aromatic or non-aromatic, monocyclic or polycyclic, heterocyclic ring;
B¹ and B² are joined to form an optionally substituted, aromatic or non-aromatic, monocyclic or polycyclic, heterocyclic ring;
each of R¹ and R² is independently hydrogen, optionally substituted alkyl; and
each of L¹ and L² is independently hydrogen, optionally substituted alkyl, or L¹ and L² are joined to form an optionally substituted, aromatic or non-aromatic, monocyclic or polycyclic, carbocyclic ring.

In certain embodiments, the luminescent label is a rhodamine dye, or an analog thereof. In some embodiments, the rhodamine dye is of formula: 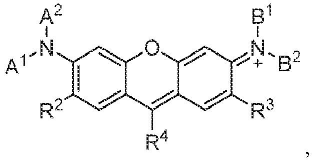 or a salt, stereoisomer, or tautomer thereof, wherein:
each of A¹ and A² is independently hydrogen, optionally substituted alkyl, optionally substituted aromatic or non-aromatic heterocyclyl, optionally substituted aromatic or non-aromatic carbocyclyl, or optionally substituted carbonyl, or A¹ and A² are joined to form an optionally substituted, aromatic or non-aromatic, monocyclic or polycyclic, heterocyclic ring;
each of B¹ and B² is independently hydrogen, optionally substituted alkyl, optionally substituted, aromatic or non-aromatic heterocyclyl, optionally substituted, aromatic or non-aromatic carbocyclyl, or optionally substituted carbonyl, or B¹ and B² are joined to form an optionally substituted, aromatic or non-aromatic, monocyclic or polycyclic, heterocyclic ring;
each of R² and R³ is independently hydrogen, optionally substituted alkyl, optionally substituted aryl, or optionally substituted acyl; and
R⁴ is hydrogen, optionally substituted alkyl, optionally substituted, optionally substituted aromatic or non-aromatic heterocyclyl, optionally substituted aromatic or non-aromatic carbocyclyl, or optionally substituted carbonyl.

In some embodiments, R⁴ is optionally substituted phenyl. In some embodiments, R⁴ is optionally substituted phenyl, wherein at least one substituent is optionally substituted carbonyl. In some embodiments, R⁴ is optionally substituted phenyl, wherein at least one substituent is optionally substituted sulfonyl.

Typically, the luminescent label comprises an aromatic or heteroaromatic compound and can be a pyrene, anthracene, naphthalene, acridine, stilbene, indole, benzindole, oxazole, carbazole, thiazole, benzothiazole, phenanthridine, phenoxazine, porphyrin, quinoline, ethidium, benzamide, cyanine, carbocyanine, salicylate, anthranilate, coumarin, fluoroscein, rhodamine or other like compound. Exemplary dyes include xanthene dyes, such as fluorescein or rhodamine dyes, including 5-carboxyfluorescein (FAM), 2'7'-dimethoxy-4'5'-dichloro-6-carboxyfluorescein (JOE), tetrachlorofluorescein (TET), 6-carboxyrhodamine (R6G), N,N,N',N'-tetramethyl-6-carboxyrhodamine (TAMRA), 6-carboxy-X-rhodamine (ROX). Exemplary dyes also include naphthylamine dyes that have an amino group in the alpha or beta position. For example, naphthylamino compounds include 1-dimethylaminonaphthyl-5-sulfonate, 1-anilino-8-naphthalene sulfonate and 2-p-toluidinyl-6-naphthalene sulfonate, 5-(2'-aminoethyl)aminonaphthalene-1-sulfonic acid (EDANS). Other exemplary dyes include coumarins, such as 3-phenyl-7-isocyanatocoumarin; acridines, such as 9-isothiocyanatoacridine and acridine orange; N-(p-(2-benzoxazolyl)phenyl)maleimide: cyanines, such as indodicarbocyanine 3 (Cy^{®}3), (2Z)-2-[(E)-3-[3-(5-carboxypentyl)-1,1-dimethyl-6,8-disulfobenzo[e]indol-3-ium-2-yl]prop-2-enylidene]-3-ethyl-1,1-dimethyl-8-(trioxidanylsulfanyl)benzo[e]indole-6-sulfonate (Cy^{®}3.5), 2-{2-[(2,5-dioxopyrrolidin-1-yl)oxy]-2-oxoethyl}-16,16,18,18-tetramethyl-6,7,7a, 8a,9,10,16,18-octahydrobenzo[2",3"]indolizino[8",7":5',6']pyrano[3',2':3,4]pyrido[1,2-a]indol-5-ium-14-sulfonate (Cy^{®}3B), indodicarbocyanine 5 (Cy^{®}5), indodicarbocyanine 5.5 (Cy^{®}5.5), 3-(-carboxy-pentyl)-3'-ethyl-5,5'-dimethyloxacarbocyanine (CyA); 1H, 5H, 11H, 15H-Xantheno[2,3,4-ij:5,6,7-i'j']diquinolizin- 18-ium, 9-[2(or 4)-[[[6-[2,5-dioxo-1-pyrrolidinyl)oxy]-6-oxohexyl]amino]sulfonyl]-4(or 2)-sulfophenyl]-2,3,6,7,12,13,16,17-octahydro-inner salt (TR or Texas Red^{®}); BODIPY^{®} dyes: benzoxazoles; stilbenes; pyrenes: and the like.

For nucleotide sequencing, certain combinations of luminescently labeled nucleotides may be preferred. In some embodiments, at least one of the luminescently labeled nucleotides comprises a cyanine dye, or analog thereof. In some embodiments, at least one luminescently labeled nucleotides comprises a rhodamine dye, or analog thereof. In some embodiments, at least two luminescently labeled nucleotides each comprise a cyanine dye, or analog thereof. In some embodiments, at least two luminescently labeled nucleotides each comprise a rhodamine dye, or analog thereof. In some embodiments, at least three luminescently labeled nucleotides each comprise a cyanine dye, or analog thereof. In some embodiments, at least three luminescently labeled nucleotides each comprise a rhodamine dye, or analog thereof. In some embodiments, at least four luminescently labeled nucleotides each comprise a cyanine dye, or analog thereof. In some embodiments, at least four luminescently labeled nucleotides each comprise a rhodamine dye, or analog thereof. In some embodiments, three luminescently labeled nucleotides comprise a cyanine dye, or analog thereof, and a fourth luminescently labeled nucleotide comprises a rhodamine dye, or analog thereof. In some embodiments, two luminescently labeled nucleotides comprise a cyanine dye, or analog thereof, and a third, and optionally a fourth, luminescently labeled nucleotide comprises a rhodamine dye, or analog thereof. In some embodiments, three luminescently labeled nucleotides comprise a rhodamine dye, or analog thereof, and a third, and optionally a fourth, luminescently labeled nucleotide comprises a cyanine dye, or analog thereof.

In some embodiments, at least one labeled nucleotides is linked to two or more dyes (e.g., two or more copies of the same dye and/or two or more different dyes).

In some embodiments, at least two luminescently labeled nucleotides absorb a first excitation energy, wherein at least one of the luminescently labeled nucleotides comprises a cyanine dye, or analog thereof, and at least one of the luminescently labeled nucleotides comprises a rhodamine dye, or an analog thereof. In some embodiments, at least two luminescently labeled nucleotides absorb a second excitation energy, wherein at least one of the luminescently labeled nucleotides comprises a cyanine dye, or analog thereof, and at least one of the luminescently labeled nucleotides comprises a rhodamine dye, or an analog thereof. In some embodiments, at least two luminescently labeled nucleotides absorb a first excitation energy, wherein at least one of the luminescently labeled nucleotides comprises a cyanine dye, or analog thereof, and at least one of the luminescently labeled nucleotides comprises a rhodamine dye, or an analog thereof, and at least two additional luminescently labeled nucleotides absorb a second excitation energy, wherein at least one of the luminescently labeled nucleotides comprises a cyanine dye, or analog thereof, and at least one of the luminescently labeled nucleotides comprises a rhodamine dye, or an analog thereof.

In some embodiments, at least two luminescently labeled nucleotides absorb a first excitation energy, wherein at least one of the luminescently labeled nucleotides has a luminescent lifetime of less than about 1 ns, and at least one of the luminescently labeled nucleotides has a luminescent lifetime of greater than 1 ns. In some embodiments, at least two luminescently labeled nucleotides absorb a second excitation energy, wherein at least one of the luminescently labeled nucleotides has a luminescent lifetime of less than about 1 ns, and at least one of the luminescently labeled nucleotides has a luminescent lifetime of greater than 1 ns. In some embodiments, at least two luminescently labeled nucleotides absorb a first excitation energy, wherein at least one of the luminescently labeled nucleotides has a luminescent lifetime of less than about 1 ns, and at least one of the luminescently labeled nucleotides has a luminescent lifetime of greater than 1 ns, and at least additional two luminescently labeled nucleotides absorb a second excitation energy, wherein at least one of the luminescently labeled nucleotides has a luminescent lifetime of less than about 1 ns, and at least one of the luminescently labeled nucleotides has a luminescent lifetime of greater than 1 ns.

In certain embodiments, the luminescent label is a dye selected from Table 1. The dyes listed in Table 1 are non-limiting, and the luminescent labels of the application may include dyes not listed in Table 1. In certain embodiments, the luminescent labels of one or more luminescently labeled nucleotides is selected from Table 1. In certain embodiments, the luminescent labels of four or more luminescently labeled nucleotides is selected from

### Table 1.

**Table 1. Exemplary fluorophores.**

| **Fluorophores** | | |
|---|---|---|
| 5/6-Carboxyrhodamine 6G | Chromis 678C | DyLight^{®} 655-B1 |
| 5-Carboxyrhodamine 6G | Chromis 678Z | DyLight^{®} 655-B2 |
| 6-Carboxyrhodamine 6G | Chromis 770A | DyLight^{®} 655-B3 |
| 6-TAMRA | Chromis 770C | DyLight^{®} 655-B4 |
| Alexa Fluor^{®} 350 | Chromis 800A | DyLight^{®} 662Q |
| Alexa Fluor^{®} 405 | Chromis 800C | DyLight^{®} 675-B1 |
| Alexa Fluor^{®} 430 | Chromis 830A | DyLight^{®} 675- B2 |
| Alexa Fluor^{®} 480 | Chromis 830C | DyLight^{®} 675-B3 |
| Alexa Fluor^{®} 488 | Cy^{®}3 | DyLight^{®} 675- B4 |
| Alexa Fluor^{®} 514 | Cy^{®}3.5 | DyLight^{®} 679-C5 |
| Alexa Fluor^{®} 532 | Cy^{®}3B | DyLight^{®} 680 |
| Alexa Fluor^{®} 546 | Cy^{®}5 | DyLight^{®} 683Q |
| Alexa Fluor^{®} 555 | Dyomics-350 | DyLight^{®} 690-B1 |
| Alexa Fluor^{®} 568 | Dyomics-350XL | DyLight^{®} 690-B2 |
| Alexa Fluor^{®} 594 | Dyomics-360XL | DyLight^{®} 696Q |
| Alexa Fluor^{®} 610-X | Dyomics-370XL | DyLight^{®} 700-B1 |
| Alexa Fluor^{®} 633 | Dyomics-375XL | DyLight^{®} 700-B1 |
| Alexa Fluor^{®} 647 | Dyomics-380XL | DyLight^{®} 730-B1 |
| Alexa Fluor^{®} 660 | Dyomics-390XL | DyLight^{®} 730-B2 |
| Alexa Fluor^{®} 680 | Dyomics-405 | DyLight^{®} 730- B3 |
| Alexa Fluor^{®} 700 | Dyomics-415 | DyLight^{®} 730-B4 |
| Alexa Fluor^{®} 750 | Dyomics-430 | DyLight^{®} 747 |
| Alexa Fluor^{®} 790 | Dyomics-431 | DyLight^{®} 747-B1 |
| AMCA | Dyomics-478 | DyLight^{®} 747-B2 |
| ATTO 390 | Dyomics-480XL | DyLight^{®} 747-B3 |
| ATTO 425 | Dyomics-481XL | DyLight^{®} 747-B4 |
| ATTO 465 | Dyomics-485XL | DyLight^{®} 755 |
| ATTO 488 | Dyomics-490 | DyLight^{®} 766Q |
| ATTO 495 | Dyomics-495 | DyLight^{®} 775-B2 |
| ATTO 514 | Dyomics-505 | DyLight^{®} 775-B3 |
| ATTO 520 | Dyomics-510XL | DyLight^{®} 775-B4 |
| ATTO 532 | Dyomics-511XL | DyLight^{®} 780-B1 |
| ATTO 542 | Dyomics-520XL | DyLight^{®} 780-B2 |
| ATTO 550 | Dyomics-521XL | DyLight^{®} 780-B3 |
| ATTO 565 | Dyomics-530 | DyLight^{®} 800 |
| ATTO 590 | Dyomics-547 | DyLight^{®} 830-B2 |
| ATTO 610 | Dyomics-547P1 | eFluor^{®} 450 |
| ATTO 620 | Dyomics-548 | Eosin |
| ATTO 633 | Dyomics-549 | FITC |
| ATTO 647 | Dyomics-549P1 | Fluorescein |
| ATTO 647N | Dyomics-550 | HiLyte^{™} Fluor 405 |
| ATTO 655 | Dyomics-554 | HiLyte^{™} Fluor 488 |
| ATTO 665 | Dyomics-555 | HiLyte^{™} Fluor 532 |
| ATTO 680 | Dyomics-556 | HiLyte^{™} Fluor 555 |
| ATTO 700 | Dyomics-560 | HiLyte^{™} Fluor 594 |
| ATTO 725 | Dyomics-590 | HiLyte^{™} Fluor 647 |
| ATTO 740 | Dyomics-591 | HiLyte^{™} Fluor 680 |
| ATTO Oxa12 | Dyomics-594 | HiLyte^{™} Fluor 750 |
| ATTO Rho101 | Dyomics-601XL | IRDye^{®} 680LT |
| ATTO Rho11 | Dyomics-605 | IRDye^{®} 750 |
| ATTO Rho12 | Dyomics-610 | IRDye^{®} 800CW |
| ATTO Rho13 | Dyomics-615 | JOE |
| ATTO Rho14 | Dyomics-630 | LightCycler^{®} 640R |
| ATTO Rho3B | Dyomics-631 | LightCycler^{®} Red 610 |
| ATTO Rho6G | Dyomics-632 | LightCycler^{®} Red 640 |
| ATTO Thio12 | Dyomics-633 | LightCycler^{®} Red 670 |
| BD Horizon^{™} V450 | Dyomics-634 | LightCycler^{®} Red 705 |
| BODIPY^{®} 493/501 | Dyomics-635 | Lissamine Rhodamine B |
| BODIPY^{®} 530/550 | Dyomics-636 | Napthofluorescein |
| BODIPY^{®} 558/568 | Dyomics-647 | Oregon Green^{®} 488 |
| BODIPY^{®} 564/570 | Dyomics-647P1 | Oregon Green^{®} 514 |
| BODIPY^{®} 576/589 | Dyomics-648 | Pacific Blue^{™} |
| BODIPY^{®} 581/591 | Dyomics-648P1 | Pacific Green^{™} |
| BODIPY^{®} 630/650 | Dyomics-649 | Pacific Orange^{™} |
| BODIPY^{®} 650/665 | Dyomics-649P1 | PET |
| BODIPY^{®} FL | Dyomics-650 | PF350 |
| BODIPY^{®} FL-X | Dyomics-651 | PF405 |
| BODIPY^{®} R6G | Dyomics-652 | PF415 |
| BODIPY^{®} TMR | Dyomics-654 | PF488 |
| BODIPY^{®} TR | Dyomics-675 | PF505 |
| C5.5 | Dyomics-676 | PF532 |
| C7 | Dyomics-677 | PF546 |
| CAL Fluor^{®} Gold 540 | Dyomics-678 | PF555P |
| CAL Fluor^{®} Green 510 | Dyomics-679P1 | PF568 |
| CAL Fluor^{®} Orange 560 | Dyomics-680 | PF594 |
| CAL Fluor^{®} Red 590 | Dyomics-681 | PF610 |
| CAL Fluor^{®} Red 610 | Dyomics-682 | PF633P |
| CAL Fluor^{®} Red 615 | Dyomics-700 | PF647P |
| CAL Fluor^{®} Red 635 | Dyomics-701 | Quasar^{®} 570 |
| Cascade^{®} Blue | Dyomics-703 | Quasar^{®} 670 |
| CF^{™}350 | Dyomics-704 | Quasar^{®} 705 |
| CF^{™}405M | Dyomics-730 | Rhoadmine 123 |
| CF^{™}405S | Dyomics-731 | Rhodamine 6G |
| CF^{™}488A | Dyomics-732. | Rhodamine B |
| CF^{™}514 | Dyomics-734 | Rhodamine Green |
| CF^{™}532 | Dyomics-749 | Rhodamine Green-X |
| CF^{™}543 | Dyomics-749P1 | Rhodamine Red |
| CF^{™}546 | Dyomics-750 | ROX |
| CF^{™}555 | Dyomics-751 | ROX |
| CF^{™}568 | Dyomics-752 | Seta^{™} 375 |
| CF^{™}594 | Dyomics-754 | Seta^{™} 470 |
| CF^{™}620R | Dyomics-776 | Seta^{™} 555 |
| CF^{™}633 | Dyomics- 777 | Seta^{™} 632 |
| CF^{™}633-V1 | Dyomics-778 | Seta^{™} 633 |
| CF^{™}640R | Dyomics-780 | Seta^{™} 650 |
| CF^{™}640R-V1 | Dyomics-781 | Seta^{™} 660 |
| CF^{™}640R-V2 | Dyomics-782. | Seta^{™} 670 |
| CF^{™}660C | Dyomics-800 | Seta^{™} 680 |
| CF^{™}660R | Dyomics-831 | Seta^{™} 700 |
| CF^{™}680 | DyLight^{®} 350 | Seta^{™} 750 |
| CF^{™}680R | DyLight^{®} 405 | Seta^{™} 780 |
| CF^{™}680R-V1 | DyLight^{®} 415-Co1 | Seta^{™} APC-780 |
| CF^{™}750 | DyLight^{®} 425Q | Seta^{™} PerCP-680 |
| CF^{™}770 | DyLight^{®} 485-L.S | Seta^{™} R-PE-670 |
| CF^{™}790 | DyLight^{®} 488 | Seta^{™}646 |
| Chromeo^{™} 642 | DyLight^{®} 504Q | Seta^{™}u 380 |
| Chromis 425N | DyLight^{®} 510-LS | Seta^{™}u 425 |
| Chromis 500N | DyLight^{®} 515-LS | Seta^{™}u 647 |
| Chromis 515N | DyLight^{®} 521-LS | Seta^{™}u 405 |
| Chromis 530N | DyLight^{®} 530-R2 | Sulforhodamine 101 |
| Chromis 550A | DyLight^{®} 543Q | TAMRA |
| Chromis 550C | DyLight^{®} 550 | TET |
| Chromis 550Z | DyLight^{®} 554-R0 | Texas Red^{®} |
| Chromis 560N | DyLight^{®} 554-R1 | TMR |
| Chromis 570N | DyLight^{®} 590-R2 | TRITC |
| Chromis 577N | DyLight^{®} 594 | Yakima Yellow^{™} |
| Chromis 600N | DyLight^{®} 610-B1 | Zenon^{®} |
| Chromis 630N | DyLight^{®} 615-B2 | Zy3 |
| Chromis 645A | DyLight^{®} 633 | Zy5 |
| Chromis 645C | DyLight^{®} 633-B1 | Zy5.5 |
| Chromis 645Z | DyLight^{®} 633-B2 | Zy7 |
| Chromis 678A | DyLight^{®} 650 | Abberior^{®®} Star 635 |
| Square 635 | Square 650 | Square 660 |
| Square 672 | Square 680 | Abberior^{®} Star 440SXP |
| Abberior^{®} Star 470SXP | Abberior^{®} Star 488 | Abberior^{®} Star 512 |
| Abberior^{®} Star 52.0SXP | Abberior^{®} Star 580 | Abberior^{®} Star 600 |
| Abberior^{®} Star 635 | Abberior^{®} Star 635P | Abberior^{®} Star RED |

Dyes may also be classified based on the wavelength of maximum absorbance or emitted luminescence. Table 2 provides exemplary fluorophores grouped into columns according to approximate wavelength of maximum absorbance. The dyes listed in Table 2 are non-limiting, and the luminescent labels of the application may include dyes not listed in Table 2. The exact maximum absorbance or emission wavelength may not correspond to the indicated spectral ranges. In certain, embodiments, the luminescent labels of one or more luminescently labeled nucleotides is selected from the "Red" group listed in Table 2. In certain embodiments, the luminescent labels of one or more luminescently labeled nucleotides is selected from the "Green" group listed in Table 2. In certain embodiments, the luminescent labels of one or more luminescently labeled nucleotides is selected from the "Yellow/Orange" group listed in Table 2. In certain embodiments, the luminescent labels of four nucleotides are selected such that all are selected from one of the "Red", "Yellow/Orange", or "Green" group listed in Table 2. In certain embodiments, the luminescent labels of four nucleotides are selected such that three are selected from a first group of the "Red", "Yellow/Orange", and "Green" groups listed in Table 2, and the fourth is selected from a second group of the "Red", "Yellow/Orange", and "Green" groups listed in Table 2. In certain embodiments, the luminescent labels of four nucleotides are selected such that two are selected from a first of the "Red", "Yellow/Orange", and "Green" group listed in Table 2, and the third and fourth are selected from a second group of the "Red", "Yellow/Orange", and "Green" groups listed in Table 2. In certain embodiments, the luminescent labels of four nucleotides are selected such that two are selected from a first of the "Red", "Yellow/Orange", and "Green" groups listed in Table 2, and a third is selected from a second group of the "Red", "Yellow/Orange", and "Green" groups listed in Table 2, and a fourth is selected from a third group of the "Red", "Yellow/Orange", and "Green" groups listed in Table 2.

**Table 2. Exemplary fluorophores by spectral range.**

| **"Green" 520-570 nm** | **"Yellow/Orange" 570-620 nm** | **"Red" 620-670 nm** |
|---|---|---|
| 5/6-Carboxyrhoadmine 6G | Alexa Fluor^{®} 594 | Alexa Fluor^{®} 633 |
| 6-TAMRA | Alexa Fluor^{®} 610-X | Alexa Fluor^{®} 647 |
| Alexa Fluor^{®} 532 | ATTO 590 | Alexa Fluor^{®} 660 |
| Alexa Fluor^{®} 546 | ATTO 610 | ATTO 633 |
| Alexa Fluor^{®} 555 | ATTO 620 | ATTO 647 |
| Alexa Fluor^{®} 568 | BODIPY^{®} 576/589 | ATTO 647N |
| ATTO 520 | BODIPY^{®} 581/591 | ATTO 655 |
| ATTO 532 | CF^{™}594 | ATTO 665 |
| ATTO 542 | CF^{™}620R | ATTO 680 |
| ATTO 550 | Chromis 570N | ATTO Rho14 |
| ATTO 565 | Chromis 577N | BODIPY^{®} 630/650 |
| BODIPY^{®} 530/550 | Chromis 600N | BODIPY^{®} 650/665 |
| BODIPY^{®} 558/568 | Dyomics-590 | CAL Fluor^{®} Red 635 |
| BODIPY^{®} 564/570 | Dyomics-591 | CF^{™} 633-V1 |
| CF^{™}514 | Dyomics-594 | CF^{™} 640R-V1 |
| CF^{™}532 | Dyomics-601XL | CF^{™}633 |
| CF^{™}543 | Dyomics-605 | CF^{™}640R |
| CF^{™}546 | Dyomics-610 | CF^{™}640R-V2 |
| CF^{™}555 | Dyomics-615 | CF^{™}660C |
| CF^{™}568 | DyLight^{®} 590-R2 | CF^{™}660R |
| Chromis 530N | DyLight^{®} 594 | CF^{™}680 |
| Chromis 550A | DyLight^{®} 610-B1 | CF^{™}680R |
| Chromis 550C | DyLight^{®} 615-B2 | CF^{™}680R-V1 |
| Chromis 550Z | HiLyte^{™} Fluor 594 | Chromeo^{™} 642 |
| Chromis 560N | LightCycler^{®®} Red 610 | Chromis 630N |
| Cy^{®}3 | PF594 | Chromis 645A |
| Cy^{®}3.5 | PF594 | Chromis 645A |
| Cy^{®}3B | PF610 | Chromis 645C |
| Dyomics-530 | Quasar^{®} 570 | Chromis 645Z |
| Dyomics-547 | Abberior^{®} Star 580 | Cy^{®}5 |
| Dyomics-547P1 | Abberior^{®} Star 600 | Cy^{®}5.5 |
| Dyomics-548 | | Dyomics-630 |
| Dyomics-549P1 | | Dyomics-631 |
| Dyomics-550 | | Dyomics-632 |
| Dyomics-554 | | Dyomics-633 |
| Dyomics-555 | | Dyomics-634 |
| Dyomics-556 | | Dyomics-635 |
| Dyomics-560 | | Dyomics-636 |
| DyLight^{®} 521-LS | | Dyomics-647 |
| DyLight^{®} 530-R2 | | Dyomics-647P1 |
| DyLight^{®} 543Q | | Dyomics-648 |
| DyLight^{®} 550 | | Dyom ics-648 P1 |
| DyLight^{®} 554-R0 | | Dyomics-649 |
| DyLight^{®} 554-R1 | | Dyomics-649P1 |
| HiLyte^{™} Fluor 532 | | Dyomics-650 |
| HiLyte^{™} Fluor 555 | | Dyomics-651 |
| PF532 | | Dyomics-652 |
| PF546 | | Dyomics-654 |
| PF555P | | DyLight^{®} 633 |
| PF568 | | DyLight^{®} 633-B1 |
| Seta^{™} 555 | | DyLight^{®} 633-B2 |
| Abberior^{®} Star 520SXP | | DyLight^{®} 650 |
| | | DyLight^{®} 655-B1 |
| | | DyLight^{®} 655-B2 |
| | | DyLight^{®} 655-B3 |
| | | DyLight^{®} 655-B4 |
| | | DyLight^{®} 662Q |
| | | DyLight^{®} 680 |
| | | DyLight^{®} 683Q |
| | | HiLyte^{™} Fluor 647 |
| | | HiLyte^{™} Fluor 680 |
| | | LightCycler^{®®} 640R |
| | | LightCycler^{®} Red 640 |
| | | LightCycler^{®} Red 670 |
| | | PF633P |
| | | PF647P |
| | | Quasar^{®} 670 |
| | | Seta^{™} 632 |
| | | Seta^{™} 633 |
| | | Seta^{™} 650 |
| | | Seta^{™} 660 |
| | | Seta^{™} 670 |
| | | Seta^{™}Tau 647 |
| | | Square 635 |
| | | Square 650 |
| | | Square 660 |
| | | Abberior^{®} Star 635 |
| | | Abberior^{®} Star 635P |
| | | Abberior^{®} Star RED |

In certain embodiments, the luminescent label may be (Dye 101), (Dye 102), (Dye 103), (Dye 104), (Dye 105), or (Dye 106), of formulae (in NHS ester form): 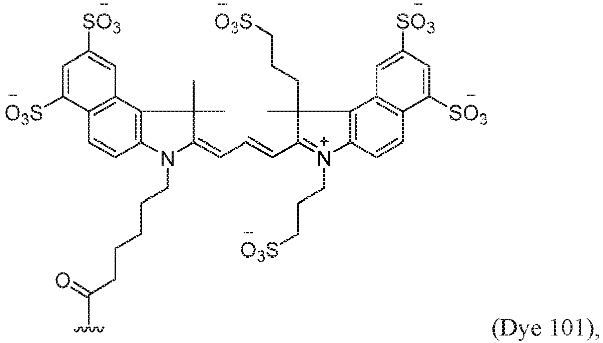 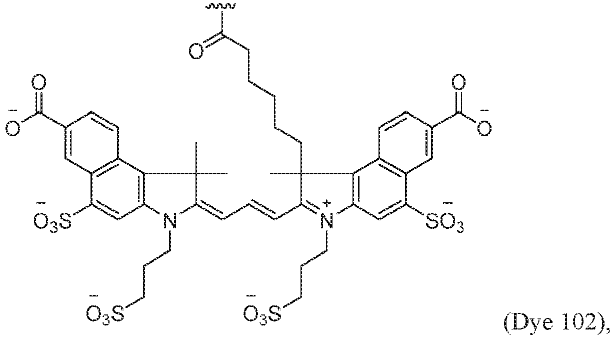 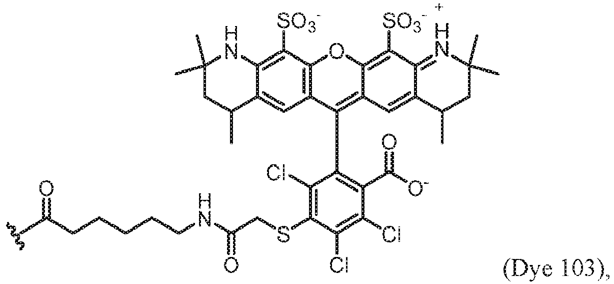 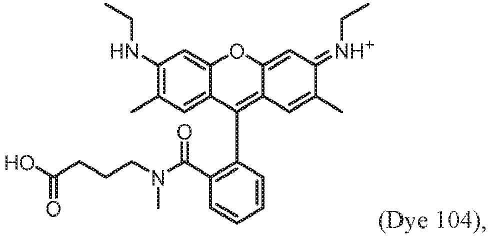 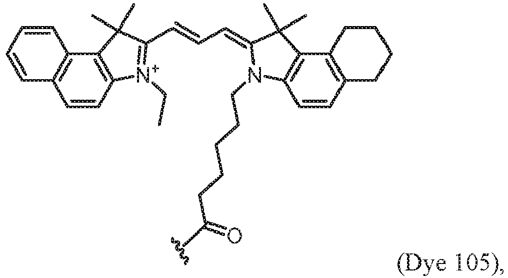 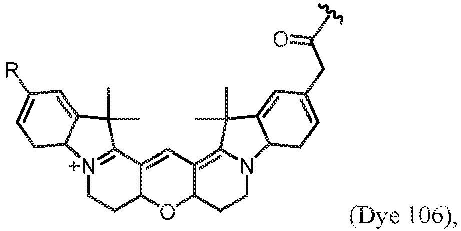 or an analog thereof. In some embodiments, each sulfonate or carboxylate is independently optionally protonated. In some embodiments, the dyes above are attached to the linker or nucleotide by formation of an amide bond at the indicated point of attachment.

In certain embodiments, the luminescent label may comprise a first and second chromophore. In some embodiments, an excited state of the first chromophore is capable of relaxation via an energy transfer to the second chromophore. In some embodiments, the energy transfer is a Förster resonance energy transfer (FRET). Such a FRET pair may be useful for providing a luminescent label with properties that make the label easier to differentiate from amongst a plurality of luminescent labels. In certain embodiments, the FRET pair may absorb excitation energy in a first spectral range and emit luminescence in a second spectral range.

For a set of luminescently labeled molecules (e.g., luminescently labeled nucleotides), the properties of a luminescently labeled FRET pair may allow for selection of a plurality of distinguishable molecules (e.g., nucleotides). In some embodiments, the second chromophore of a FRET pair has a luminescent lifetime distinct from a plurality of other luminescently labeled molecules. In some embodiments, the second chromophore of a FRET pair has a luminescent intensity distinct from a plurality of other luminescently labeled molecules. In some embodiments, the second chromophore of a FRET pair has a luminescent lifetime and luminescent intensity distinct from a plurality of other luminescently labeled molecules. In some embodiments, the second chromophore of a FRET pair emits photons in a spectral range distinct from a plurality of other luminescently labeled molecules. In some embodiments, the first chromophore of a FRET pair has a luminescent lifetime distinct from a plurality of luminescently labeled molecules. In certain embodiments, the FRET pair may absorb excitation energy in a spectral range distinct from a plurality of other luminescently labeled molecules. In certain embodiments, the FRET pair may absorb excitation energy in the same spectral range as one or more of a plurality of other luminescently labeled molecules.

In some embodiments, two or more nucleotides can be connected to a luminescent label, wherein the nucleotides are connected to distinct locations on the luminescent label. A non-limiting example could include a luminescent molecule that contains two independent reactive chemical moieties (e.g., azido group, acetylene group, carboxyl group, amino group) that are compatible with a reactive moiety on a nucleotide analog. In such an embodiment, a luminescent label could be connected to two nucleotide molecules via independent linkages. In some embodiments, a luminescent label can comprise two or more independent connections to two or more nucleotides.

In some embodiments, two or more nucleotides can be connected to a luminescent dye via a linker (*e.g.,* a branched linker or a linker with two or more reactive sites onto which nucleotides and/or dyes can be attached). Accordingly, in some embodiments, two or more nucleotides (*e.g.,* of the same type) can be linked to two or more dyes (*e.g.,* of the same type).

In some embodiments, a luminescent label can comprise a protein with luminescent properties. In some embodiments, one or more nucleotides are connected to a luminescent protein. In some embodiments, one or more nucleotides are connected to a luminescent protein via connections to distinct sites of the protein. In certain embodiments, the luminescent labels of four nucleotides are selected such that one nucleotide is labeled with a fluorescent protein while the remaining three nucleotides are labeled with fluorescent dyes (e.g., the non-limiting examples in Tables 1 and 2). In certain embodiments, the luminescent labels of four nucleotides are selected such that two nucleotides are labeled with fluorescent proteins while the remaining two nucleotides are labeled with fluorescent dyes (e.g., the non-limiting examples in Tables 1 and 2). In certain embodiments, the luminescent labels of four nucleotides are selected such that three nucleotides are labeled with fluorescent proteins while the remaining nucleotide is labeled with a fluorescent dye (e.g., the non-limiting examples in Tables 1 and 2). In some embodiments, the luminescent labels of four nucleotides are selected such that all four nucleotides are labeled with fluorescent proteins.

According to some aspects of the application, luminescent labels *(e.g.,* dyes, for example fluorophores) can damage polymerases in a sequencing reaction that is exposed to excitation light. In some aspects, this damage occurs during the incorporation of a luminescently labeled nucleotide, when the luminescent molecule is held in close proximity to the polymerase enzyme. Non-limiting examples of damaging reactions include the formation of a covalent bond between the polymerase and luminescent molecule and emission of radiative or non-radiative decay from the luminescent molecule to the enzyme. This can shorten the effectiveness of the polymerase and reduce the length of a sequencing run.

In some embodiments, a nucleotide and a luminescent label are connected by a relatively long linker or linker configuration to keep the luminescent label away from the polymerase during incorporation of the labeled nucleotide. The term "linker configuration" is used herein to refer to the entire structure connecting the luminescent molecule(s) to the nucleotide(s) and does not encompass the luminescent molecule(s) or the nucleotide(s).

In some embodiments, a single linker connects a luminescent molecule to a nucleotide. In some embodiments, a linker contains one or more points of divergence so that two or more (*e.g..* 2, 3, 4, 5. 6, 7, 8, 9, 10, or more) nucleotides are connected to each luminescent molecule, two or more *(e.g.,* 2, 3, 4, 5, 6, 7, 8, 9, 10, or more) luminescent molecules are connected to each nucleotide, or two or more *(e.g.,* 2. 3, 4, 5, 6, 7, 8, 9, 10. or more) nucleotides are connected to two or more (*e.g.,* 2, 3, 4, 5, 6, 7, 8, 9, 10, or more) luminescent molecules.

In some embodiments, the linker configuration determines the distance between the luminescent label and the nucleotide. In some embodiments, the distance is about 1 nm or 2 nm to about 20 nm. For example, more than 2 nm, more than 5 nm, 5-10 nm, more than 10 nm, 10-15 nm, more than 15 nm, 15-20 nm, more than 20 nm. However, the distance between the luminescent label and the nucleotide cannot be too long since the luminescent label needs to be within the illumination volume to be excited when the nucleotide is held within the active site of the enzyme. Accordingly, in some embodiments, the overall linker length is less than 30 nm, less than 25 nm, around 20 nm, or less than 20 nm.

In some embodiments, a protecting molecule is included within a linker configuration. A protecting molecule can protect the polymerase from the damaging reactions that can occur between the enzyme and the luminescent label. Non-limiting examples of protecting molecules include nucleic acids (*e.g*., deoxyribonucleic acid, ribonucleic acid), for example nucleic acids comprising one or more protecting elements. In some embodiments, the protecting molecule is an oligonucleotide (e.g., a DNA oligonucleotide, an RNA oligonucleotide, or a variant thereof).

In some embodiments, a protecting molecule is connected to one or more luminescent molecules and to one or more nucleotide molecules. As used herein, a nucleotide in this context refers to a nucleoside polyphosphate that can be incorporated into a growing nucleic acid, e.g., in the context of a sequencing reaction. In some embodiments, the luminescent molecule(s) are not adjacent to the nucleotide(s). For example, one or more luminescent molecules can be connected on a first side of the protecting molecule and one or more nucleotides can be connected to a second side of the protecting molecule, wherein the first and second sides of the protecting molecule are distant from each other. In some embodiments, they are on approximately opposite sides of the protecting molecule.

The distance between the point at which a protecting molecule is connected to a luminescent label and the point at which the protecting molecule is connected to a nucleotide can be a linear measurement through space or a non-linear measurement across the surface of the protecting molecule. The distance between the luminescent label and nucleotide connection points on a protecting molecule can be measured by modeling the three-dimensional structure of the protecting molecule. In some embodiments, this distance can be 2, 4, 6, 8, 10, 12, 14, 16, 18. 20 nm or more. Alternatively, the relative positions of the luminescent label and nucleotide on a protecting molecule can be described by treating the structure of the protecting molecule as a quadratic surface (*e.g*., ellipsoid, elliptic cylinder). In some embodiments, the luminescent label and the nucleotide are separated by a distance that is at least one eighth of the distance around an ellipsoidal shape representing the protecting molecule. In some embodiments, the luminescent label and the nucleotide are separated by a distance that is at least one quarter of the distance around an ellipsoidal shape representing the protecting molecule. In some embodiments, the luminescent label and the nucleotide are separated by a distance that is at least one third of the distance around an ellipsoidal shape representing the protecting molecule. In some embodiments, the luminescent label and the nucleotide are separated by a distance that is one half of the distance around an ellipsoidal shape representing the protecting molecule.

In some embodiments, where a protecting molecule comprises a nucleic acid (e.g., a nucleic acid linker having one or more protecting elements), the distance between the luminescent label (e.g., one or more luminescent labels) and nucleotide (e.g., one or more nucleoside polyphosphates) attachment points on the nucleic acid can be measure based on the number of bases within the nucleic acid that occur between the luminescent label and the nucleotide. In some embodiments, the number of nucleic acid subunits in a linker (e.g., number of nucleotides in a single-stranded linker or base pairs in a double-stranded linker) separating the attachment point of a label from the attachment point of a nucleoside polyphosphate can be from 10-100 (e.g., 10-25, 25-50, 50-75, 75-100) or more. For example, in some embodiments, the nucleic acid linker is double-stranded. In such embodiments, distance between the attachment points of the luminescent label and the nucleotide can be measured by the number of base pairs that occur within the double-stranded nucleic acid (e.g., the base pairing between the nucleobases of first and second oligonucleotide strands of the nucleic acid linker). In some embodiments, the luminescent label and nucleotide attachment points on the nucleic acid linker (e.g., a linear nucleic acid linker) are separated by at least 5 base pairs, between 5 and 10 base pairs, at least 10 base pairs, between 10 and 15 base pairs, at least 15 base pairs, between 15 and 20 base pairs, at least 20 base pairs, between 20 and 25 base pairs, at least 25 base pairs, between 25 and 30 base pairs, at least 30 base pairs, between 30 and 35 base pairs, at least 35 base pairs, between 35 and 40 base pairs, at least 40 base pairs, between 40 and 45 base pairs, at least 45 base pairs, between 45 and 50 base pairs, at least 50 base pairs, between 50 and 75 base pairs, at least 75 base pairs, between 75 and 100 base pairs, or more. In some embodiments, it is contemplated that the inclusion of one or more structural motif protecting elements (e.g., stem-loops) may result in a greater number of bases and/or base pairs to occur between the luminescent label and the nucleotide. Thus, in some embodiments, the luminescent label and nucleotide attachment points on the nucleic acid linker are separated by at least 50 base pairs, between 50 and 60 base pairs, at least 60 base pairs, between 60 and 70 base pairs, at least 70 base pairs, between 70 and 80 base pairs, at least 80 base pairs, between 80 and 90 base pairs, at least 90 base pairs, between 90 and 100 base pairs, at least 100 base pairs, between 100 and 150 base pairs, at least 150 base pairs, between 150 and 200 base pairs, at least 200 base pairs, or more. In some embodiments, the number of base pairs in a double-stranded nucleic acid linker (or nucleotides in a single-stranded nucleic acid linker) is less than 500, less than 450, less than 400, less than 350, less than 300, or less than 250.

Protecting molecules, such as nucleic acid linkers comprising one or more protecting elements, can be useful to provide a steric barrier that prevents a luminescent label from getting near the polymerase. Protecting molecules can be useful to absorb, or protect the polymerase from, radiative and non-radiative decay emitted by a luminescent molecule. Protecting molecules can be useful to provide both a steric barrier and a decay barrier between the luminescent label and the polymerase.

The size of a protecting molecule should be such that a luminescent label is unable or unlikely to directly contact the polymerase when a nucleotide is held within the active site of the enzyme. The size of a protecting molecule should also be such that an attached luminescent label is within the illumination volume to be excited when a nucleotide is held within the active site of the enzyme. The size of a protecting molecule should be chosen with consideration to the linker that is selected to connect a luminescent label to the protecting molecule and the linker that is selected to connect a nucleotide to the protecting molecule. The protecting molecule and the linkers used to connect the luminescent label and nucleotide (e.g., nucleoside polyphosphate) comprise the linker configuration, wherein the size of the linker configuration should be such that the luminescent label is unable to directly contact the polymerase when the nucleotide is held within the active site of the enzyme.

The protecting molecule (and/or the linker configuration comprising the protecting molecule) is preferably water soluble. In some embodiments, it is preferable that the protecting molecule (and/or the linker configuration comprising the protecting molecule) has a net negative charge.

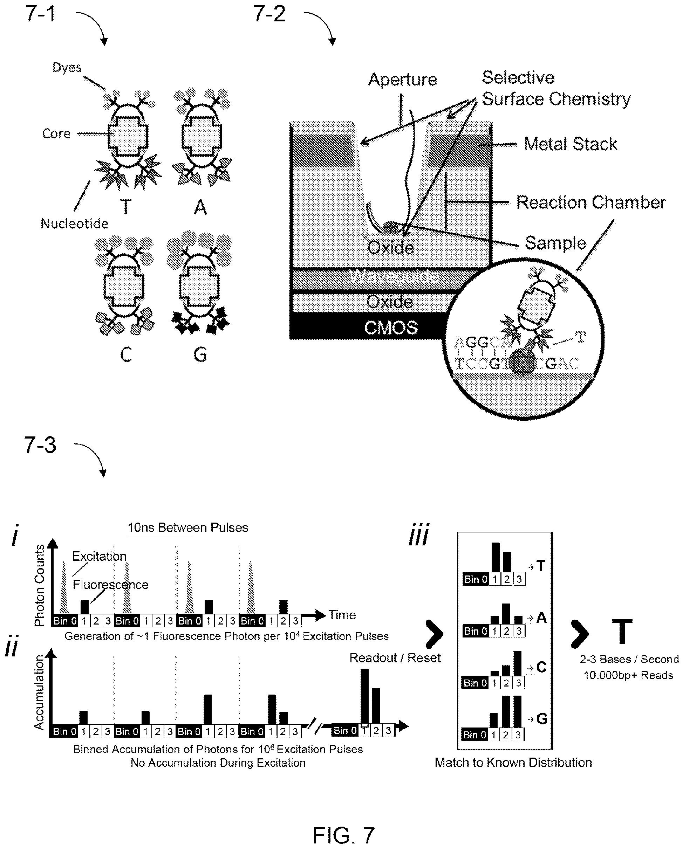

FIG. 7 further illustrates a non-limiting example of a sequencing experiment and how unique luminescent properties can be used to distinguish among a plurality of luminescently labeled nucleotides 7-1. The luminescent label connected to each base (thymine, adenine, cytosine, guanine) has luminescent properties (e.g., luminescent lifetime, luminescent intensity, and/or emission wavelength) that allow each labeled nucleotide to be distinguished from the plurality of labeled nucleotides. The inclusion of multiple nucleotides of the same type functions to accelerate incorporation rates in a sequencing reaction.

A sequencing experiment utilizing the luminescently labeled nucleotides 7-1 can be conducted in exemplary reaction vessel 7-2. The reaction takes place in a chamber above the waveguide, which serves as a conduit for excitation energy, delivering the excitation energy to the sample in the bottom of the reaction chamber by the evanescent wave from the waveguide. The aperture blocks light radiating from the waveguide to bulk sample and ambient and/or stray light from the sensor, as well as providing a fabrication path for the reaction chamber. The reaction chamber is an etched structure that places the sample on the bottom and within a region of high excitation from the evanescent wave of the waveguide. Selective surface chemistry is used to provide the bottom and sidewall of the reaction chamber with different composition, so that the sample can be selectively localized to the bottom of the reaction chamber.

In some embodiments, selective surface chemistry, e.g., as generically depicted in exemplary reaction vessel 7-2. can be achieved using different techniques for selective surface functionalization and/or surface passivation. For example, reaction vessel 7-2 can comprise a Metal Stack, wherein the exposed portion of the metal at the sidewall surface comprises a metal oxide. In some embodiments, the metal oxide surface can be passivated to render it inert or minimally reactive toward a functionalizing agent and/or a Sample. As shown, the "Oxide" layer can, in some embodiments, refer to a transparent layer composed of silica on its exposed surface(s). In some embodiments, the Oxide or silica surface can be selectively functionalized to confine the Sample at or near the bottom surface of the reaction vessel 7-2.

The incorporation of a specific nucleotide can be distinguished from among four luminescently labeled nucleotides during a sequencing reaction per the exemplary workflow 7-3. Throughout the course of an experiment, there are two distinct periods: a pulse period and a detection period. During the pulse period. lasting 20 picoseconds, no emission light is collected. Following the pulse period is the detection period, lasting 10 nanoseconds, wherein four time bins capture emission events occurring over the detection period (i). A pulse and detection period comprise one cycle. Emission events are continuously binned and accumulated over the course of 1 million cycles (ii). The overall distribution of emission events across time bins are representative of luminescent lifetime and can be used to match a particular set of a data to a known lifetime distribution (*iii*). In some embodiments, the distribution of emission events (e.g., luminescent lifetime) does not distinguish one luminescently labeled base from a plurality of other labeled molecules. In addition to the distribution of emission events, the quantity of emission events (e.g., luminescent intensity) can be used to identify a single molecule from a plurality of others.

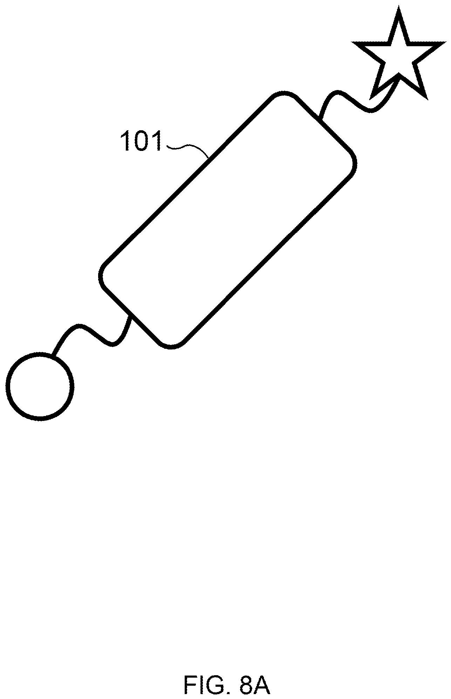

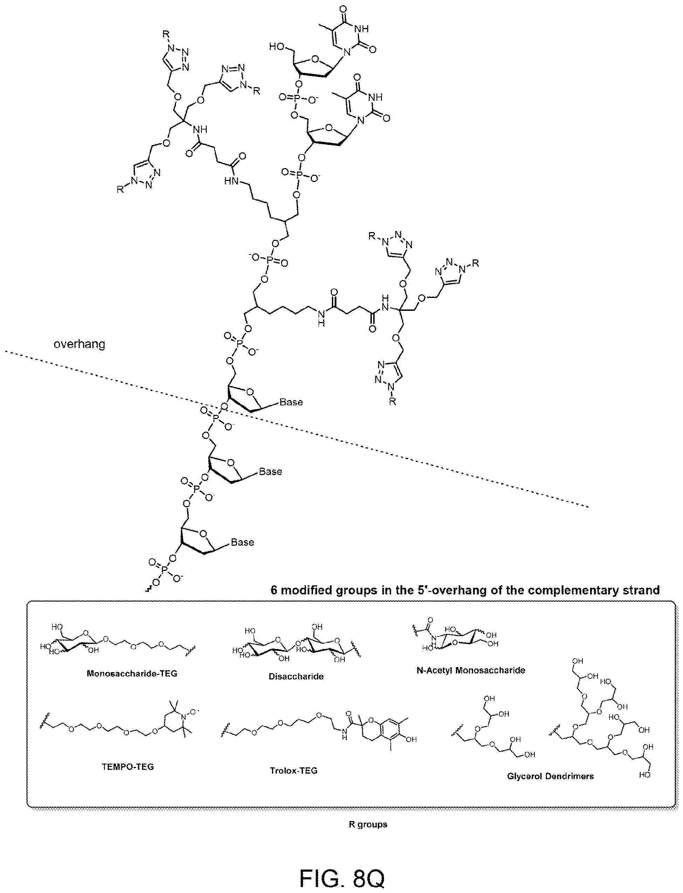

FIG. 8A is a non-limiting example of a luminescent molecule and a nucleotide separated by a non-protein (e.g., nucleic acid) protecting molecule (101). Non-limiting examples of non-protein protecting molecules can include nucleic acid molecules, e.g., deoxyribonucleic acid, ribonucleic acid, and combinations thereof. In some embodiments, a nucleic acid protecting molecule comprises one or more protecting elements (e.g., located between the attachment sites of the one or more nucleotides and the one or more labels). As shown, the luminescent molecule and the nucleotide are attached directly to the non-protein protecting molecule (e.g., covalently attached). In some embodiments, the luminescent molecule and the nucleotide are attached directly to a contiguous part of the non-protein protecting molecule. For example, in some embodiments, the non-protein protecting molecule is a nucleic acid molecule, and the luminescent molecule and/or nucleotide are bound directly to a nucleotide of the nucleic acid molecule. In some embodiments, the luminescent molecule and/or nucleotide are attached to the non-protein protecting molecule via a linker that is not a contiguous part of the non-protein protecting molecule. For example, in some embodiments, the non-protein protecting molecule is a nucleic acid molecule, and the luminescent molecule and/or nucleotide are attached to the nucleic acid via a linker.

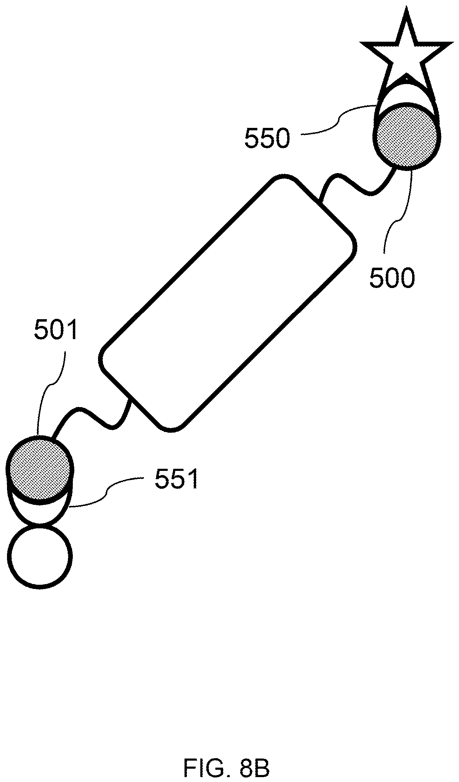

In some embodiments, the luminescent molecule and the nucleotide can be attached to the non-protein (e.g., nucleic acid) protecting molecule via reactive moieties, as depicted in FIG. 8B. In this example, a reactive moiety (550) on the luminescent molecule is covalently attached to the non-protein protecting molecule via a corresponding reactive moiety (500) on the non-protein protecting molecule. A reactive moiety (551) on the nucleotide is covalently attached to the non-protein protecting molecule (101) via a corresponding reactive moiety (501) on the non-protein protecting molecule. In some embodiments, the reactive moiety 500 and/or the reactive moiety 501 are attached directly to a contiguous part of the non-protein protecting molecule. In some embodiments, the reactive moiety 500 and/or the reactive moiety 501 are attached to the non-protein protecting molecule via a linker that is not a contiguous part of the non-protein protecting molecule.

In some embodiments, one or more luminescent labels and/or one or more nucleoside polyphosphates can be attached to a nucleic acid linker (e.g., a non-protein protecting molecule) using chemical coupling techniques known in the art. For example, in some embodiments, click chemistry techniques (e.g., copper-catalyzed, strain-promoted, copper-free click chemistry, etc.) can be used to attach the one or more luminescent labels and the one or more nucleoside polyphosphates to the nucleic acid. Accordingly, the reactive moiety pairs 501/551 and 500/550 depicted in FIG. 8B can include, in some embodiments, reactive amines, azides, alkynes, nitrones, alkenes (e.g., cycloalkenes), tetrazines, tetrazoles, and other reactive moieties suitable for click reactions and similar coupling techniques.

In some embodiments, the non-protein protecting molecule is an oligonucleotide (e.g., a DNA oligonucleotide, an RNA oligonucleotide, or a variant thereof). In some embodiments, the oligonucleotide is single-stranded. In some embodiments, a luminescent label is attached directly or indirectly to one end of the single-stranded oligonucleotide (e.g., the 5' end or the 3' end) and one or more nucleotides are attached directly or indirectly to the other end of the single-stranded oligonucleotide (e.g., the 3' end or the 5' end). For example, the single-stranded oligonucleotide can comprise a luminescent label attached to the 5' end of the oligonucleotide and one or more nucleotides (e.g., of the same type) attached to the 3' end of the oligonucleotide. Alternatively, in some embodiments, the single-stranded oligonucleotide can comprise a luminescent label attached to the 3' end of the oligonucleotide and one or more nucleotides (e.g., of the same type) attached to the 5' end of the oligonucleotide.

In some embodiments, the oligonucleotide is double-stranded (e.g., the oligonucleotide comprises two annealed, complementary oligonucleotide strands). In some embodiments, a luminescent label is attached directly or indirectly to one end of the double-stranded oligonucleotide and one or more nucleotides are attached directly or indirectly to the other end of the double-stranded nucleotide. In some embodiments, a luminescent label is attached directly or indirectly to one strand of the double-stranded oligonucleotide and one or more nucleotides (e.g., of the same type) are attached directly or indirectly to the other strand of the double-stranded nucleotide. For example, the double-stranded oligonucleotide can comprise a luminescent label attached to the 5' end of one strand of the oligonucleotide and one or more nucleotides (e.g., of the same type) attached to the 5' end of the other strand. Alternatively, in some embodiments, the double-stranded oligonucleotide can comprise a luminescent label attached to the 3' end of one strand of the oligonucleotide and one or more nucleotides (e.g., of the same type) attached to the 3' end of the other strand.

In some embodiments, one or more luminescent labels and/or one or more nucleotides (e.g., of the same type) can be attached at one or more different positions on a single-stranded or double-stranded oligonucleotide, including at one or more terminal and/or internal positions of the oligonucleotide.

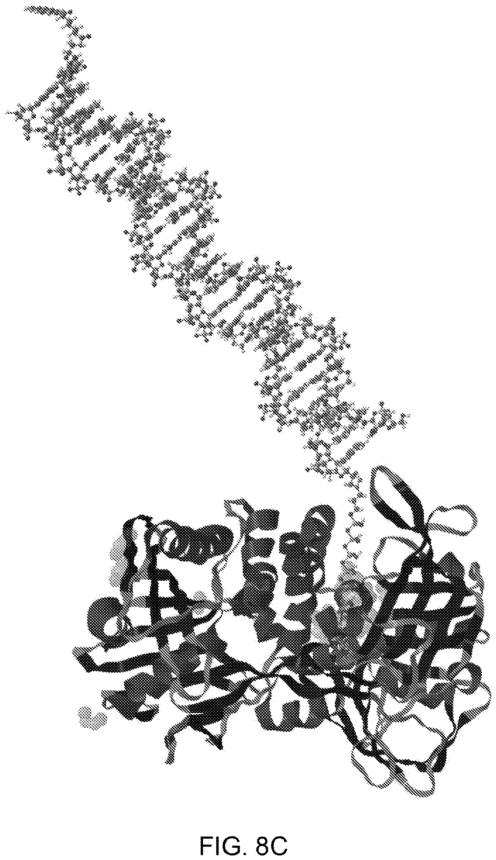

An exemplary embodiment in which a luminescently labeled nucleotide comprises an oligonucleotide protecting molecule is depicted in FIG. 8C. As shown, the oligonucleotide provides a distance between the luminescent label (e.g., one or more dyes) and the polymerase when a nucleotide is bound to the polymerase. Thus, in some embodiments, the oligonucleotide can be useful to function as a steric barrier that prevents or limits the extent of interactions between the luminescent label and the polymerase. Additionally, the oligonucleotide can absorb radiative and/or non-radiative decay emitted by the luminescent molecule. Such functionalities can advantageously protect the polymerase from degradation or loss of function.

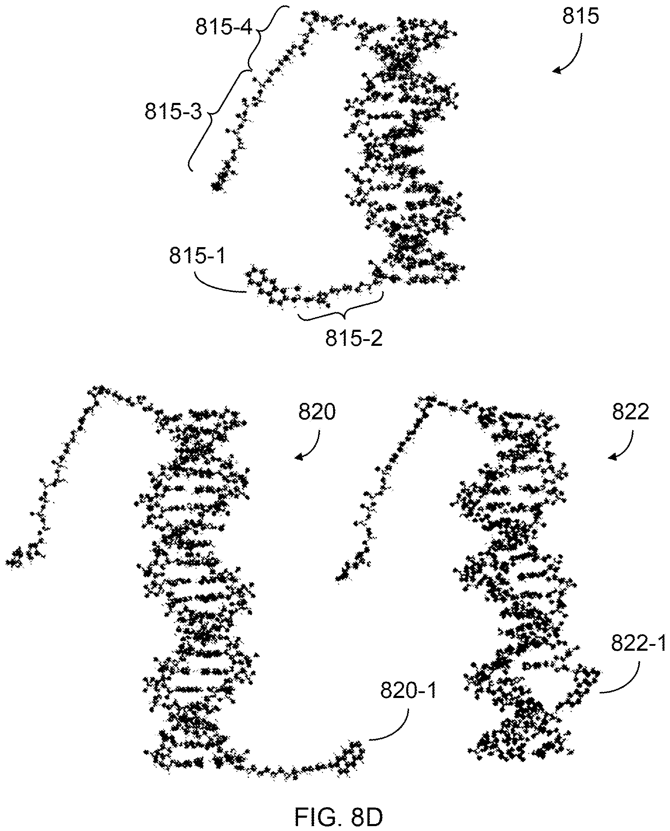

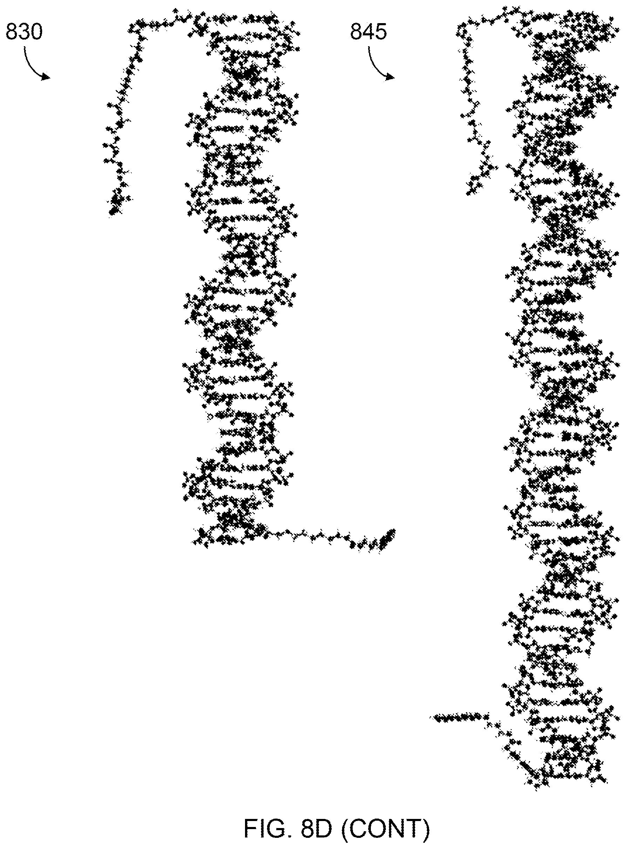

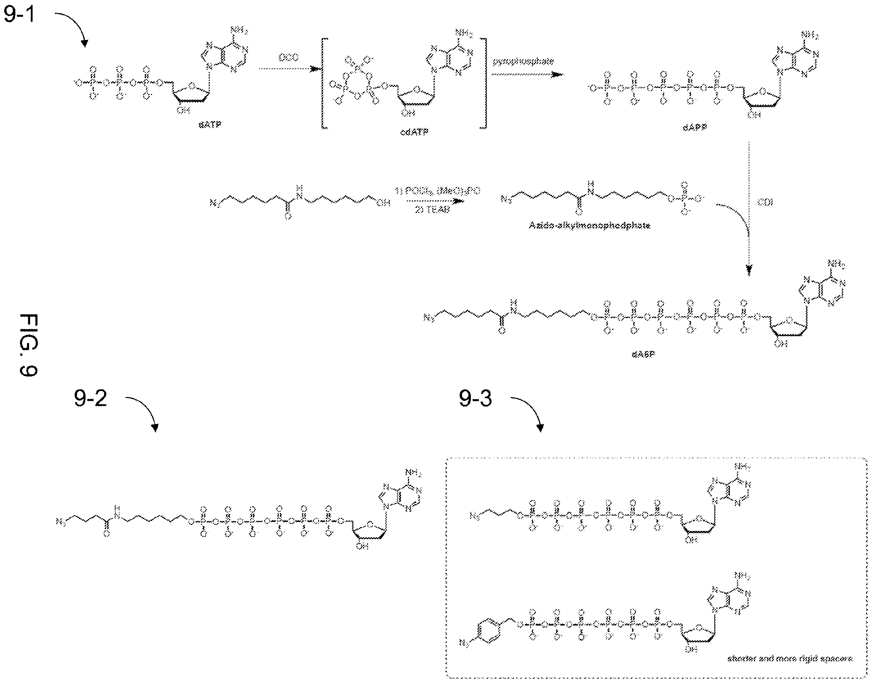

The non-limiting example shown in FIG. 8C depicts a double-stranded oligonucleotide protecting molecule comprising a luminescent label attached to one end (e.g., the 5' end) of one strand and a nucleotide attached to the other end (e.g., the 3' end) of the same strand. In some embodiments, the double-stranded oligonucleotide comprises a luminescent label attached to a first strand and one or more nucleotides attached to a second strand, as depicted in FIG. 8D. As shown, nucleic acid 815 (e.g., a double-stranded oligonucleotide) comprises 15 base pairs. Nucleic acid 815 comprises a luminescent label 815-1 attached to a first oligonucleotide strand of the nucleic acid via a linker 815-2 and a nucleotide 815-3 (e.g., nucleoside polyphosphate depicted as a nucleoside hexaphosphate) attached to a second oligonucleotide strand of the nucleic acid via a linker 815-4. The chemical structure of linker 815-4 attaching nucleotide 815-3 to the nucleic acid 815 is shown in FIG. 9-2, although any suitable linker may be designed according to the linker molecules described elsewhere herein.

FIG. 8D further depicts nucleic acids 820 and 822, each comprising a double-stranded oligonucleotide having 20 base pairs. As shown, nucleic acid 820 comprises an external luminescent label 820-1. In some embodiments, an "external" luminescent label refers to a luminescent label that is attached to a terminal (e.g.. 5' or 3') end of an oligonucleotide strand. In some embodiments, an external luminescent label is attached at a 5'-most or 3'-most base on an oligonucleotide strand. However, in some embodiments, the oligonucleotide strand can comprise additional bases as an overhanging region in which the additional bases do not base pair with an opposite strand of the nucleic acid. In such embodiments, it may be said that the external luminescent label is attached at a base on the oligonucleotide strand that forms a 5'-most or 3'most base pairing with an opposite strand. Nucleic acid 822 comprises an internal luminescent label 822-1. In some embodiments, an "internal'' luminescent label refers to a luminescent label that is attached at a position within an oligonucleotide strand having one or more base-paired nucleotides on either side along the oligonucleotide strand. Thus, if at least one nucleotide upstream and at least one nucleotide downstream from the attachment site along the strand are base-paired with an opposite strand, then the luminescent label can said to be an internal luminescent label. In some embodiments, the internal luminescent label is attached to the oligonucleotide strand via an abasic site or a nucleobase. In some embodiments, the internal luminescent label is integrated within the oligonucleotide backbone.

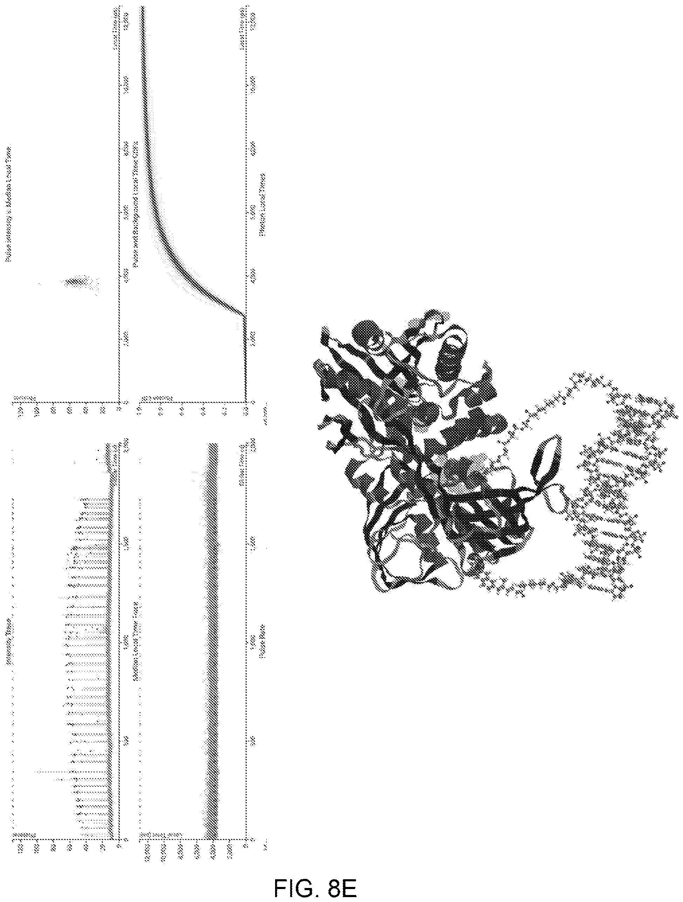

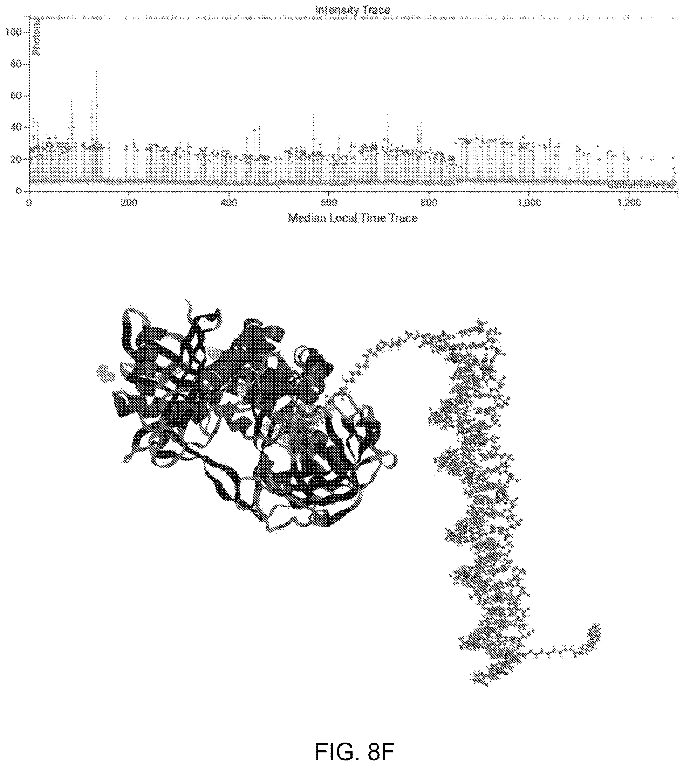

It should be appreciated that oligonucleotide protecting molecules can be of any length, as shown by the varied lengths of the exemplary constructs in FIG. 8D (e.g., a 15 base-pair nucleic acid 815, a 20 base-pair nucleic acid 820, a 30 base-pair nucleic acid 830. and a 45 base-pair nucleic acid 845). For example, exemplary oligonucleotides consisting of 15 base pairs and 25 base pairs are shown in FIG. 8E and FIG. 8F, respectively, along with exemplary results from sequencing experiments. In some embodiments, an oligonucleotide can have a length of about 10 or more, about 20 or more, about 30 or more, about 40 or more, about 50 or more, about 60 or more, about 70 or more, about 80 or more, about 90 or more, about 100 or more, about 125 or more, about 150 or more, about 175 or more, about 200 or more, about 250 or more, about 300 or more, about 350 or more, about 400 or more, about 450 or more, about 500 or more. In some embodiments, the oligonucleotide has a length of less than 20, less than 30, less than 40. less than 50, less than 60, less than 70, less than 80, less than 90, less than 100, less than 125, less than 150, less than 175, less than 200, less than 250, less than 300, less than 350, less than 400, less than 450, less than 500, or less than 600 bases. In some embodiments, the oligonucleotide linker is sufficiently long to protect the polymerase from emissions from the label that is attached to the oligonucleotide linker, while also being short enough to allow the label to be excited and detected during a sequencing reaction.

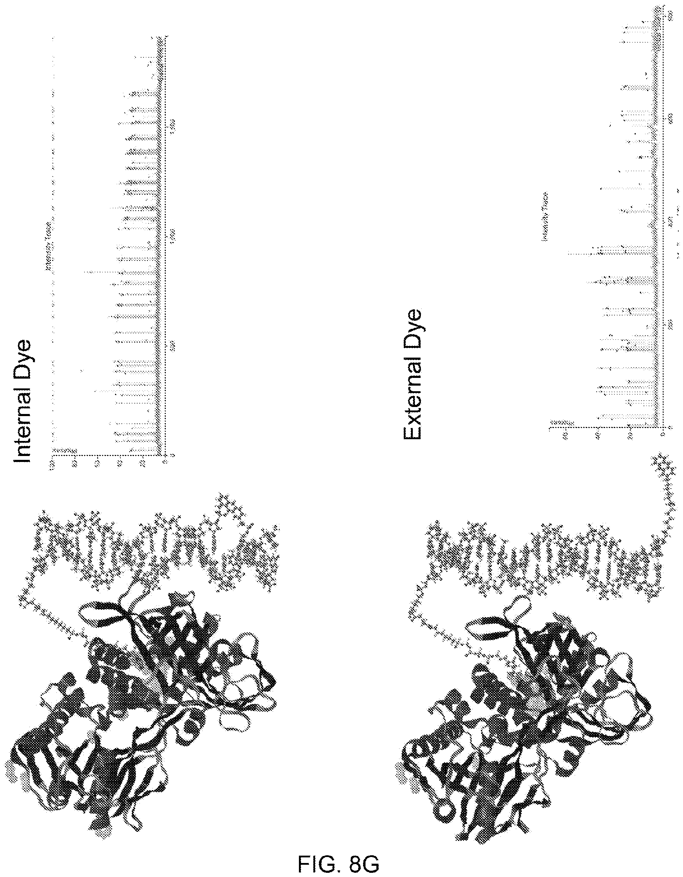

It should be appreciated that the luminescent label and/or the one or more nucleotides can be attached at any position in the oligonucleotide. In some embodiments, the luminescent label and/or one or more nucleotides are attached at or approximately at the 5' or 3' end. In some embodiments, the luminescent label is attached at an internal position of the oligonucleotide such that the luminescent label is in close proximity to a larger portion of the oligonucleotide. For example, FIG. 8G depicts an exemplary oligonucleotide comprising an internal dye and an exemplary oligonucleotide comprising an external dye, along with exemplary results utilizing either one in sequencing experiments. In some embodiments, an internal luminescent label (e.g., an internal dye) generally refers to a luminescent label that is surrounded by a larger portion of the oligonucleotide in close proximity when compared to an external luminescent label (e.g., an external dye). In some embodiments, the larger portion of the oligonucleotide surrounding the luminescent label provides a greater barrier for absorbing radiative and/or non-radiative decay emitted by the luminescent label.

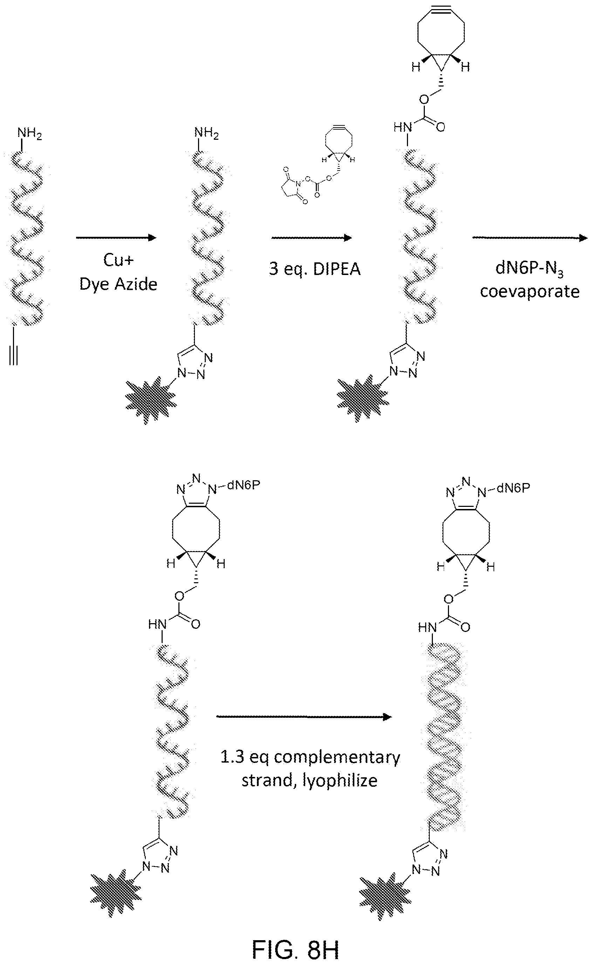

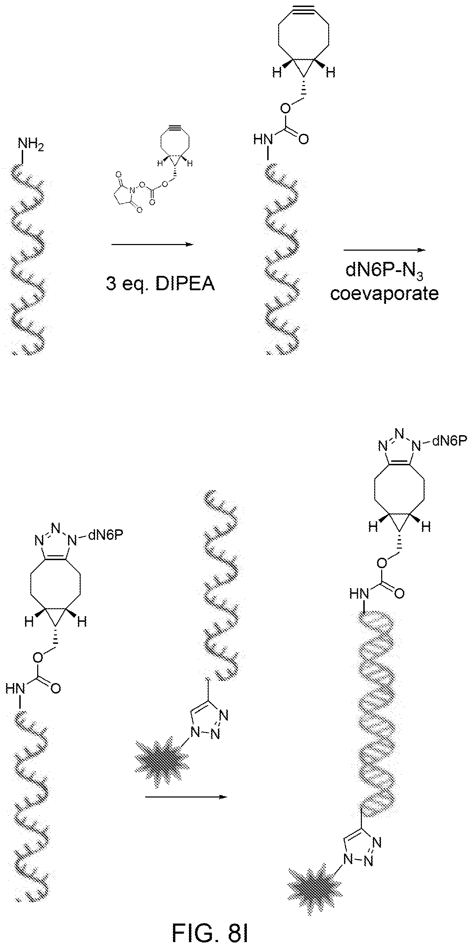

Luminescently labeled nucleotides comprising oligonucleotide protecting molecules can be generated using any method known in the art. For example, FIG. 8H depicts one non-limiting approach wherein a luminescent label and a nucleotide are attached at opposite ends of the same strand of the oligonucleotide. In this embodiment, the luminescent label and the nucleotide are attached to a first strand using reactive group chemistry, and a second, complementary strand is subsequently annealed to the first strand. As indicated above, in some embodiments, a luminescent label and one or more nucleotides can be attached to different strands of an oligonucleotide protecting molecule (e.g., at the 3' and/or 5' ends). FIG. 8I depicts an exemplary method wherein a first strand comprising a luminescent label is annealed to a second, complementary strand comprising a nucleotide.

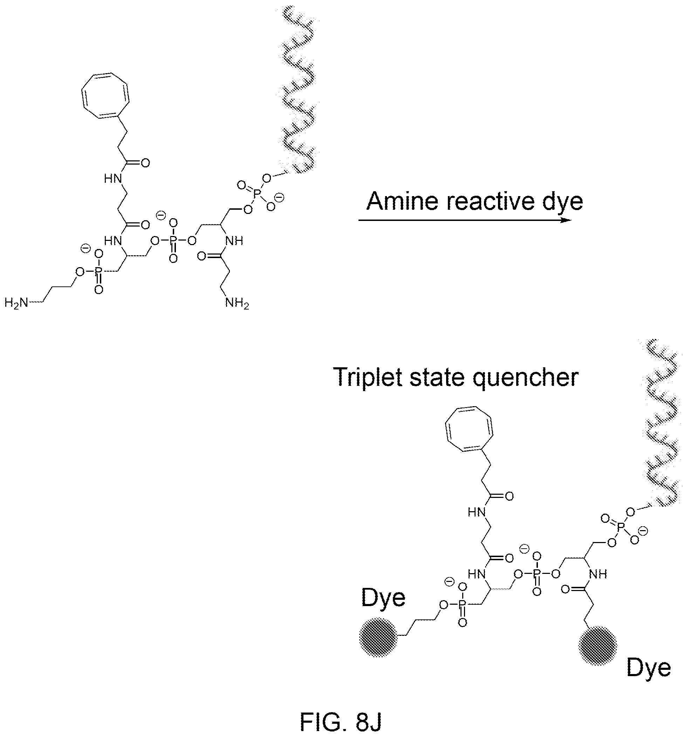

As described herein, a protecting molecule can comprise a luminescent label (e.g., a label having one or more dyes). In some embodiments, the protecting molecule can comprise one or more moieties (e.g., energy-absorbing moieties) useful for absorbing radiative and/or non-radiative decay emitted from a luminescent label. For example, FIG. 8J depicts a non-limiting method of attaching multiple dyes to a strand of an exemplary oligonucleotide protecting molecule, wherein reactive group chemistry is used to covalently attach two dyes to a linker on the oligonucleotide. As shown, the linker comprises a triplet state quencher, which can absorb any potentially deleterious effects arising from a luminescent label being excited into the highly reactive state. In the example shown in FIG. 8J, the energy-absorbing moiety is shown covalently attached to the same strand as the luminescent label (e.g., dye molecules). In some embodiments, the energy-absorbing moiety can be adjacent to the luminescent label, but attached to a complementary strand that is annealed to the strand having the luminescent label, as described below.

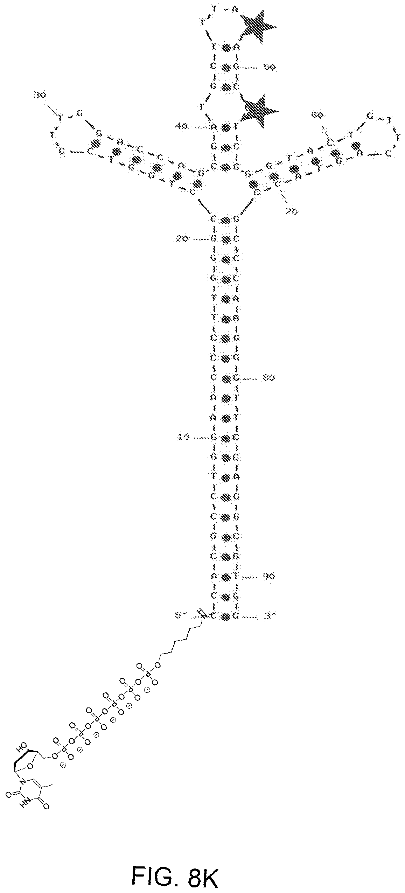

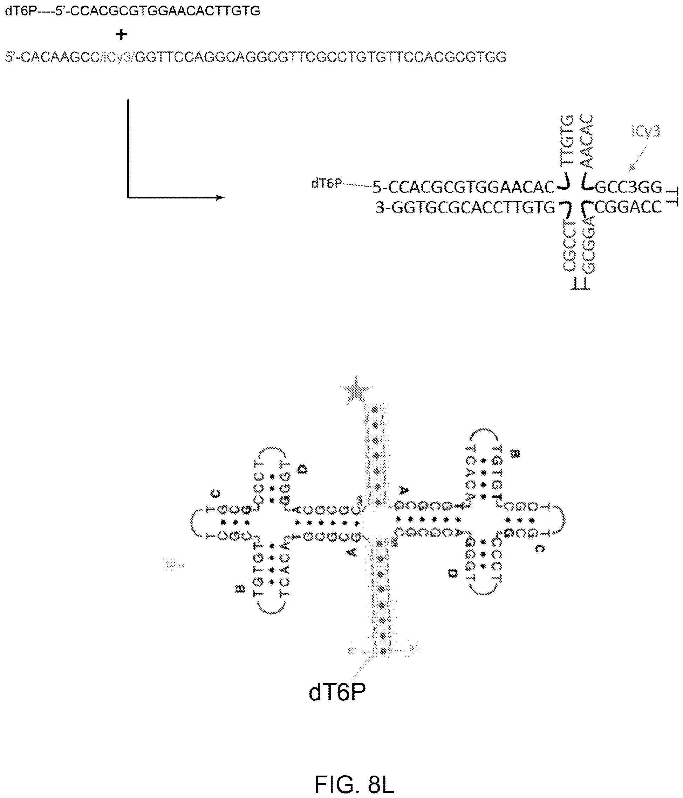

In addition to chemical moieties that provide polymerase protecting effects, oligonucleotide structural motifs are contemplated herein. In some embodiments, one or more unlabeled structural motifs (e.g., an oligonucleotide sequence capable of forming a stable stem-loop or other structure) are located on the nucleic acid linker between the luminescent label and the nucleoside polyphosphate (e.g., an unlabeled stem structure is present between the position of a label and a position of a nucleoside polyphosphate). For example, FIG. 8K depicts an oligonucleotide protecting molecule comprising three stem loop structures. In some embodiments, an oligonucleotide protecting molecule can comprise one or more stem loop structures (e.g., 2, 3, 4, 5, 6, 7, 8, 9, or more) and can be single-stranded. In some embodiments, an oligonucleotide protecting molecule can comprise one or more stem loop structures (e.g., 2, 3, 4. 5, 6, 7, 8, 9, or more) and can be double-stranded. For example, FIG. 8L depicts exemplary double-stranded oligonucleotide protecting molecules comprising stem loop structures. In some embodiments, the increased structure in the region between the luminescent label and one or more nucleotides provided by the stem loops provides a larger area in which the oligonucleotide can sterically hinder interactions between the luminescent label and a polymerase.

In some embodiments, the oligonucleotide protecting molecule comprises one stem loop (e.g., a hairpin structure). In some embodiments, the oligonucleotide protecting molecule comprises two stem loops. In some embodiments, the oligonucleotide protecting molecule comprises three stem loops. In some embodiments, the oligonucleotide protecting molecule comprises four stem loops. In some embodiments, the oligonucleotide protecting molecule comprises 5, 6, 7, 8. 9, or 10 or more stem loops.

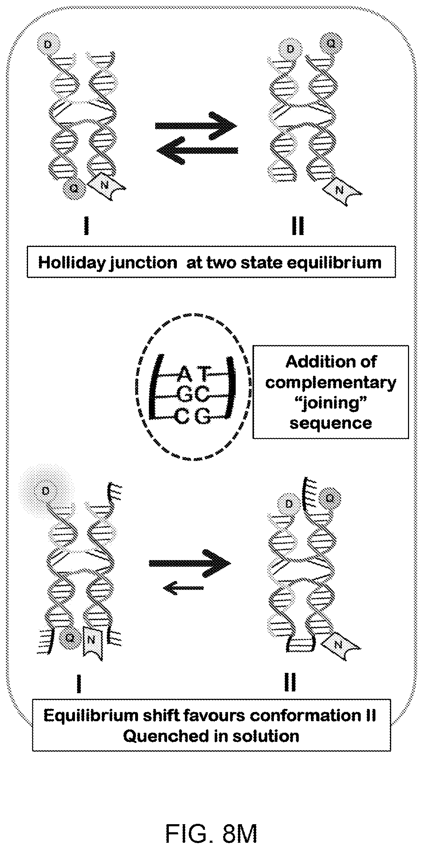

Additional nucleic acid structural motifs are contemplated for an oligonucleotide protecting molecule described herein. In some embodiments, the oligonucleotide protecting molecule comprises three or more nucleic acid strands (e.g., 3, 4, 5, 6, 7, or 8 or more nucleic acid strands). In some embodiments, the oligonucleotide protecting molecule comprises four nucleic acid strands. For example, the oligonucleotide protecting molecule can comprise a Holliday junction, as depicted in FIG. 8M. One or more structural motifs (e.g., stem loops, Holliday junctions) can, in some embodiments, increase the rigidity of the oligonucleotide protecting molecule to maximize the average distance between a luminescent label and a polymerase. In some embodiments, the relevant average distance refers to the distance between the luminescent label and the polymerase when a nucleoside polyphosphate attached to the label is in the polymerase active site (e.g., during incorporation into a growing strand).

In some embodiments, additional structural motifs (e.g., based on physical properties conferred by the sequence of the oligonucleotide protecting molecule, and/or based on one or more chemical modifications of one or more portions of the oligonucleotide protecting molecule) can be included in a single-stranded or double-stranded oligonucleotide protecting molecule, for example to increase the rigidity or to provide particular 3-dimensional configuration. In some embodiments, one or more annealed regions (e.g., stem structures, or hybridized complementary regions of two oligonucleotides) can be stabilized (e.g., by covalent modification).

In some embodiments, one or more positions on an oligonucleotide protecting molecule can be modified to include one or more energy absorbing moieties. In some embodiments, one or more energy absorbing moieties can be added to the same nucleic acid strand that the label is attached to (see FIG. 8J as a non-limiting example). In some embodiments, one or more energy absorbing moieties can be added to a different strand from the strand that is labeled in the context of a double-stranded oligonucleotide protecting molecule (e.g., the one or more energy absorbing moieties can be included on the complementary strand that is attached to one or more nucleotides), for example as described in more detail below.

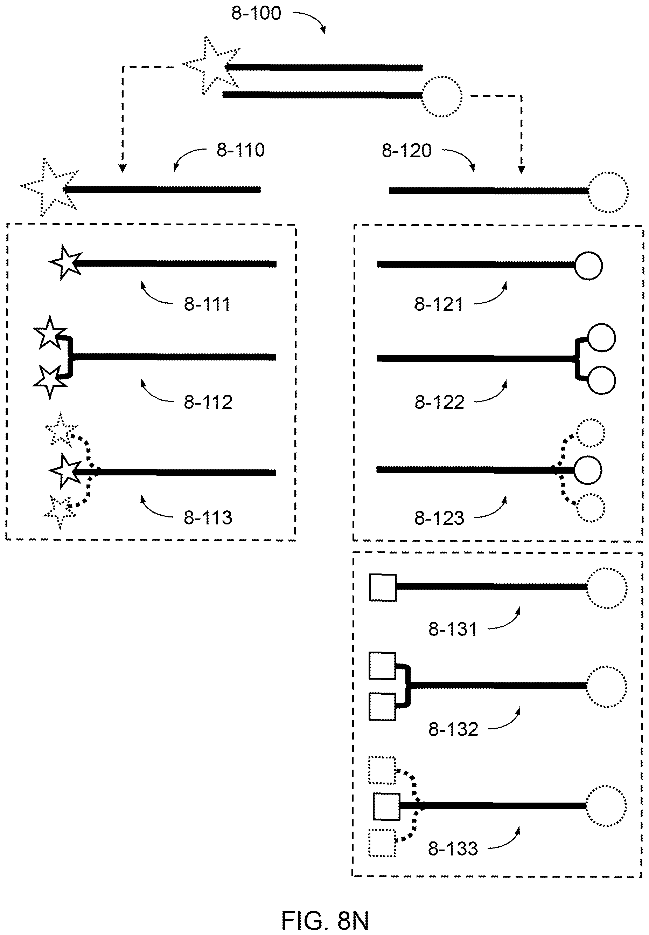

Accordingly, in some embodiments, a luminescently labeled nucleotide can be provided in a generic form 8-100 depicted in FIG. 8N. As shown, a luminescent label domain (star shape, dotted line) is non-covalently linked to a nucleotide domain (circle shape, dotted line) via a dimerization domain. In some embodiments, the dimerization domain refers to a region that provides a non-covalent linkage between the luminescent label domain and the nucleotide domain. For example, in some embodiments, the generic structure 8-100 is a complementary oligonucleotide dimer comprising a labeled oligonucleotide strand 8-110 comprising a luminescent label domain and an unlabeled oligonucleotide strand 8-120 comprising a nucleotide domain. It should be understood that, in the context of an oligonucleotide protecting molecule (e.g., an oligonucleotide dimer), a nucleotide domain refers to the one or more nucleotides (e.g., nucleoside phosphates) that are configured to be incorporated into a growing nucleic acid strand (e.g., during a sequencing reaction). In some embodiments, the one or more nucleotides comprise one or more nucleoside monophosphates or nucleoside polyphosphates (e.g., nucleoside di- or triphosphates, or nucleosides with more than three 5' phosphates, etc.). In some embodiments, the one or more nucleoside phosphates (e.g., nucleoside polyphosphates) may be attached through a terminal phosphate to an oligonucleotide (e.g., an unlabeled oligonucleotide strand) that forms part of a protecting molecule as described in this application. In some embodiments of any of the compositions or methods described in this application, a phosphate portion (e.g., a polyphosphate portion) of a nucleoside phosphate (e.g., of a nucleoside polyphosphate) includes one or more phosphates or variants thereof. For example, in some embodiments, a phosphate portion (e.g., a polyphosphate portion) of a nucleoside phosphate (e.g., of a nucleoside polyphosphate) can include a phosphate ester, a thioester, a phosphoramidate, an alkyl phosphonate linkage, other suitable linkage, or more than one such modifications, or a combination of two or more thereof. In some embodiments, the labeled and unlabeled strands are substantially complementary to one another (e.g., over the length of a dimerization domain wherein the strands within the dimerization domain can have, for example, at least 60%, at least 70%, at least 75%, at least 80%, at least 85%, at least 90%, at least 95%, at least 98%, at least 99%, or 100% complementary to one another). In such embodiments, the labeled and unlabeled strands may be annealed to form a double-stranded construct, where the annealed portion constitutes the dimerization domain, and the annealing of the two strands produces the luminescently labeled nucleotide 8-100. In some embodiments, a dimerization domain contains 5 to 100 base pairs, for example, 10 to 50 base pairs, 15 to 45 base pairs, or other range of base pairs, for example based on a combination of any of the following non-limiting sizes. In some embodiments, a dimerization domain contains at least 5, at least 10, at least 1 5, at least 20, at least 25, at least 30, at least 35, at least 45, at least 50, at least 55, at least 60, at least 65, at least 70, at least 75, at least 100, at least 250 base pairs. In some embodiments, a dimerization domain contains no more than 500, no more than 300, no more than 200, no more than 150, no more than 100, no more than 75, no more than 70, no more than 65, no more than 60, no more than 55, no more than 50, no more than 45, no more than 40, no more than 35, no more than 30, no more than 25, no more than 20, no more than 15, no more than 10 base pairs. In some embodiments, the dimerization domain contains 15 base pairs. In some embodiments, the dimerization domain contains 30 base pairs. In some embodiments, the dimerization domain contains 45 base pairs.

In some embodiments, the luminescent label domain and the nucleotide domain may be advantageously separated by a dimerization domain. For example, in some embodiments, where generic form 8-100 refers to a double-stranded oligonucleotide construct, the luminescent label domain and nucleotide domain are depicted at opposing ends of an annealed oligonucleotide dimer. Accordingly, in some embodiments, the luminescent label domain and the nucleotide domain are each attached at or near the 3' end (e.g.. attached at the 3'-most nucleotide, attached at a nucleotide located within 1%, 2%, 5%, 10%, 15%, 20%, 25%, 30%, 35%, 40%, or 45% of the 3'-most nucleotides in an oligonucleotide sequence) of the labeled and unlabeled strands, respectively. In some embodiments, the luminescent label domain and the nucleotide domain are each attached at or near the 5' end (e.g., attached at the 5'-most nucleotide, attached at a nucleotide located within 1%, 2%, 5%, 10%, 15%, 20%, 25%, 30%, 35%, 40%, or 45% of the 5'-most nucleotides in an oligonucleotide sequence) of the labeled and unlabeled strands, respectively.

As shown in FIG. 8N, a labeled strand 8-110 comprises a luminescent label domain (star shape, dotted line). In some embodiments, a luminescent label domain refers to a region of the labeled strand having one or more luminescent molecules. For example, in some embodiments, a luminescent label domain of a labeled strand 8-111 consists of one luminescent molecule (star shape, solid line). In some embodiments, a luminescent label domain of a labeled strand 8-112 consists of two luminescent molecules. As depicted in structure 8-112, a luminescent label domain having more than one luminescent molecule may resemble a branched structure. It should be appreciated that the number of luminescent molecules to be included on a single labeled strand may be left up to the practitioner based on desired properties (e.g., luminescent properties such as lifetime, intensity, quantum yield, etc.). Non-limiting examples of the number of luminescent molecules attached to a luminescent label domain include 2-10, (e.g., 2, 3, 4, 5, 6, 7, 8, 9, 10), 10-15, 15-20, or other suitable number. The luminescent molecules can be attached in a linear, branched, combination of linear and branched, or other configuration. Accordingly, a generic labeled strand 8-113 is shown with a luminescent label domain having a first luminescent molecule and two additional branched luminescent label moieties (dotted lines), each luminescent label moiety having one or more luminescent molecules.

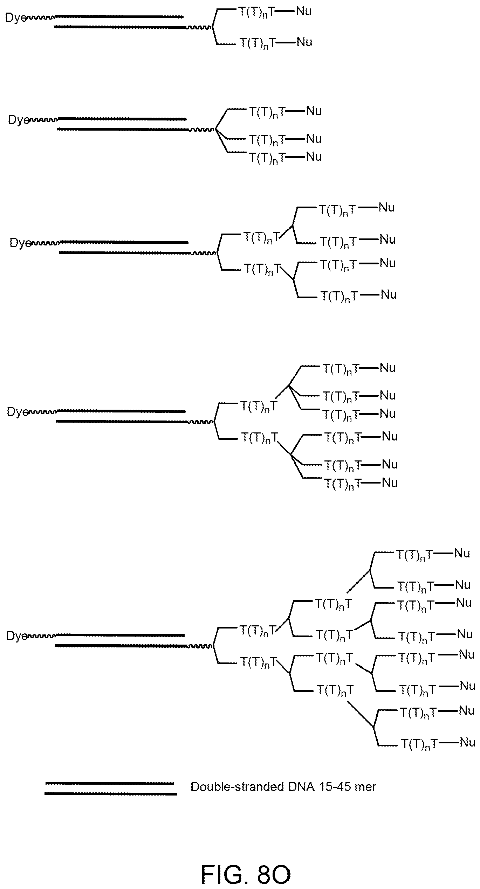

In some embodiments, a nucleotide domain of an unlabeled strand may be designed in a similar fashion. For example, as shown in FIG. 8N. in some embodiments, an unlabeled strand 8-120 comprises a nucleotide domain (circle shape, dotted line). In some embodiments, a nucleotide domain refers to a region of the unlabeled strand having one or more nucleotides (e.g., one or more nucleoside polyphosphates). In some embodiments, a nucleotide domain of an unlabeled strand 8-121 consists of one nucleotide (circle shape, solid line). In some embodiments, a nucleotide domain of an unlabeled strand 8-122 consists of two nucleotides. As depicted in structure 8-122, a nucleotide domain having more than one nucleotide may resemble a branched structure. It should be appreciated that the number of nucleotides to be included in a nucleotide domain on a single unlabeled strand may be left up to the practitioner based on desired properties (e.g., properties in a nucleic acid sequencing reaction, such as reaction kinetics, read length, proximity to polymerizing enzyme, etc.). Non-limiting examples of the number of nucleotides attached to a nucleotide domain include 2-10, (e.g., 2, 3, 4, 5, 6, 7, 8, 9, 10), 10-15, 15-20, or other suitable number. The nucleotides can be attached in a linear, branched, combination of linear and branched, or other configuration. Accordingly, a generic unlabeled strand 8-123 is shown with a nucleotide domain having a first nucleotide and two additional branched nucleotide moieties (dotted lines), each nucleotide moiety having one or more nucleotides. Non-limiting examples of branched nucleotides are shown in FIG. 8O, which depicts various unlabeled strand configurations having more than one nucleotide (Nu) in a nucleotide domain via branched thymidine oligonucleotide linkers. Although thymidine is depicted in the linkers by way of example, in some embodiments, alternative oligonucleotide linkers may be used (e.g., cytidine, uridine, adenosine, guanosine, or any combination thereof). In some embodiments, an unlabeled strand may comprise additional modifications which may be useful, for example, in a nucleic acid sequencing reaction.

As depicted in FIG. 8N, an unlabeled strand 8-131 comprising a nucleotide domain may further comprise an energy-absorbing modification (square shape, solid line). In some embodiments, an energy-absorbing modification refers to any chemical moiety that protects the integrity of a nucleic acid sequencing reaction (e.g., from the damaging effects on the polymerase of reactive species generated by an excited state of the label). For example, in some embodiments, the energy-absorbing modification protects the integrity of a sequencing reaction by any of the following: absorbing, quenching, or otherwise mitigating the effects of a reactive species (e.g., reactive oxygen species, free radicals, or any triplet state molecules); providing a steric barrier between a luminescent label domain and a nucleotide domain; increasing read length in a sequencing reaction when compared to a reaction conducted in the absence of the energy-absorbing modification; increasing read accuracy in a sequencing reaction when compared to a reaction conducted in the absence of the energy-absorbing modification; or any combination thereof.

As shown, in some embodiments, the energy-absorbing modification is at or near an end of the unlabeled strand that is opposite from the nucleotide domain. As should be appreciated from the generic oligonucleotide dimer 8-100, the energy-absorbing modification in the unlabeled strand 8-131 may be advantageously positioned adjacent to the luminescent label domain in an annealed construct. Accordingly, in some embodiments, where a luminescent label domain and a nucleotide domain are attached at or near the 3' ends of their respective strands, the energy-absorbing modification is attached at or near the 5' end (e.g., attached at the 5'-most nucleotide, attached at a nucleotide located within 1%, 2%, 5%, 10%, 15%, 20%, 25%, 30%, 35%, 40%, or 45% of the 5'-most nucleotides in an oligonucleotide sequence) of the unlabeled strand. In some embodiments, where a luminescent label domain and a nucleotide domain are attached at or near the 5' ends of their respective strands, the energy-absorbing modification is attached at or near the 3' end (e.g., attached at the 3'-most nucleotide, attached at a nucleotide located within 1%, 2%, 5%, 10%, 15%, 20%, 25%, 30%, 35%, 40%, or 45% of the 3'-most nucleotides in an oligonucleotide sequence) of the unlabeled strand. In some embodiments, an unlabeled strand may comprise more than one energy-absorbing modification. In some embodiments, energy-absorbing modification(s) may be included within an oligonucleotide overhang (e.g., a 3' or 5' overhang) that is not part of the complementary dimerization domain. The overhang can be of any suitable length, for example from 1 to 10 (e.g., around 5), or 10 to 20 nucleotides in length (or other suitable length).

For example, in some embodiments, an unlabeled strand 8-132 comprises two energy-absorbing modifications. As depicted in structure 8-132, an unlabeled strand having more than one energy-absorbing modification may resemble a branched structure. It should be appreciated that an unlabeled strand may comprise any number of energy-absorbing modifications, which may vary, for example, depending on the properties of a given energy-absorbing modification or the properties and/or number of luminescent molecule(s) in a luminescent label domain. Non-limiting examples of the number of energy-absorbing modifications (e.g., attached to either one or both strands) include 2-10, (e.g., 2, 3, 4, 5, 6, 7, 8, 9, 10), 10-15, 15-20, or other suitable number. The energy-absorbing modifications can be attached in a linear, branched, combination of linear and branched, or other configuration. Accordingly, a generic unlabeled strand 8-133 is shown with a first energy-absorbing modification and two additional branched energy-absorbing domains (dotted lines), each energy-absorbing domain having one or more energy-absorbing modifications.

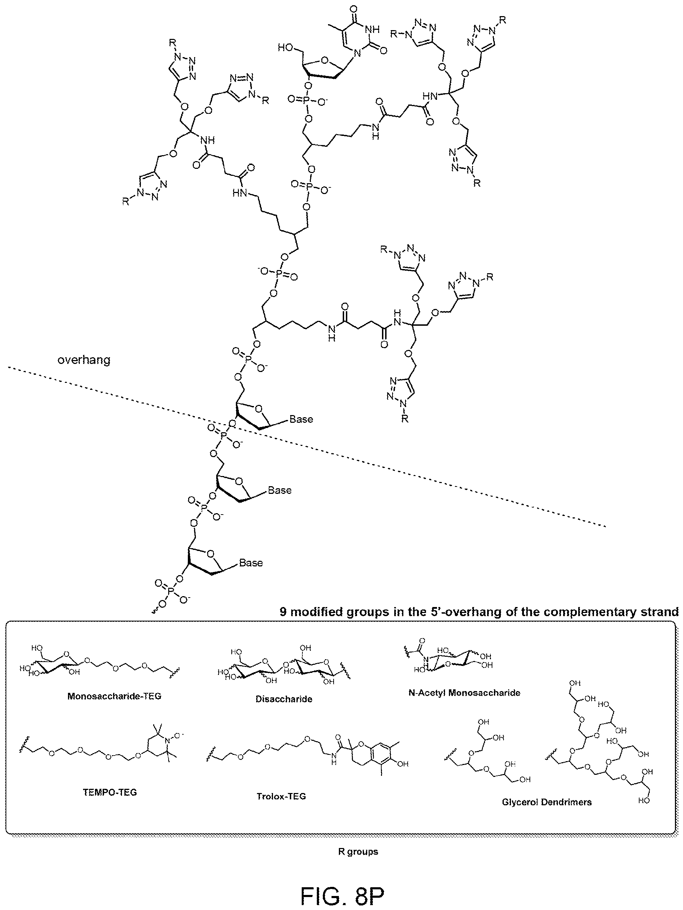

In some embodiments, one or more energy-absorbing modifications may be made to an unlabeled strand according to the non-limiting structures depicted in FIGs. 8P-8Q. In some embodiments, one or more energy-absorbing modifications may be made to an unlabeled strand according to the non-limiting structures depicted below. In these examples, the one or more energy-absorbing modifications are shown attached to a 5' end of an oligonucleotide strand (e.g., an unlabeled oligonucleotide strand).

In some embodiments, a nucleic acid linker comprises one or more energy-absorbing modifications. In some embodiments, the one or more energy-absorbing modifications comprise a plurality of quenching moieties (e.g., triplet-state quenchers). Quenching moieties can comprise any dye molecule that can detectably quench an emission from the one or more luminescent labels described herein. A suitable quenching moiety can be selected based on the specific types of dye molecule(s) of the one or more luminescent labels. For example, in some embodiments, an appropriate quenching moiety possesses an absorption band that exhibits at least some spectral overlap with an emission band of the luminescent label. In some embodiments, this overlap may occur with emission of the donor occurring at a lower or even higher wavelength emission maximum than the maximal absorbance wavelength of the quenching moiety, provided that sufficient spectral overlap exists. In some embodiments, energy transfer may also occur through transfer of emission of the donor to higher electronic states of the acceptor. In some embodiments, an appropriate quenching moiety is capable of absorbing emissions of a particular energy level from the one or more luminescent labels that may be damaging to a polymerase. In such embodiments, a quenching moiety can be selected to preferentially (e.g., selectively) absorb a portion of the emission spectrum that includes emission energy levels potentially damaging to the polymerase without absorbing the portion of the emission spectrum that is being detected as a signal (e.g., during a sequencing reaction).

A wide variety of chemically reactive dyes and fluorophores that are suitable for use as a quenching moiety are known in the art (see for example MOLECULAR PROBES HANDBOOK, Sixth Ed., Richard P. Haugland, ed. (1996), in particular Chapters 1-3; MOLECULAR PROBES HANDBOOK, Seventh Ed., Richard P. Haugland, ed.; BIOPROBES 26 (October 1997); BIOPROBES 27 (February 1998); BIOPROBES 28 (May 1998); BIOPROBES 29 (November 1998); BIOPROBES 30 (January 1999); BIOPROBES 31 (May 1999): BIOPROBES 32 (December 1999); and BIOPROBES 33 (February 2000); all incorporated herein by reference). The spectral properties of candidate dyes in solution or when conjugated to oligonucleotides are known or are readily measured using a spectrofluorometer.

In some embodiments, the quenching moiety is a pyrene, an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine, a carbocyanine, a carbostyryl, a porphyrin, a salicylate, an anthranilate, an azulene, a perylene, a pyridine, a quinoline, a coumarin (including hydroxycoumarins and aminocoumarins and fluorinated and sulfonated derivatives thereof (as described in U.S. Pat. No. 5,830,912 to Gee et al. (1998) and U.S. Pat. No. 5,696,157 to Wang et al. (1997), incorporated herein by reference), a polyazaindacene (e.g. U.S. Pat. No. 4,774,339 to Haugland, et al. (1988); U.S. Pat. No. 5,187,288 to Kang, et al. (1993); U.S. Pat. No. 5,248,782 to Haugland, et al. (1993); U.S. Pat. No. 5,274,1 13 to Kang, et al. (1993); 5,433,896 to Kang, et al.(1995); U.S. Pat. No. 6,005,113 to Wu et al. (1999), all incorporated herein by reference), a xanthene, an oxazine or a benzoxazine, a carbazine (U. S. Pat. No. 4,810,636 to Corey (1989), incorporated by reference), or a phenalenone or benzphenalenone (U.S. Pat. No. 4,812,409 Babb et al. (1989), incorporated by reference).

In some embodiments, the quenching moiety is a non-fluorescent dye. For example, in some embodiments, the quenching moiety can be selected from azo dyes (such as DABCYL or DABSYL dyes and their structural analogs), triarylmethane dyes such as malachite green or phenol red, 4',5z-diether substituted fluoresceins (U.S. Pat. No. 4,318,846 (1982)), or asymmetric cyanine dye quenchers (PCT Int. App. WO 99 37,717 (1999)).

In some embodiments, a nucleic acid linker comprises an unlabeled strand having two or more (e.g.. three) quenching moieties. For example, in some embodiments, an unlabeled strand comprises a tris-trolox modified strand depicted below. However, other similar structures could be used and incorporated into a nucleic acid linker (e.g., at a location between the luminescent label(s) and the nucleoside polyphosphate(s)).

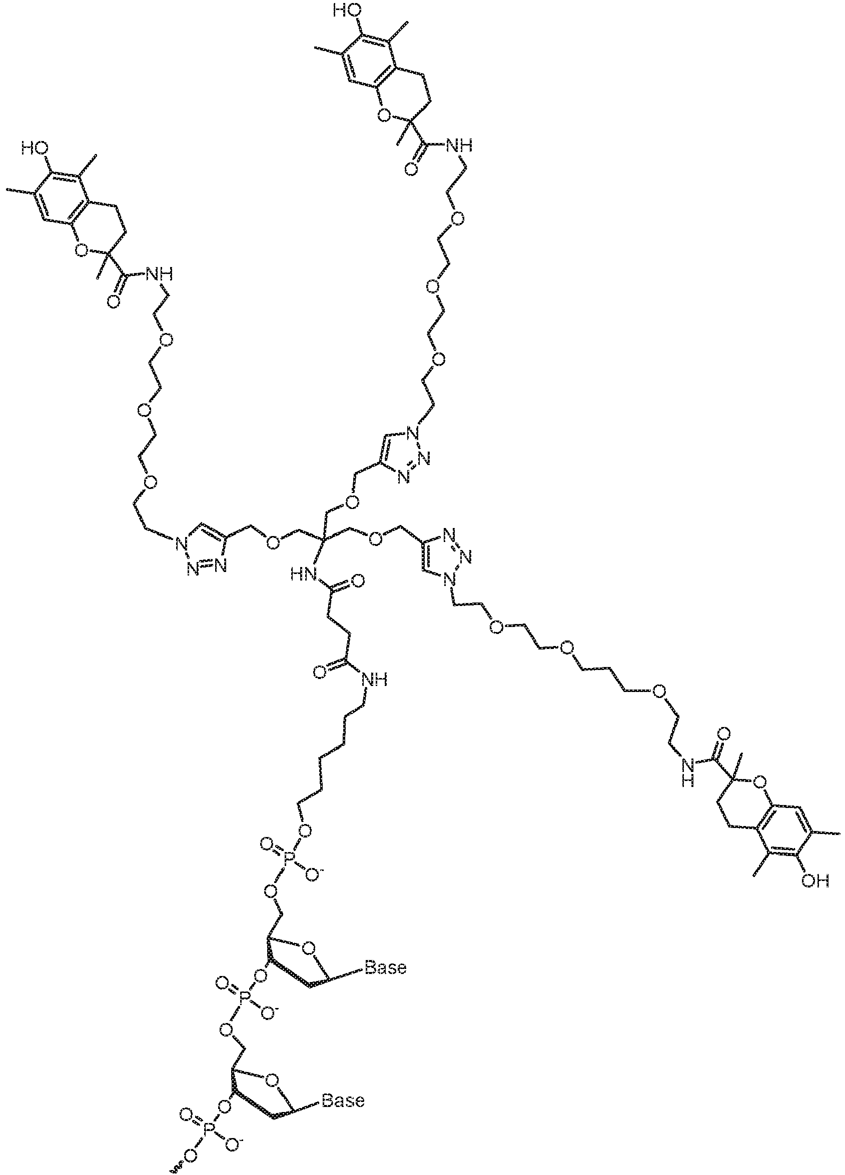

In some embodiments, an unlabeled strand comprising three quenching moieties is a tris-tempo modified strand:

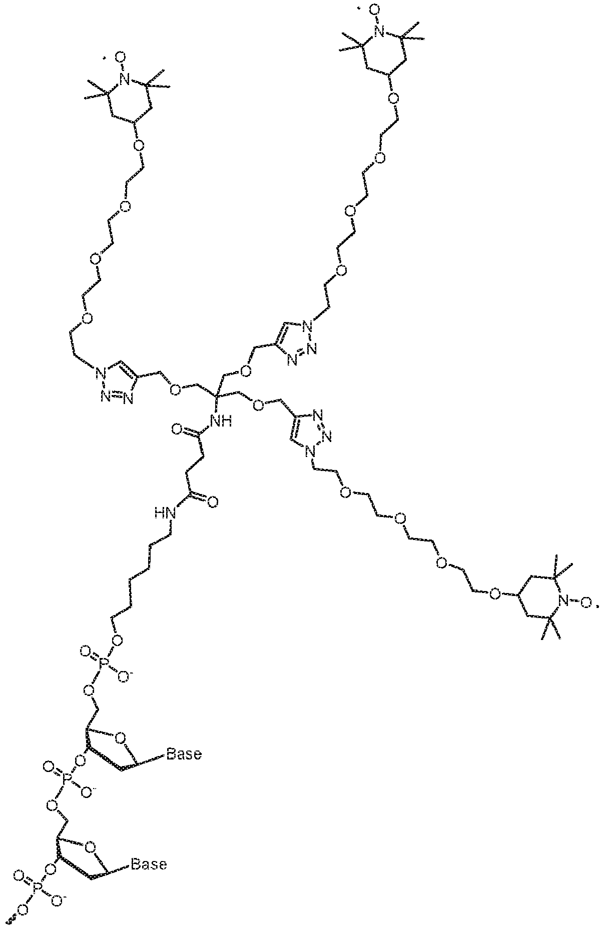

In some embodiments, a nucleic acid linker comprises one or more energy-absorbing modifications that do not function as chromophores. In such embodiments, the one or more energy-absorbing modifications can function by providing steric barriers between a luminescent label and a nucleoside polyphosphate that prevent the luminescent label from interacting with a polymerase. In some embodiments, a nucleic acid linker comprises a branched polymeric structure that functions as a steric barrier. In some embodiments, the branched polymeric structure comprises two or more polyether-based structures. For example, in some embodiments, the branched polymeric structure comprises polyethylene glycol (PEG)-based structures, as shown in the below structure depicting a terminal modification of an unlabeled strand. However, it should be appreciated that other similar branching structures could be used and incorporated into a nucleic acid linker (e.g., at a location between the luminescent label and the nucleoside polyphosphate).

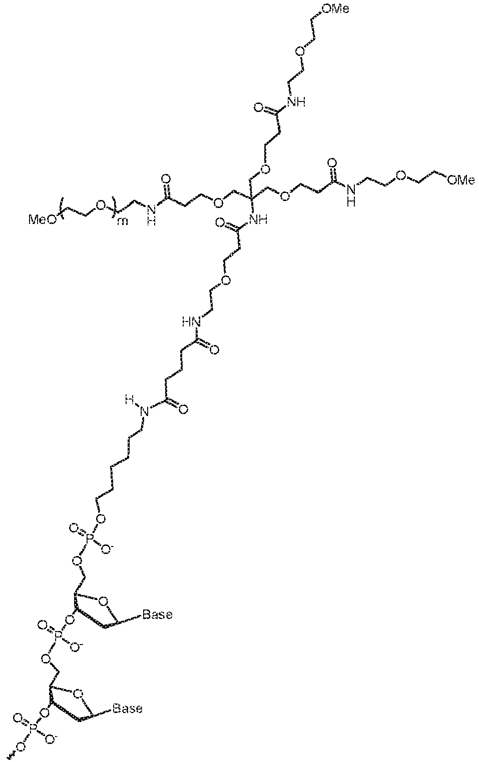

In some embodiments, a nucleic acid linker comprises a dendritic structure that functions as a steric barrier. For example, in some embodiments, a nucleic acid linker comprises an unlabeled strand having a polyester-16-hydroxyl bis-MPA dendron modification, as shown in the below structure depicting a terminally-modified unlabeled strand. It should be appreciated that any number of branches are envisioned for use in these and similar embodiments, as the number of branches can be optimized according to a particular labeled nucleotide configuration. For example, in some embodiments, a nucleic acid linker having a plurality of luminescent labels may require a greater number of branches to provide a larger steric barrier relative to a similar linker having a single luminescent label.

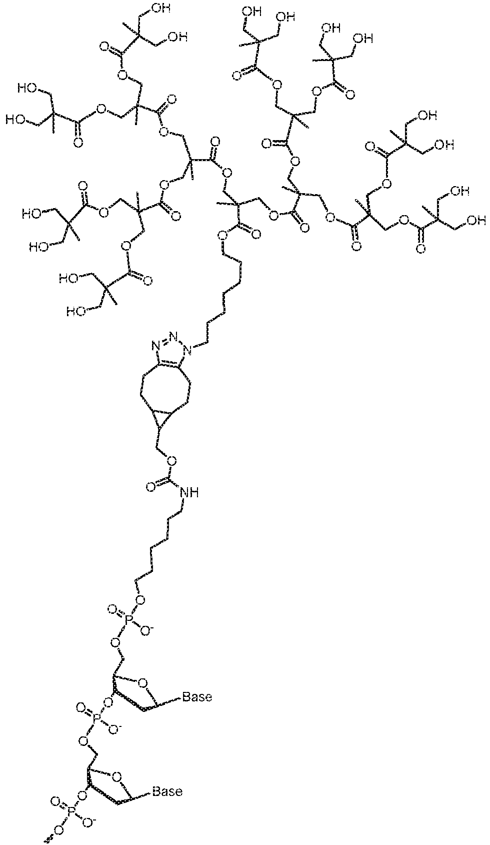

In some embodiments, a terminally-modified unlabeled strand is a polyester-8-hydroxyl bis-MPA dendron modified strand:

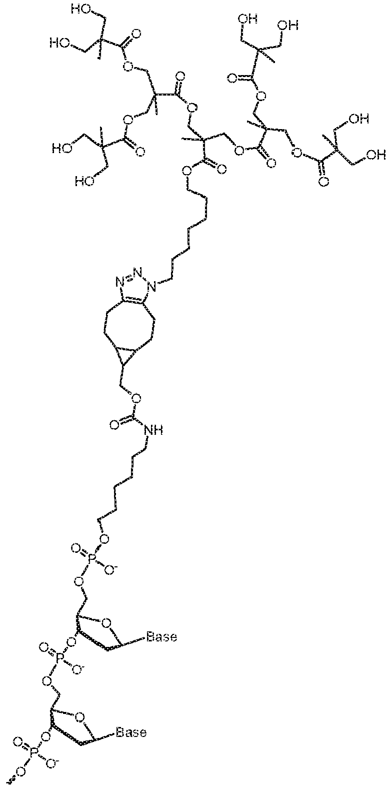

As described in the above, in some embodiments, an energy-absorbing modification is a modification to an unlabeled strand that protects the integrity of a sequencing reaction. In accordance with the techniques provided in the present disclosure, nucleic acid sequencing reactions may be conducted using a plurality of types of luminescently labeled nucleotides, where the luminescent properties of the nucleotides are unique to certain types of nucleotides. Thus, in some embodiments, detection of one or more of these unique luminescent properties allows for the identification of a specific type of nucleotide. As such, in some embodiments, any energy-absorbing modifications made to one or more luminescently labeled nucleotides should not interfere with the ability to detect specific types of nucleotides based on the respective unique luminescent properties. For example, various dyes (Chromis530N, Dylight554R1, and Cy3B) were each attached to a labeled strand and each labeled strand was annealed to unlabeled strands having either a 6-glycose-overhang energy-absorbing modification or a 6-glycerol dendrimer overhang energy absorbing modification, and luminescent intensity and lifetime values were measured for each oligonucleotide dimer. As shown in the results presented in Table 3, any changes in luminescent properties due to the energy-absorbing modifications approximately scaled across dye molecules. Thus, the luminescently labeled oligonucleotide dimers retained unique luminescent properties that would allow for the identification of each in a mixture.

**Table 3. Effects of energy-absorbing modifications on labeled oligonucleotides.**

| Normalized intensity | | | |
|---|---|---|---|
| | Chromis530N | Dylight554R1 | Cy3B |
| Standard duplex | 0.60 ± 0.05 | 0.36 ± 0.06 | 1.00 ± 0.17 |
| Modified with 6-glycose-overhang | 0.66 ± 0.05 | 0.38 ± 0.05 | 1.00 ± 0.12 |
| Modified with 6-glycerol dendrimer overhang | 0.75 ± 0.13 | 0.38 ± 0.06 | 1.00 ± 0.20 |
| | | | |

| Lifetime (ns) | | | |
|---|---|---|---|
| | Chromis530N | Dylight554R1 | Cy3B |
| Standard duplex | 4.0 ± 0.2 | 3.3 ± 0.2 | 2.5 ± 0.2 |
| Modified with 6-glvcose-overhang | 4.3 ± 0.2 | 3.3 ± 0.2 | 2.6 ± 0.1 |
| Modified with 6-glycerol dendrimer overhang | 4.6 ± 0.4 | 4.0 ± 0.4 | 2.9 ± 0.3 |

Accordingly, a luminescent label may be attached to the molecule directly. e.g., by a bond, or may be attached via a linker or a linker configuration. In certain embodiments, the linker comprises one or more phosphates. In some embodiments, a nucleotide is connected to a luminescent label by a linker comprising one or more phosphates. A linker described as having one or more phosphates refers to a linker that comprises one or more phosphates present within the linker structure (not directly attached to the one or more phosphates of a nucleotide). In some embodiments, a nucleotide is connected to a luminescent label by a linker comprising three or more phosphates. In some embodiments, a nucleotide is connected to a luminescent label by a linker comprising four or more phosphates.

In certain embodiments, a linker comprises an aliphatic chain. In some embodiments a linker comprises -(CH₂)ₙ-, wherein n is an integer from 1 to 20, inclusive. In some embodiments, n is an integer from 1 to 10, inclusive. In certain embodiments, a linker comprises a heteroaliphatic chain. In some embodiments, a linker comprises a polyethylene glycol moiety. In some embodiments , a linker comprises a polypropylene glycol moiety. In some embodiments, a linker comprises (CH₂CH₂O)ₙ , wherein n is an integer from 1 to 20. inclusive. In some embodiments, a linker comprises -(CH₂CH₂O)ₙ-, wherein n is an integer from 1 to 10, inclusive. In certain embodiments, a linker comprises -(CH₂CH₂O)₄-. In certain embodiments, a linker comprises one or more arylenes. In some embodiments, a linker comprises one or more phenylenes (e.g., para-substituted phenylene). In certain embodiments, a linker comprises a chiral center. In some embodiments, a linker comprises proline, or a derivative thereof. In some embodiments, a linker comprises a proline hexamer, or a derivative thereof. In some embodiments, a linker comprises coumarin, or a derivative thereof. In some embodiments, a linker comprises naphthalene, or a derivative thereof. In some embodiments, a linker comprises anthracene, or a derivative thereof. In some embodiments, a linker comprises a polyphenylamide, or a derivative thereof. In some embodiments, a linker comprises chromanone, or a derivative thereof. In some embodiments, a linker comprises 4-aminopropargyl-L-phenylalanine, or a derivative thereof. In certain embodiments, a linker comprises a polypeptide.

In some embodiments, a linker comprises an oligonucleotide. In some embodiments, a linker comprises two annealed oligonucleotides. In some embodiments, the oligonucleotide or oligonucleotides comprise deoxyribose nucleotides, ribose nucleotide, or locked ribose nucleotides.

In certain embodiments, a linker comprises a photostabilizer. In some embodiments, the linker is of formula: 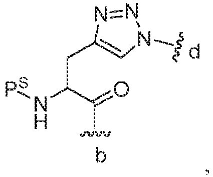 wherein P^{S} is a photostabilizer, the position labeled d is attached to a luminescent label, and the position labeled b is attached to a nucleotide. In some embodiments, the position labeled d is attached to a luminescent label by a linker as described herein. In some embodiments, the position labeled b is attached to a nucleotide by a linker as described herein.

In certain embodiments, a linker comprises one or more phosphates, an aliphatic chain, a heteroaliphatic chain, and one or more amides (e.g., -C(=O)NH-). In certain embodiments, a linker comprising one or more phosphates and an aliphatic chain can be synthesized via the exemplary reaction scheme 9-1 depicted in FIG. 9. In certain embodiments, a linker contains one or more functionalizable, reactive moieties (e.g., acetylene group, azido group).

### EQUIVALENTS AND SCOPE

Various aspects of the present application may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Also, the invention may be embodied as a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

The invention is further described by the following numbered embodiments.
1. A luminescently labeled nucleotide comprising one or more luminescent labels connected to one or more nucleoside polyphosphates via a nucleic acid, wherein the nucleic acid comprises a protecting element.
2. The luminescently labeled nucleotide of embodiment 1, wherein the nucleic acid is single-stranded.
3. The luminescently labeled nucleotide of embodiment 1, wherein the nucleic acid is double-stranded.
4. The luminescently labeled nucleotide of embodiment 3, wherein the nucleic acid comprises:
   a first oligonucleotide strand that comprises the one or more luminescent labels attached at an internal position having one or more nucleotides on either side along the first oligonucleotide strand; and
   a second oligonucleotide strand that comprises the one or more nucleoside polyphosphates, wherein the second oligonucleotide strand is annealed to the first oligonucleotide strand.
5. The luminescently labeled nucleotide of any preceding embodiment, wherein the two or more nucleoside polyphosphates are attached to the nucleic acid via a linker that comprises a plurality of thymidine nucleotides.
6. The luminescently labeled nucleotide of embodiment 5, wherein the linker comprises a branched linker.
7. The luminescently labeled nucleotide of any preceding embodiment, wherein the protecting element comprises at least one energy-absorbing modification.
8. The luminescently labeled nucleotide of embodiment 7, wherein the at least one energy-absorbing modification comprises a triplet state quencher.
9. The luminescently labeled nucleotide of embodiment 7, wherein the at least one energy-absorbing modification comprises a dendron modification.
10. The luminescently labeled nucleotide of embodiment 7, wherein the at least one energy-absorbing modification comprises a monosaccharide-TEG, a disaccharide, an N-acetyl monosaccharide, a TEMPO-TEG, a trolox-TEG, or a glycerol dendrimer.
11. The luminescently labeled nucleotide of any preceding embodiment, wherein the protecting element comprises one or more unlabeled stem-loops.
12. The luminescently labeled nucleotide of embodiment 11, wherein at least one of the one or more luminescent labels is attached at a loop of a stem-loop that is separated from the one or more nucleoside polyphosphates by the one or more unlabeled stem-loops.
13. The luminescently labeled nucleotide of any one of embodiments 4-12, further comprising a third oligonucleotide strand annealed to at least one of the first and second oligonucleotide strands.
14. The luminescently labeled nucleotide of embodiment 13, further comprising a fourth oligonucleotide strand annealed to at least one of the first, second, and third oligonucleotide strands.
15. The luminescently labeled nucleotide of embodiment 13, wherein the oligonucleotide strands form a Holliday junction.
16. A method of determining the sequence of a template nucleic acid comprising:
   (i) exposing a complex in a target volume, the complex comprising the template nucleic acid, a primer, and a polymerizing enzyme, to a plurality of types of luminescently labeled nucleotides, wherein each type of luminescently labeled nucleotide comprises a luminescently labeled nucleotide according to embodiment 1;
   (ii) directing a series of pulses of one or more excitation energies towards a vicinity of the target volume;
   (iii) detecting a plurality of emitted photons from luminescently labeled nucleotides during sequential incorporation into a nucleic acid comprising the primer; and
   (iv) identifying the sequence of incorporated nucleotides by determining timing and optionally frequency of the emitted photons.

## Claims

1. A luminescently labeled nucleotide comprising one or more luminescent labels connected to one or more nucleoside polyphosphates via a nucleic acid linker,
wherein the nucleic acid comprises a protecting element, and
wherein the protecting element comprises at least one energy-absorbing modification or one or more stem loops,
optionally wherein the at least one energy-absorbing modification or the one or more stem loops act as a steric and/or absorbing barrier that intercepts radiative and/or non-radiative decay emitted by the luminescent label.

2. The luminescently labeled nucleotide of claim 1, wherein the nucleic acid linker is single-stranded.

3. The luminescently labeled nucleotide of claim 1, wherein the nucleic acid linker is double-stranded.

4. The luminescently labeled nucleotide of claim 3, wherein the nucleic acid linker comprises:
a first oligonucleotide strand that comprises the one or more luminescent labels attached at an internal position having one or more nucleotides on either side along the first oligonucleotide strand; and
a second oligonucleotide strand that comprises the one or more nucleoside polyphosphates, wherein the second oligonucleotide strand is annealed to the first oligonucleotide strand.

5. The luminescently labeled nucleotide of any preceding claim, wherein two or more of the nucleoside polyphosphates are attached to the nucleic acid linker via a thymidine linker that comprises a plurality of thymidine nucleotides.

6. The luminescently labeled nucleotide of claim 5, wherein the thymidine linker comprises a branched thymidine linker.

7. The luminescently labeled nucleotide of any of claims 1-6, wherein the at least one energy-absorbing modification comprises a dendron modification, and optionally wherein the dendron modification is a glycerol dendrimer.

8. The luminescently labeled nucleotide of any of claims 1-6, wherein the at least one energy-absorbing modification comprises a triplet state quencher, and optionally wherein the triplet state quencher is TEMPO-TEG or trolox-TEG.

9. The luminescently labeled nucleotide of any of claims 1-6, wherein the at least one energy-absorbing modification comprises a monosaccharide-TEG, a disaccharide, an N-acetyl monosaccharide, a TEMPO-TEG, a trolox-TEG, or a glycerol dendrimer.

10. The luminescently labeled nucleotide of any preceding claim, wherein at least one of the one or more luminescent labels is attached at a loop of a stem-loop that is separated from the nucleoside polyphosphate by the one or more stem-loops.

11. The luminescently labeled nucleotide of any one of claims 4-10, wherein the nucleic acid linker further comprises a third oligonucleotide strand annealed to at least one of the first and second oligonucleotide strands.

12. The luminescently labeled nucleotide of claim 11, wherein the nucleic acid linker further comprises a fourth oligonucleotide strand annealed to at least one of the first, second, and third oligonucleotide strands.

13. The luminescently labeled nucleotide of claim 12, wherein the oligonucleotide strands form a Holliday junction.

14. The luminescently labeled nucleotide of any preceding claim, wherein the distance between the point at which the nucleic acid linker is connected to at least one of the one or more luminescent labels and the point at which the nucleic acid linker is attached to at least one of the one or more nucleoside polyphosphates is from 2 nm to 20 nm and wherein the distance is measured by modeling a three-dimensional structure of the nucleic acid linker and measuring the distance between the points as a linear measurement through space or a non-linear measurement across the surface of the three-dimensional structure of the nucleic acid linker.

15. A method of determining the sequence of a template nucleic acid, the method comprising:
(i) exposing a complex in a target volume, the complex comprising the template nucleic acid, a primer, and a polymerizing enzyme, to a plurality of types of luminescently labeled nucleotides, wherein each type of luminescently labeled nucleotide comprises a luminescently labeled nucleotide according to any of claims 1-14;
(ii) directing a series of pulses of one or more excitation energies towards a vicinity of the target volume;
(iii) detecting a plurality of emitted photons from luminescently labeled nucleotides during sequential incorporation into a nucleic acid comprising the primer; and
(iv) identifying the sequence of incorporated nucleotides by determining timing and optionally frequency of the emitted photons.
